(19) **Europäisches Patentamt**
**European Patent Office**
**Office européen des brevets**

(11) **EP 3 404 568 B1**

(12) **EUROPEAN PATENT SPECIFICATION**

(45) Date of publication and mention
of the grant of the patent:
**12.07.2023  Bulletin 2023/28**

(21) Application number: **18173025.0**

(22) Date of filing: **17.05.2018**

(51) International Patent Classification (IPC):
**G16B 40/00** *(2019.01)* **G16B 50/00** *(2019.01)*
**G16B 40/20** *(2019.01)* **G16B 50/10** *(2019.01)*
**G16B 50/20** *(2019.01)* **G16B 50/30** *(2019.01)*
**G16H 10/00** *(2018.01)* **G16B 99/00** *(2019.01)*

(52) Cooperative Patent Classification (CPC):
**G16B 50/00; G16B 40/00; G16B 40/20;
G16B 50/10; G16B 50/20; G16B 50/30;
G16B 99/00**

(54) **A SYSTEM AND A METHOD FOR DISCOVERY OF PREDICTED SITE-SPECIFIC PROTEIN PHOSPHORYLATION CANDIDATES**

SYSTEM UND VERFAHREN ZUR ENTDECKUNG VON VORHERGESAGTEN ORTSSPEZIFISCHEN PROTEINPHOSPHORYLIERUNGSKANDIDATEN

SYSTÈME ET PROCÉDÉ PERMETTANT DE DÉCOUVRIR DES CANDIDATS DE PHOSPHORYLATION DE PROTÉINES PRÉDITS SPÉCIFIQUES À UN SITE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.05.2017  EP 17171925**

(43) Date of publication of application:
**21.11.2018  Bulletin 2018/47**

(73) Proprietor: **FUJITSU LIMITED
Kanagawa 211-8588 (JP)**

(72) Inventors:
• **NOVÁCEK, Vit**
  **Co. Galway (IE)**
• **COSTABELLO, Luca**
  **Co. Galway (IE)**
• **VANDENBUSSCHE, Pierre-Yves**
  **Co. Galway (IE)**
• **MITSUISHI, Yutaka**
  **Kanagawa 211-8588 (JP)**

(74) Representative: **Haseltine Lake Kempner LLP
Cheapside House
138 Cheapside
London EC2V 6BJ (GB)**

(56) References cited:
**EP-A1- 1 339 006**

• **NEUBERGER GEORG ET AL: "pkaPS: prediction of protein kinase A phosphorylation sites with the simplified kinase-substrate binding model", BIOLOGY DIRECT, BIOMED CENTRAL LTD, LO, vol. 2, no. 1, 12 January 2007 (2007-01-12), page 1, XP021025705, ISSN: 1745-6150, DOI: 10.1186/1745-6150-2-1**
• **O. BUZKO ET AL: "A kinase sequence database: sequence alignments and family assignment", BIOINFORMATICS., vol. 18, no. 9, 1 September 2002 (2002-09-01), pages 1274-1275, XP055405747, GB ISSN: 1367-4803, DOI: 10.1093/bioinformatics/18.9.1274**
• **PIETER KELCHTERMANS ET AL: "Machine learning applications in proteomics research: How the past can boost the future", PROTEOMICS, vol. 14, no. 4-5, 1 March 2014 (2014-03-01), pages 353-366, XP055405990, DE ISSN: 1615-9853, DOI: 10.1002/pmic.201300289**
• **AMOS BAIROCH ET AL: "The SWISS-PROT protein sequence data bank", NUCLEIC ACIDS RESEARCH, vol. 19, no. Supplement, 25 April 1991 (1991-04-25), pages 2247-2249, XP055405809, DOI: https://doi.org/10.1093/nar/19.suppl.2247**

EP 3 404 568 B1

## Description

## Technological Field

The present invention relates to a system and a method for the discovery of site-specific phosphorylation statements, the statements linking a kinase, a predicted phosphorylation site, and a protein substrate. Arrangements are based on converting phosphorylation data into knowledge graphs, augmenting the graphs by relevant contextual information and consequent predictions using latent feature models.

The invention has multiple applications in the area of system and molecular biology and pharmacology. It is primarily used for human proteins and kinases, but may be applied to other species if the appropriate data is available.

## Background

Phosphorylation (the addition of phosphoryl group ($PO_3$) to a molecule) is a posttranslational modification of specific substrates by kinase proteins, and is a ubiquitous protein interaction driving many important signalling pathways in living cells. Phosphorylation often regulates for instance cell growth, division or programmed death. Disruptions of these regulatory processes are the main culprit of many serious diseases such as cancer. Therefore phosphorylation is one of the key topics studied in system and molecular biology, as well as in pharmacology.

Yet the rate of discoveries of new phosphorylation reactions and their function is still rather slow. One of the main reasons is the large number of combinations of possible site-specific phosphorylations; exploring this space is hard even with modern high-throughput technologies. Another reason is that there are experimental difficulties in establishing which of the candidate reactions actually do happen in cells and lead to a significant effect.

Also finding specific kinases binding to known phosphorylation sites is still challenging. Computational tools for phosphorylation prediction are therefore in high demand. The existing approaches have limitations, though. Some focus only on several kinases and/or a selected range of substrates. All tools compute predictions based on local protein features, disregarding broader network context. Importantly, none of the prior art tools allow for prediction of phosphorylations by querying for a specific kinase/protein. This substantially limits their potential for semi-automated experiment design aimed at pathways related to kinases of interest, since one would generally need to query the tools for thousands of possible substrates to do so.

Document Neuberger, G et al. pkaPS: prediction of protein kinase A phosphorylation sites with the simplified kinase-substrate binding model, Biology direct 2:1, pp. 1-23 represents the closest prior art and discloses the importance of determining the complete protein substrate set for each kinase. The document fails to disclose how to generate negative examples when needed.

Automated prediction of site-specific phosphorylation candidates is much desired as it may substantially reduce the space of possible phosphorylations to be experimentally verified. This may lead to much increased rate of discoveries in cellular signalling, which may in turn contribute to more efficient treatment of diseases like cancer.

According to an embodiment of a first aspect of the invention, there is provided a system (or apparatus) for discovery of predicted site-specific protein phosphorylation statements, the statements linking a predicted phosphorylation site, a protein substrate and a kinase, the system comprising:

a motif converter which is to receive inputs from:

a phosphorylation data set storing known site-specific statements each in a site-specific format linking a kinase, a phosphorylation site and a protein substrate;
a kinase family database; and
a protein sequence database,
the motif converter being arranged to use the site-specific statements and additional information from the kinase family database and protein sequence database to produce motif-based phosphorylation statements each in a generalised format linking a kinase,
a motif in the form of a generalised scoring matrix for amino acids at a phosphorylation site and to either side of the phosphorylation site; and a protein substrate;
a link predictor to predict new motif-based statements, the link predictor including:

a negative motif-based statements generator to generate negative statements from the motif-based statements;
a candidate generator to generate candidate statements in the generalised format by combination of the motif-based statements and to generate negative candidate statements by combination of the candidate statements with the negative statements;
a relational learner to use the candidate statements and the negative candidate statements in a statistical relational learning model to score the candidate statements and retain predicted motif-based phosphorylation statements in the generalised format; and

a site-specific converter to receive inputs from the protein sequence database and to receive the predicted motif-based phosphorylation statements in the generalised format and which is arranged to produce predicted site-specific phosphorylation statements in the site-specific format by using a motif conformance score for possible phosphorylation sites;

wherein the negative motif-based statements generator is to create negative statements by one or more of: replacing the substrate in a motif-based statement <K, M, S> with a different substrate in that for each phosphorylation site on the different substrate, the context sequence is compared with the motif of the motif-based statement, and if the conformance is negative, the substrate S in a motif-based statement <K, M, S> is replaced by a different substrate S' and a negative statement <K, M, S'> is provided; and replacing the kinase in a motif-based statement with a kinase from a different kinase family in that for each phosphorylation site on the different substrate, the context sequence is compared with the motif of the motif-based statement, and if the conformance is negative, the kinase K in a motif-based statement <K, M, S> is replaced by a different kinase K' and a negative statement <K', M, S> is provided.

The system can predict unknown phosphorylations based on an entire network of known, manually curated phosphorylations. Predictions based on both substrate and kinase queries are possible. This approach outperforms state of the art systems and, for example, predicts 9 actual phosphorylations by LATS1, AKT1 kinases in humans that were not known previously.

The system obtains predicted site-specific protein phosphorylation statements, the statements linking a kinase, a predicted phosphorylation site, and a protein substrate. The site-specific statements with these three entities may be represented as <K (kinase), L or site, S (substrate)>. The link represents the predicted phosphorylation interaction at the given site on the substrate using the kinase.

The motif converter takes a phosphorylation data set storing known site-specific statements each in a site-specific format linking a kinase, a phosphorylation site and a protein substrate and uses these and additional information from a kinase family database and a protein sequence database to convert the site-specific statements to motif-based phosphorylation statements. These are in a "generalised" format in the sense that they link a kinase, a motif in the form of a generalised scoring matrix for amino acids at a phosphorylation site and to either side of the phosphorylation site; and a protein substrate. The generalised statements with these three entities may be represented as <K (kinase), M (motif), S (substrate)>. In embodiments, the motif is provided before the generalised statements are created, it may be viewed as a (new type of) consensus sequence pattern which is derived by grouping of the input site specific statements. The motif is learned from a group of phosphorylation sites and their amino acid contexts (in general, protein sequence motifs are learned from a set of protein sequences, of which the sites and their contexts are a sub-type). Hence the motif creation may be seen more as a group process than as a site-by-site process.

The link predictor can be viewed as operating in a single process, or as including a learning phase and a prediction phase. The following description separates the functions into these two phases. To build a model (for predicting new motif-based statements), the link predictor includes a negative motif-based statements generator to generate negative statements from the motif-based statements, and a candidate generator, which creates candidate statements in the generalised format by combination of the motif-based statements. The candidate generator also generates negative candidate statements (indicating that phosphorylation is not expected to occur at the stated motif on the stated substrate using the stated kinase. The negative candidates are generated by combination of the motif-based candidate statements and the negative motif-based statements). The positives and negatives are needed to train link prediction models using a relational learner. A best-performing model may be selected.

The relational learner uses the candidate statements and the negative candidate statements in a statistical relational learning model to score and maybe also rank the candidate statements (and potentially the negative candidate statements). This can be used in training the model(s), with train/test/validation splits of (verified) input data.

In a prediction phase, the link predictor operates on proteins of interest entered by a user, or on a range of proteins or even on all proteins. Candidate statements and negative candidate statements are produced in the generalised format as before (but based on the query, e.g. covering all possible interactions of the specific query protein(s) with the phosphorylation network). The scoring of the candidate statements by the model allows retention of higher scored/ranked statements as predicted statements (the most likely unknown phosphorylations).

Finally, the site-specific converter uses the protein sequence database to convert the predicted motif-based phosphorylation statements into the site-specific format (using a motif conformance score for possible phosphorylation sites, which is a mathematical measure of similarity between the motif and a specific sequence). This site-specific format is required by users of the system, because it allows identification of predicted phosphorylation locations along with the substrate and kinase, which may then be further explored.

The motif converter may

associate each site-specific statement with a kinase family in the kinase family database;

use the protein sequence database to form a context sequence of an amino acid at the phosphorylation site for each site-specific statement and a plurality of amino acids to either side of the phosphorylation site;

group the context sequences by kinase family;

for the context sequences in each kinase family, compute one or more position-specific scoring matrices each of which provides a score for each amino acid at each position of the context sequence length, each position-specific scoring matrix defining a motif;

for each site-specific statement, compute the conformance between the context sequence for that site-specific statement and each motif in the kinase family in which the context sequence is grouped;

for each site-specific statement, create a motif-based statement linking the kinase, the best performing motif and the protein substrate.

The context sequence and motif are of the same length, which may be from 3 to 21 amino acids to either side of the amino acid (and the amino acid at the phosphorylation site), and may be from 7 to 15 amino acids to either side. In fact, any integer number above zero may work with the technique, so potentially just the location amino acid and 1 acid either side could be used. However the results vary quite widely: this length is an important hyperparameter of the methods, and it appears that a length 7 or 15 amino acids to either side may be the most suitable in most circumstances. Very short context windows are not likely to bring good results. The window need not be centred on the phosphorylation site. Normally the number of amino acids considered to the left and to the right of the phosphorylation site is equal, but there may be more amino acids to one side, for example there may be 3 on the left and 5 on the right to produce a context sequence of full length 9.

in more detail, the negative statements generator is to create negative statements by one or more of: replacing the substrate in a motif-based statement (<K, M, S>) with a different substrate and replacing the kinase in a motif-based statement with a kinase from a different kinase family. Thus there are two methods of making negative statements.

In the first method, the substrate S in a motif-based statement <K, M, S> is replaced by a different substrate S' in that for each phosphorylation site on the different substrate, the context sequence is compared with the motif of the motif-based statement, and if the conformance is negative, the negative statement <K, M, S'> is provided. As an aside, it is noted that the order of the entities in the statements used herein is arbitrary and that any order is acceptable and equivalent if used consistently.

In the second method, the kinase K in a motif-based statement <K, M, S> is replaced by a different kinase K' in that for each kinase which is not in the same family as kinase K, a statement <K', M, S> is provided.

In the generation of negative statements, the negative statements and the motif-based statements may be compared and any statement that appears both as one of the negative statements and as one of the motif-based statements is deleted from the negative statements.

A negative statements filter in the candidate generator may then, for each kinase and motif in a candidate statement, create negative candidate statements as the combination of the kinase, the motif and all substrates associated with the kinase and the motif in the negative statements.

The negative statements filter may also, for each motif and substrate in a candidate statement, create negative candidate statements as the combination of the motif, the substrate and all kinases associated with the substrate and the motif in the negative statements. Hence there are two methods of creating negative candidate statements which may be used,

The negative statements filter may discard from the negative candidate statements any statement that appears both as one of the negative candidate statements and as one of the candidate statements.

The relational learner may learn a model from the motif-based statements and negative statements and may then use the model to score the candidate statements and the negative candidate statements.

The candidate generator may create candidate statements as the combination of every kinase included in the motif-based statements with every motif included in the motifbased statements and every substrate included in the motif-based statements (a Cartesian product) minus the motif-based statements themselves. The candidates are not used in the training of the model, but only scored later, once the model has been created.

To do this, the relational learner may score and rank a candidate statement and all the negative candidate statements with the same kinase and motif or with the same kinase and substrate and output the rank and score of the candidate statement.

A protein statements generator may receive input from a protein context database linking proteins by their interactions and may produce protein context statements, for use in the link predictor. This additional information enriches the knowledge used in the system.

The site-specific converter may:

input the predicted motif-based phosphorylation statements;

for each predicted motif-based phosphorylation statements extract a list of possible phosphorylation sites from a phosphorylation sites database;

for each phosphorylation site, extract the site context sequence from the protein sequence database and compute the conformance of the site context sequence with the motif in the predicted motif-based phosphorylation statement;

if the conformance for the phosphorylation site is above a predefined score (for example zero), produce a predicted site-specific phosphorylation statement in the site-specific format linking the kinase and the substrate in the predicted motif-based statement with the phosphorylation site.

According to an embodiment of a second aspect of the invention, there is provided a computer-implemented method for discovery of predicted site-specific protein phosphorylation statements, the statements linking a predicted phosphorylation site, a protein substrate and a kinase, the method comprising:
using data from:

a phosphorylation data set storing known site-specific statements each in a site-specific format linking a kinase, a phosphorylation site and a protein substrate;
a kinase family database; and
a protein sequence database,
using the site-specific statements and additional information from the kinase family database and protein sequence database to produce motif-based phosphorylation statements each in a generalised format linking a kinase, a motif in the form of a generalised scoring matrix for amino acids at a phosphorylation site and to either side of the phosphorylation site; and a protein substrate;
generating negative statements from the motif-based statements;
generating candidate statements in the generalised format by combining the motifbased statements;
generating negative candidate statements by combination of the candidate statements with the negative statements;
using the candidate statements and negative candidate statements in a statistical relational learning model to score the candidate statements and retain predicted motifbased phosphorylation statements in the generalised format;
receiving data inputs from the protein sequence database and the predicted motif-based phosphorylation statements in the generalised format and producing predicted site-specific phosphorylation statements in the site-specific format by using a motif conformance score for possible phosphorylation sites; wherein
the generation of negative motif-based statements generator creates negative statements by one or more of: replacing the substrate in a motif-based statement <K, M, S> with a different substrate in that for each phosphorylation site on the different substrate, the context sequence is compared with the motif of the motif-based statement, and if the conformance is negative, the substrate S in a motif-based statement <K, M, S> is replaced by a different substrate S' and a negative statement <K, M, S'> is provided; and
replacing the kinase in a motif-based statement with a kinase from a different kinase family in that for each phosphorylation site on the different substrate, the context sequence is compared with the motif of the motif-based statement, and if the conformance is negative, the kinase K in a motif-based statement <K, M, S> is replaced by a different kinase K' and a negative statement <K', M, S> is provided.

A method according to preferred embodiments of the present invention may comprise any combination of the apparatus or computer program aspects. Methods or computer programs according to further embodiments may be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software.

According to a further aspect there is provided a program comprising instructions, which when executed by a computer, cause the computer to carry out the method steps according to any of the preceding method definitions or any combination thereof.

In general the computer may comprise the elements listed as being configured or arranged to provide the functions defined. For example this computer may include memory, processing, and a network interface.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network. Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. For example, the steps of the invention may be performed in a different order and still achieve desirable results. Multiple test script versions may be edited and invoked as a unit without using object-oriented programming technology; for example, the elements of a script object may be organized in a structured database or a file system, and the operations described as being performed by the script object may be performed by a test control program.

Elements of the invention have been described using the terms "converter", "predictor", "generator", "learner" etc. The skilled person will appreciate that such functional terms and their equivalents expressed in terms of units may refer to parts of the system that are spatially separate but combine to serve the function defined. Equally, the same physical parts of the system may provide two or more of the functions defined.

For example, separately defined means may be implemented using the same memory and/or processor as appropriate.

Preferred features will now be described, purely by way of example, with references to the accompanying drawings, in which:-

Figure 1A is a block diagram of a system overview for information;
Figure 1B is a flow chart of a method in a general arrangement, also for information;
Figure 1C is a block diagram of a more detailed system overview, also for information;
Figure 2 is a block diagram including main system components in a link prediction unit/predictor of a general arrangement which is an embodiment of the invention;
Figure 3 is a diagram of a converter to motif-based statements, which may be used in an invention embodiment;
Figure 4 is a flow chart of a motif discovery unit, which may be used in an invention embodiment;
Figure 5 is flow chart of a motif-based statement generator, which may be used in an invention embodiment;
Figure 6 is block diagram of a knowledge base enrichment unit to add protein context statements, which may be used in an invention embodiment;
Figure 7 is a flow chart of protein context statements of a generation unit which may be used in an invention embodiment;
Figure 8 is a block diagram of a link prediction unit, which may be used in an invention embodiment;
Figure 9 is a flow chart of a negative motif-based statements generation unit, which may be used in an invention embodiment;
Figure 10 is a flow chart of a negative protein context statements generation unit, which may be used in an invention embodiment;
Figure 11 is a block diagram of a candidate generation unit, which may be used in an invention embodiment;
Figure 12 is a flow chart of a positive candidate statements generation unit, which may be used in an invention embodiment;
Figure 13 is a flow chart of a negative candidate statements generation unit, which may be used in an invention embodiment;
Figure 14 is a flow chart of a relational learning unit which may be used in an invention embodiment;
Figure 15 is a block diagram of a conversion to site-specific statements unit, which may be used in an invention embodiment;
Figure 16 is a flow chart conversion to site-specific statements unit, which may be used in an invention embodiment;
Figure 17 is a flow chart of training of a phosphorylation prediction system, according to an embodiment;
Figure 18 is a flow chart of making predictions using a specific phosphorylation prediction system, according to an embodiment;
Figure 19 is a group of 3 plots of mass spectrometer validation of a subset of predicted phosphorylations; and
Figure 20 is a block diagram of a computing device to carry out methods of arrangements.

Arrangements falling under the scope of the claims form embodiments of the invention.

Most current approaches to phosphorylation prediction tend to have a conceptually similar approach to the phosphorylation prediction problem. They typically start from a data set on potential phosphorylation sites (which are relatively easy and cheap to obtain in large quantities due to techniques like mass spectrometry). Then they identify substrates that contain potential phosphorylation sites (usually focusing only on relatively few possible site/substrate combinations). The last step is identification of kinases (enzymes used to phosphorylate proteins) that could target these sites in the substrates. The specific prior arts differ in the exact methodology of the particular steps, but not so much in the general approach. The main reason for use of the same general approach is that until relatively recently, this has been possibly the only plausible approach to the problem as there were no other types of data applicable to predicting phosphorylations. However, in the last decade, several large, machine-readable and manually curated phosphorylation networks datasets have become available. These data sets present a summary of current knowledge of all site-specific phosphorylation relations in the form of a knowledge graph, also known as a graph database. This is a valuable source of information since the latent structure of such knowledge bases may typically yield many new discoveries as has been shown in other domains, such as in knowledge base completion or knowledge fusion. The inventors have realised that this knowledge graph may also be easily combined with other relevant sources of contextual information (such as protein interaction networks) which is an additional advantage over the data sets typically used by prior art for phosphorylation prediction. Yet there is no existing technique capable of tapping into this type of information to predict new phosphorylations at the moment.

Graph databases/knowledge graphs may be used to maintain large "semantic networks" that may store large amounts of structured and unstructured data in various fields. There are several types of graph representations. Graph data may be stored in memory as multidimensional arrays, or as symbols linked to other symbols. Another form of graph representation is the use of "tuples," which are finite sequences or ordered lists of objects, each of a specified type, such as a Uniform Resource Identifier, URI. The knowledge base itself may well use tuples for storage, as do many data stores. A tuple containing n objects is known as an "n-tuple," where n may be any non-negative integer greater than zero. A tuple of length 2 (a 2-tuple) is commonly called a pair, a 3-tuple is called a triple, a 4-tuple is called a quadruple, and so on.

Triples are one of the most commonly used tuples. Each triple may be visualised as a subject-predicate-object (s-p-o) statement or relation. The triples may be Resource Description Framework (RDF) triples. The Resource Description Framework is a general method for conceptual description or modelling of information that is a standard for semantic networks. Standardising the modelling of information in a semantic network allows for interoperability between applications operating on a common semantic network. RDF maintains a vocabulary with unambiguous formal semantics, by providing the RDF Schema (RDFS) as a language for describing vocabularies in RDF.

An RDF graph/triples may be queried (accessed) using the SPARQL Protocol and RDF Query Language (SPARQL). It was standardized by the RDF Data Access Working Group (DAWG) of the World Wide Web Consortium, and is considered a key semantic web technology.

The inventors have come to the realisation that the prediction of new phosphorylations from knowledge graphs that summarise the relevant biological knowledge may be associated with the following technical problems (which the inventors have identified):

**Technical Problem A) Neglecting the potential of known phosphorylations and related contextual information represented as knowledge graphs.** Phosphorylation knowledge bases and protein networks present graph statistical patterns and regularities such as autocorrelation, block structure, global and long-range statistical dependencies over chains of proteins made of different relation types. These patterns may be used as foundations for effective phosphorylation predictions from known, curated data sets. However, designing predictive models that learn as many combinations of such patterns as possible is not straightforward in the phosphorylation context. This is due to the fact that for successful solutions, approaches from diverse fields of biology, bioinformatics and relational machine learning have to be combined in a non-obvious way.

**Technical Problem B) Lack of tools that can learn from context-dependent networks.** Phosphorylation knowledge graphs are made of untyped binary relations that are, however, dependent on contexts (i.e., the phosphorylation sites). This prevents straightforward processing with state-of-the-art machine learning models, since these models are designed to work on typed binary relations without contexts. Therefore, the context information has to be converted into relation types in order for the models to work. In this context, typed relations are pairwise relations between entities A and B (e.g., proteins) where the relationship is of a specific type R (e.g., phosphorylation or inhibition), and untyped relations are pairwise relations between entities A and B (e.g., proteins) where the type of relationship is not specified (i.e., the statement says nothing beyond the fact that A and B are somehow associated)

**Technical Problem C) Inappropriateness of standard negative generation techniques for phosphorylation knowledge graphs.** Machine learning models typically used for mining knowledge graphs (such as statistical relational learning) require negative examples. These are difficult to establish in many realistic use cases due to the Open World Assumption (missing knowledge should not be treated as negative, but merely as unknown). This is also the case of phosphorylation (one cannot simply take statements not represented in the current data sets as negatives as they may possibly represent phosphorylations that simplify have not been discovered yet).

To the best of the inventors' knowledge, there is no bioinformatics technique capable of tapping into phosphorylation knowledge graphs to predict new phosphorylations. Additionally, no one has solved the technical problems presented above. More specifically:

Technical Problem A is not solved because the prior art adopts the traditional approach to phosphorylation prediction (starting with the sites and associating likely substrates and kinases with them). The graph-like structures representing phosphorylations are often an output of the prior art methods, but not a primary input. Therefore these works cannot take into account the available manually curated knowledge represented as phosphorylation networks. They also cannot easily combine this data with other graph-like data (such as protein interactions) that may be very beneficial for making the predictions.

No other work tackles phosphorylation prediction with a graph-based approach. Therefore there are also no existing solutions to transform the untyped, context-dependent phosphorylation data sets into actionable knowledge graphs composed of typed, contextindependent relationships. Thus there is no current proposed prior art solution for Technical Problem B.

Techniques for generating plausible negative examples under Open World Assumption have been proposed in the state-of-the-art. However, these techniques are generally not reliable in the phosphorylation context, since they may theoretically produce some negative statements that are positive in real life, which may seriously hamper the performance of the prediction models. While there are existing approaches for generating negative examples of phosphorylation sites, there is no prior art concerning negative examples of the whole phosphorylation statements.

Arrangements may solve or mitigate one or more of Technical Problems A to C.

Technical Solution for Solving the Problem

Introduction

Arrangements present a novel approach to computational discovery of site-specific phosphorylation reactions. The approach combines knowledge graphs, relational learning, and transformation of phosphorylation data using consensus motifs of kinase families. The arrangements may solve any or all of technical problems A) B) or C) as follows:

A) By adopting statistical relational learning, arrangements may solve Technical Problem A because they may take into account the whole phosphorylation network and additional context information as a comprehensive knowledge graph. This approach may utilise statistical patterns hidden in the data and leverage the presence of known specific relations to predict new phosphorylations. For example, the system may exploit long-range statistical dependencies over chains of proteins with different relation types, which none of the prior art does.

B) To solve Technical Problem B (i.e., overcoming the lack of models able to process context-dependent phosphorylation graphs), arrangements convert phosphorylation data sets into typed binary relations. The types are based on discovery of substrate sequence motifs preferred by particular kinase families. These motifs are secondary structures grouping similarities in structure between sequences. They are extracted from phosphorylation site contexts: the amino acids (or more strictly speaking, amino acid residues) to either side of the amino acid forming the phosphorylation site. Phosphorylation site contexts are transformed into predicates that correspond to motifs having the best compatibility with the site context sequence. These predicates provide a generalisation of the site context information that may be meaningfully compared across different statements and thus support state of the art statistical relational learning.

C) Arrangements generate negatives for phosphorylation statements based on the actual biology of phosphorylation, not using the standard methods that may produce erroneous results (Technical Problem C). This solution makes use of the negative conformance of substrate sequences with respect to the predicate motifs and the fact that kinases from different families tend to bind to different substrates at different sites.

**System Overview**

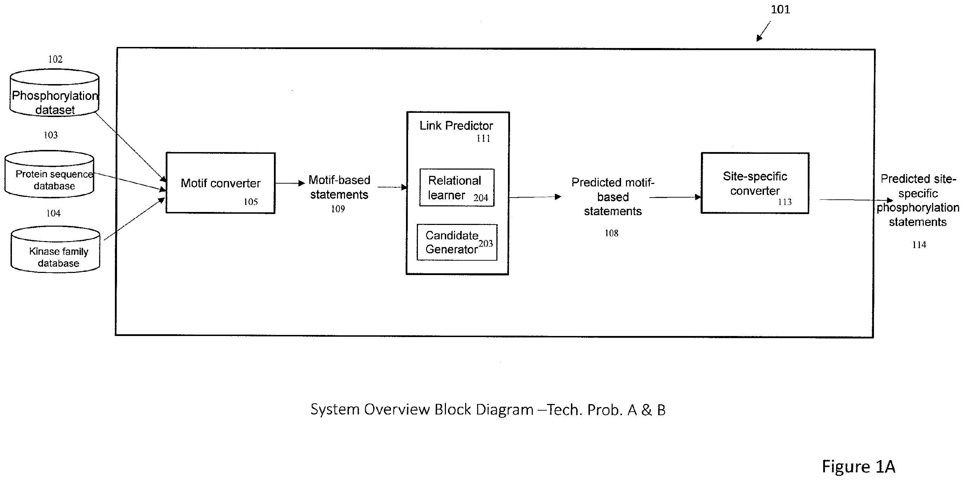

Figure 1A presents an overview of a general arrangement. The components present in the system 101 figure are as follows:

- Phosphorylation data set or network 102 is provided as an input to component 105 (the converter). It may consist of descriptions of phosphorylation interactions between a protein kinase and a protein substrate at a given location in the substrate amino acid sequence. It is assumed that each interaction may represented as a statement (or triple) of the form:

⟨*protein kinase, phosphorylation site location, protein substrate*⟩.

- Protein sequence database 103 is provided as an input for the converter to motifbased statements 105 and the converter to site-specific statements 113. At the very least, it consists of information on proteins amino acid sequences. It is assumed that each protein entry may contain at least the following information:
⟨*protein, substrate sequence*⟩,

e.g., ⟨P04637, MEEPQSDPSVEPPLSQETFS....⟩

- Kinase family database 104 is provided as an input to the motif converter 105. It consists of a mapping from all protein kinases to their corresponding kinase family. It is assumed that the mapping is represented in the form: ⟨*protein kinase, kinase family*⟩.
- The motif converter for conversion to motif-based statements 105 converts the phosphorylation network consisting of site-specific statements to generalised motifbased statements 109, as will be explained in more detail in the following. A motif is a common or consensus sequence pattern (of amino acids) relating to one or more proteins.
- Motif-based statements 109 are converted phosphorylation statements with (actionable) motif-based predicates. Together, they form a knowledge graph.
- Link Predictor/Prediction Unit 111 handles the prediction of new motif-based phosphorylation statements. It uses a candidate generator 203 to create new candidates from the motif-based statements and a relational learner 204 to use the candidate statements in a learning model and to score them and thus provide a set of predicted statements which is a subset of the candidate statements. The output statements may be scored based on the level of confidence the model associates with them. Such weights may be provided alongside of the statements (e.g., in the same tuple).
- The site-specific converter 113 for conversion to Site-specific Statements converts the predicted motif-based statements to a "standard" format of site-specific phosphorylation statements which may consequently be processed by the end users (i.e., biomedical scientists) for their own purposes. The component preserves any scores generated by link predictor 111 and possibly augments them by scores specific to the conversion process. This results in the construction of predicted site specific phosphorylation statements 114.
- The predicted site specific phosphorylation statements 114 may be list(s) of statements in the form:
⟨*protein kinase, phosphorylation site location, protein substrate, [scores]* ⟩.Each statement may include a set of scores generated by Link Predictor 111 and Site-specific converter 113). These scores may be used for ranking the results (which may be naturally used for determining the statements that are most likely to correspond to actual phosphorylations).

One idea of arrangements as depicted in Figure 1A is to process the known phosphorylation data and potentially also related contextual data as a knowledge graph of motif-based statements 109, using a link prediction technology. This approach may yield previously unknown (predicted) phosphorylation statements 114. The predictive model can be trained on publicly available phosphorylation and protein interaction networks (such as PhosphoSitePlus). The two-way conversion between the site-specific phosphorylation statements and statements with predicates that generalise the site information allows to treat phosphorylation networks as knowledge graphs. The conversion is based on identifying consensus site motifs preferred by kinase families. The converted data allows the use of link prediction - a recent advance in statistical relational learning - to discover candidate phosphorylation reactions.

Conversion of the site-specific (i.e., protein site-context-dependent) statements to triples with actionable predicates (i.e., links) is based on generalisations of the phosphorylation sites (as motifs). The conversions between these types of statements are realised by the two converters and may use auxiliary information on the proteins and phosphorylation sites. Arrangements may be capable of generating and scoring any statement that includes kinase and substrate present in the input data, and a site that corresponds to motifs generated from the input data. This can be used for producing a broad range of predictions on any protein present in the input data.

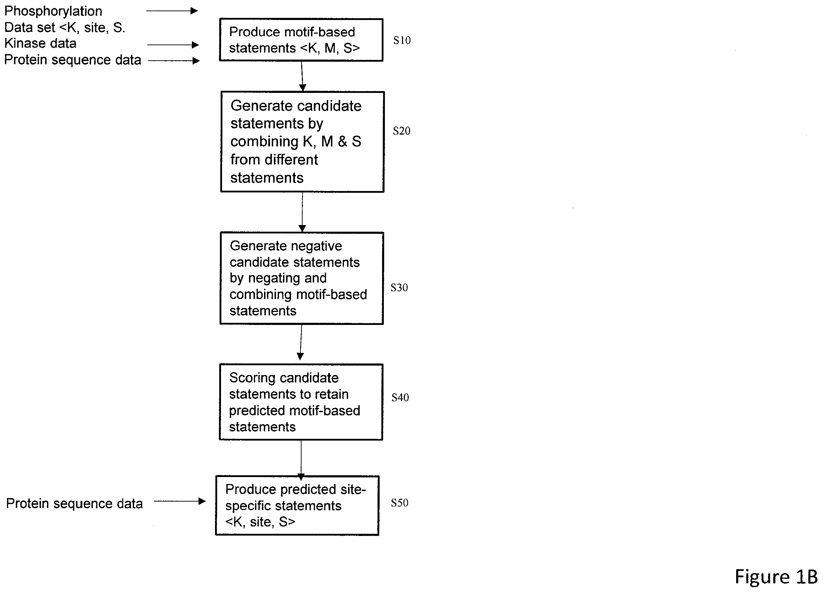

Figure 1B is a flow chart depicting the method of a general arrangement. In step S10, motifbased statements <K, M, S> are produced from the inputs of a phosphorylation data set which has statements of the form <K, site, S>. In order to do this the method uses kinase data indicating families which kinases are grouped into, as well as protein sequence data which gives the sequence for proteins. From the motif-based statements generated in step S10, candidate sequences are generated in step S20 by combining the kinases, motifs and substrates from different statements. This may be as a Cartesian product in that each kinase is combined with each motif and each substrate. In step S30 negative candidates are generated. This is by negating the motif-based statements and then use of combining, potentially using a Cartesian product. Preferred methods of negation are detailed later.

In step S40 the candidate statements are scored (also using the negative candidate statements) and only higher scoring candidate statements are retained as predicted motifbased statements.

Finally in step S50 protein sequence data is input to allow the motif-based statements to be converted to predicted site-specific statements in the form <K, site, S>.

## Example 1

*Examples of existing knowledge on phosphorylations (i.e., site-specific phosphorylation statements) are: ⟨P04049, S99, Q92934⟩, ⟨P23588, S497, P04049⟩, ⟨P04049, S259, P04049⟩. In these statements, the first element corresponds to a kinase protein (the phosphorylation "agent") and the third element corresponds to a substrate protein (the phosphorylation "patient"). The second element corresponds to a phosphorylation site in the substrate amino acid sequence. The site identifier is composed of the amino acid residue (indicated by the letter) and the position of the residue in the substrate sequence (indicated by the number).*
*P04049 in the statements above refers to RAF1, an important human regulatory kinase involved in many pathways related to cancer. The examples show how a kinase may also act as a substrate and thus participate in so called phosphorylation cascades. Kinases may also phosphorylate themselves in feedback loops.*
*It is assumed that a biologist wants to find out whether or not RAF1 / P04049 could phosphorylate the substrate Q92915 which refers to Fibroblast growth factor 14, a not so well understood protein that is suspected to be involved in nervous system development and possibly also in related malignancies.*
*The existing techniques cannot predict anything if provided with just a list of statements (i.e., knowledge graph) like the ones above. They typically require custom data sets built from protein and phosphorylation site databases, manually processed features and positive /negative example batches.*
*Arrangements may, however, process the data from publicly available resources purely automatically and come up with a prediction such as (P04049, T190, Q92915), which may consequently be processed by the biologist in a wet lab that is a physical, real-life laboratory as opposed to a simulation or mathematical exploration. This illustrates how arrangements target Technical Problem A differently from the prior art.*

## Example 2

*The link prediction unit 111, however, requires statements with actionable predicates, not the phosphorylation site information in order to be able to produce meaningful predictions. Using prior art in link prediction with statements like ⟨P04049, S99, Q92934⟩, gives meaningless results since S99 in protein Q92934 generally means something totally different from S99 in any other protein. This is due to the fact that it is just a pointer to a specific place in a substrate protein sequence and not something that may be generalised across different proteins (a key requirement for an actionable predicate in the scope of link prediction).*
*The inventors have realised that the statements therefore need to be converted before and after link prediction to produce meaningful results. An example of a statement converted to an internal format that may be meaningfully processed by link prediction is (P04049, TKL2, Q92934) where TKL[2] refers to a predicate that generalises information on multiple phosphorylation sites and therefore may be compared across different substrate proteins.*
*This addresses Technical Problem B and is handled by Converters 105, 113 which connect between site-specific and generalised (motif-based) in Figure 1A. Examples of more technical details in the conversion process will be given later, after the description of the specific components.*

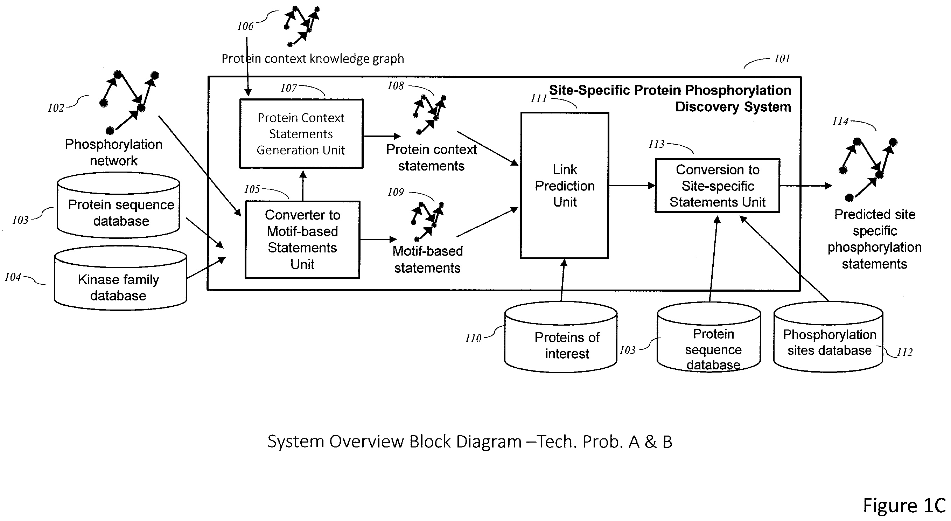

Figure 1C is an overview of a more specific arrangement. In addition to the parts already described for Figure 1A above, Figure 1C includes the following components:

- Protein context knowledge graph 106 is provided as an input to component 107 (the protein context statement generator). It consists of statements related to the protein kinases and protein substrates such as additional protein-protein interactions. It is assumed the knowledge graph is represented in the form of a tuples:

  ⟨*subject, relation, object*⟩
  indicating that *subject* and *object* entities (proteins) hold a relationship *relation (such as the two bind together).*

- Protein Context Statements Generation Unit 107 extracts additional statements from protein context knowledge graph on the proteins kinases and substrates part of the motif-based statements. This leads to the construction of protein context statements for the proteins for which there are site-specific statements, represented in the form of a tuple:
  ⟨*subject, relation, object*⟩
- Protein context statements 108 selected triples from protein context knowledge graph 106 expressing additional contextual information on protein kinases and protein substrates coming from the motif-based statements 109.
- Proteins of interest 110 Kinases or substrate proteins of interest may be provided as an input to component 111 (the link predictor). This input may consist of a list of one or more proteins for which the user would like to have predictions (e.g., information on which substrates given kinases possibly phosphorylate at which specific site). If this input is not provided, the predictions may be computed on a set of all possible phosphorylations induced by the kinases and substrates present in the input data.

- Phosphorylation sites database 112 may be provided as an input for component 113. It consists of information on all potential phosphorylation sites of protein substrates. It is assumed that each protein entry contains at least the following information:

⟨*protein, list of phosphorylation sites*⟩,
e.g., ⟨*P04637, [S106, T523, ...]*⟩.

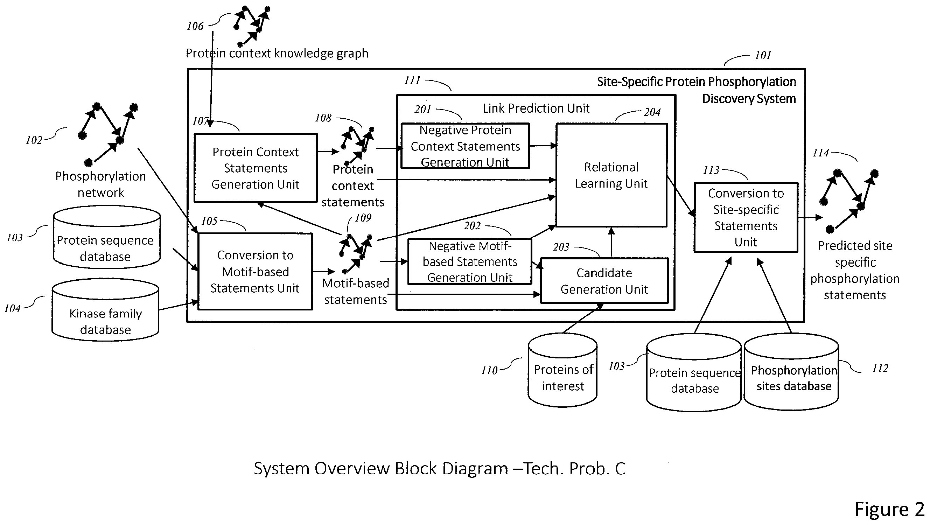

The approach of arrangements solving Technical Problem C is illustrated in Figure 2 (which gives more details of components that may be used in the Link Predictor 111 of Figure 1). Components present in the figure are as follows:

- **Negative protein context statements generation unit 201** generates negative statements based on any positive protein context statements 108, using a known subject and object corruption technique in link prediction state of the art (see, for example Maximilian Nickel et al. "A review of relational machine learning for knowledge graphs" In: arXiv preprint arXiv: 1503.00759 (2015)).
- **Negative motif-based statements generation unit 202** generates negative statements from the motif-based positive phosphorylation statements 109 using a technique according to an arrangement.
- **Candidate generation unit 203** generates a set of candidate motif-based statements related to the proteins of interest 110 (or all proteins from the kinases and substrates present in the input data), and a corresponding subset of negative candidate statements. These statements may be used for generating ranked phosphorylation predictions by relational learner 204, once a model has been learned as explained below.
- **Relational learning unit 204** is a (possibly off-the-shelf) statistical relational learning model that learns from the enriched knowledge graph (for example positive and negative motif-based statements as well as optional positive and negative protein context statements), and uses the learned model for *link prediction,* i.e., for computing scores of the candidate statements generated by candidate generator 203. The scores may be used later on for determining which of the statements are most likely to be actual phosphorylations.

The Link Predictor/Prediction Unit (111 macro-component from Figure 1) is detailed by Figure 2. There are multiple possible solutions for predicting links in graph-like knowledge bases (see for example Matt Gardner and Tom M Mitchell. "Efficient and Expressive Knowledge Base Completion Using Subgraph Feature Extraction". In: EMNLP. 2015, pp. 1488-149 and Robert West et al. "Knowledge Base Completion via Search-based Question Answering". In: Proceedings of the 23rd International Conference on World Wide Web. WWW '14. Seoul, Korea. ACM, 2014, pp. 515-526, or the approaches reviewed in Maximilian Nickel et al. "A review of relational machine learning for knowledge graphs". In: arXiv preprint arXiv: 1503.00759 (2015)). Any off-the-shelf relational learning algorithm may be used for implementing the Link Predictor 111. However, most easily applicable, accurate and tractable solutions require examples of negative statements (see Nickel et al) for both the phosphorylation and for any protein context statements 108, 109. For the protein context statements 108 that are processed by the model as is (i.e., without conversion to a different format), one strategy is to use a standard technique used in the field of link prediction in negative protein context generator 201. However, the motif-based statements 109 require a specific method 202 which is the core of an advantageous solution to Technical Problem C.

The relational learning unit 204 takes the positive and negative phosphorylation and any protein context statements as the primary input and learns a predictive model. The model may then be used to produce ranked phosphorylation statements related to an externally defined (e.g., by experts) list of proteins of interest 110. The ranked statements produced by the model are converted to the site-specific predicted phosphorylation statements 114 (by converter 113) which are the final product of the system.

## Example 3

*The converted positive statement <P04049, TKL2, Q92934> from the previous example needs to be accompanied by corresponding negative statements in order for some relational learning units to work. However, the standard corruption-based technique (see Maximilian Nickel et al) applied in traditional link prediction approaches may be unsafe in this particular case.*

*This is due to the indirect nature of the predicate* TKL[2]. *Using a standard approach common in the prior art will result in association of corrupted kinases or substrates with the complement of the positive statement: statements <X, TKL[2] Q9293> or <P04049, TKL[2] Y>, where X and Y never occur with the <TKL[2], Q9293> and <P04049, TKL[2]> (predicate,*

*object) and (subject, predicate) pairs, respectively.*

*These corrupted statements may still be positive after the reverse conversion to the site-specific format 113. For instance, a corruption <P04049, TKL², Q02750> corresponds to the site-specific statement ⟨P04049, S222, 002750⟩. However, this statement represents an actual known phosphorylation, and therefore considering its counterpart <P04049, TKL², Q02750> would result in flawing the training of the link prediction model.*

*Therefore another, more biologically secure method is proposed here for generating the negatives corresponding to the statements converted by the converter into motif-based statements 105. This approach utilises a process based on the same biological assumptions as the conversion process (explained in detail in further examples later), which addresses Technical Problem C better is possible with the prior art.*

The following sections provide details of the particular components present in the high-level Figures 1 and 2. Tables are referred to in the examples. A kinase and a substrate in the phosphorylation network 102 are denoted as "Kin" + *<number>* and "Sub" + *<number>* respectively. A protein which appears in the protein context knowledge graph 106 but does not appear in the phosphorylation network 102 is denoted as "Prot" + *<number>*. Note that these notations are for understandability and simplicity although a kinase and a substrate are also proteins, and some proteins may be both a kinase and a substrate.

**Conversion to Motif-Based Statements**

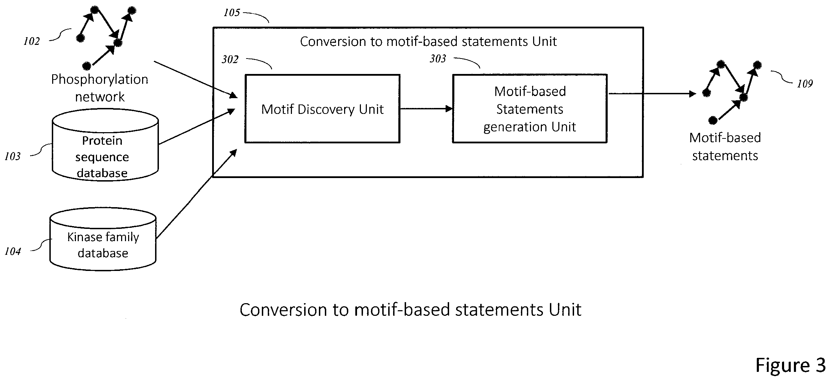

Figure 3 describes in detail the conversion to motif-based statements.

The converter 105 is a "macro component" composed of the following specific components:

- **Motif Discovery Unit 302** aims at finding position-specific scoring matrices (used as motifs) for each kinase family.
- **Motif-based Statement Generation Unit 303** converts the site-specific phosphorylation statements to (positive) motif-based statements 109 using the best conforming motif from the same kinase family.

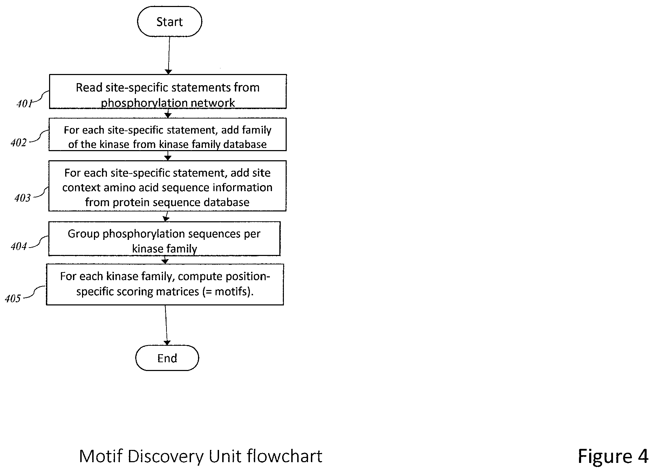

The flowchart in Figure 4 describes how an arrangement discovers motifs from site-specific statements using steps 401 to 405 described below:

- **401**. This process starts by reading all the site-specific statements of the form: ⟨*protein kinase, phosphorylation site location, protein substrate*⟩ from the phosphorylation network 102.
- **402.** Then for each statement's kinase, the system looks for its corresponding kinase family in the kinase family database 104. The information as to the kinase family is added to the site-specific statement.
- **403.** Similarly, the context interims of (left-hand-side/right-hand-side) amino acids to either side in the protein sequence (of the phosphorylation site) is added for each site-specific statement using information from the protein sequence database 103. This results in a "context sequence" of amino acids.
- **404.** The next step groups context sequences by kinase family resulting in a mapping from kinase family to a list of phosphorylation site context sequences of protein substrates phosphorylated by kinases of that family.
- **405.** The last step is to compute position-specific scoring matrices of the motifs.

The positional scoring matrix is a representation of a motif and these two terms are used interchangeably in the rest of the text. For each possible amino acid position in a motif sequence (of, say 7 to 15 positions to either side) centred on the phosphorylation site), the motif provides a score (e.g., the log likelihood score) for each amino acid at that position. More details for a position weight matrix may be found at https://en.wikipedia.org/wiki/Position_weight_matrix. An off-the-shelf bioinformatics tool may be used to compute the motifs. As a result of this process, a list of motifs is generated for each kinase family. Note that all the motifs for a kinase family may be processed at once and that a number of batches created by dividing the motifs may be processed separately (results being merged). For creating batches, the motifs may be divided by a prefixed size of a batch, may be divided by the protein (one alphabet using the single letter code) of the phosphorylation site, and or both may be combined.

**Example 4. Motif Discovery Unit**

*Step 401 reads the site-specific statements from the phosphorylation network as*

**[Table 1] Phosphorylation Table**

| Kinase | Phosphorylation site | Substrate |
|--------|---------------------|-----------|
| Kin00 | S99 | Sub00 |

(continued)

| Kinase | Phosphorylation site | Substrate |
|---|---|---|
| Kin00 | S619 | Sub01 |
| Kin01 | S211 | Sub02 |
| Kin02 | T62 | Sub03 |
| ... | ... | ... |

and creates the Augmented Phosphorylation Table 2 with a statement ID (abbreviated as "stmt. id"), an empty kinase family, and an empty context sequence (to be filled later). As shown in Table 1, a kinase may phosphorylate more than one substrate in general. A kinase may phosphorylate more than one site on the same substrate in general (not illustrated). For example, for the first record in Table 1, the first record in Table 2 is created with the statement id "000"

[Table 2] Augmented Phosphorylation Table

| Stmt. id | Kinase | Kinase family | Phosphorylation site | Substrate | Context sequence |
|---|---|---|---|---|---|
| 000 | Kin00 | | S99 | Sub00 | |
| 001 | Kin00 | | S619 | Sub01 | |
| 002 | Kin01 | | S211 | Sub02 | |
| 003 | Kin02 | | T62 | Sub03 | |
| ... | ... | | ... | ... | |

Step 402 refers to Kinase Family Table 3 in the kinase family database (104), and for each record in Augmented Phosphorylation Table 2, fills into the "kinase family" cell a kinase family corresponding to the kinase in the "kinase" cell.

[Table 3] Kinase Family Table

| Kinase | Kinase family |
|---|---|
| Kin00 | TKL |
| Kin01 | TKL |
| Kin02 | AGC |
| ... | ... |

For example, for the first record (with the statement id "000") in Table 2, because the kinase is "Kin00" and its family is "TKL" derived from Table 3, "TKL" is filled into the "kinase family" cell. Finally, Augmented Phosphorylation Table 2 becomes Augmented Phosphorylation Table 4.

[Table 4] Augmented Phosphorylation Table

| Stmt. id | Kinase | Kinase family | Phosphorylation site | Substrate | Context sequence |
|---|---|---|---|---|---|
| 000 | Kin00 | TKL | S99 | Sub00 | |
| 001 | Kin00 | TKL | S619 | Sub01 | |
| 002 | Kin01 | TKL | S211 | Sub02 | |
| 003 | Kin02 | AGC | T62 | Sub03 | |
| ... | ... | ... | ... | ... | |

Step 403 refers to Protein Sequence Table 5 in the protein sequence database (103), and for each record in Augmented Phosphorylation Table 4, fills the "context sequence".

**[Table 5] Protein Sequence Table**

| Protein | Sequence |
|---------|----------|
| Sub00 | MFQIPEFEPS EQEDSSSAER GLGPSPAGDG FSGSGKHHRQ APGLLWDASH QQEQPTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWDRNLG RGSSAPSQ |
| ... | ... |

*Processing of the first record (with the statement id "000") in Table 4 is shown as a concrete example as follows. Because the substrate is "Sub00", the record with the protein "Sub00" is looked up and the sequence "MFQIPEFEPS EQEDSSSAER GLGPSPAGDG PSGSGKHHRQ APGLLWDASH QQEQPTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWDRNLG RGSSAPSQ" (a space is inserted by every 10 characters for visibility) is obtained. Because the phosphorylation site is "S99", the site context around the 99th character "S" is extracted. Any length may be possible, but here the left 7 characters and the right 7 characters (and the 99th character itself) are defined as a context (relevant part of the sequence). In this example, the sequence from the 92nd character and the 106th character, that is, "PFRGRSRSAPPNLWA" (underlined), is extracted and filled into the "context sequence" cell. Finally, Augmented Phosphorylation Table 4 becomes Augmented Phosphorylation Table 6.*

**[Table 6] Augmented Phosphorylation Table**

| Stmt. id | Kinase | Kinase family | Phosphorylation site | Substrate | Context sequence |
|----------|--------|---------------|----------------------|-----------|------------------|
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S819 | Sub02 | SLPKINRSASEPSLH |
| 003 | Kin02 | AGC | T62 | Sub03 | RRPESFTTPEGPKPR |
| ... | ... | ... | ... | ... | ... |

*Step 404 groups context sequences by each kinase family and creates Context Sequences Table 7.*

**[Table 7] Context sequences Table**

| Kinase family | Context sequences |
|---------------|-------------------|
| TKL | {PFRGRSRSAPPNLWA, SLPKINRSASEPSLH, ... } |
| AGC | {RRPESFTTPEGPKPR, ...} |
| ... | ... |

*For example, for the kinase family "TKL", the context sequences in the first three records (with the statement ids "000", "001", and "002") are grouped. Note that there may be more records in the whole table though there are only three records in the exemplified records. As a result, "FPFRGRSRSAPPNLWA, SLPKINRSASEPSLH, ...}" is obtained for "TKL" and is stored in Context Sequences Table 7 as shown in the first record.*
*Step 405 computes motifs for each kinase family and creates Motif Table 8.*

**[Table 8] Motif Table**

| Kinase family | Modif id | PSSM | | | | | | | | | | | | | | | | | | | | |
|---|---|---|---|---|---|---|---|---|---|---|---|---|---|---|---|---|---|---|---|---|---|---|
| | | | A | C | D | E | F | G | H | I | K | L | M | N | P | Q | R | S | T | V | W | Y |
| TKL | TKL0 | 1 | 1 | 66 | 4 | -22 | 54 | 32 | 28 | -137 | 89 | 47 | -45 | 57 | 37 | 59 | -29 | -72 | -115 | 10 | 45 | 30 |
| | | 2 | 1 | 6E | -16 | 6 | 29 | -4 | -2 | 22 | 48 | 6 | -134 | 44 | 18 | 10 | 35 | -29 | -64 | -58 | 45 | -39 |
| | | 3 | -14 | -124 | 3 | as | -96 | -20 | 72 | 46 | -68 | -24 | 29 | -25 | 45 | -7 | 93 | -55 | -52 | 22 | -379 | -2 |
| | | 4 | 12 | 24 | 36 | -61 | 74 | 19 | 51 | -11 | 46 | 5 | 79 | 55 | -8 | 46 | -92 | -65 | -53 | -41 | -390 | -3 |
| | | 5 | 34 | 476 | 42 | -26 | -4 | 30 | -42 | 46 | 10 | 14 | 47 | -5 | 36 | -45 | 44 | -39 | -53 | 8 | -390 | -3 |
| | | 6 | 21 | 63 | 34 | 2 | 25 | -40 | 24 | 18 | 18 | 48 | 46 | -52 | -10 | 6 | 69 | 0 | -87 | -90 | 429 | 73 |
| | | 7 | 10 | 63 | 34 | -12 | -6 | 57 | -98 | -32 | 18 | 12 | 46 | 41 | -38 | 33 | -52 | -20 | -44 | 6 | 42 | -100 |
| | | 8 | -551 | -637 | -885 | -952 | -686 | -693 | -832 | -903 | -988 | -908 | -896 | -820 | -873 | -902 | -925 | 207 | 238 | -770 | -642 | 76 |
| | | 9 | -16 | -33 | -19 | 27 | 44 | 18 | 50 | -30 | -12 | -5 | 28 | 28 | 35 | 34 | -121 | -5 | -30 | 20 | -402 | 52 |
| | | 10 | 23 | -127 | -19 | 27 | -44 | 6 | -5 | 45 | 10 | -26 | -88 | -50 | 53 | -26 | 53 | 7 | -102 | 7 | 43 | 4 |
| | | 11 | 63 | 65 | 41 | 5 | 109 | -6 | 27 | -10 | -108 | -24 | -17 | -5 | -20 | 23 | -15 | -31 | -66 | 74 | 140 | 75 |
| | | 12 | 26 | -118 | 52 | 7 | -92 | -51 | -1 | 8 | 40 | 41 | -81 | 15 | 4 | 4 | 1 | -26 | 49 | 11 | 198 | -38 |
| | | 13 | -26 | -27 | 6 | 8 | 0 | 22 | 53 | 45 | 33 | 26 | -80 | -1 | -4 | -88 | 26 | -51 | 22 | 67 | -342 | -88 |
| | | 14 | -42 | 98 | -14 | 31 | -38 | -3 | -329 | 70 | 14 | 74 | 32 | -71 | -135 | 60 | 2 | -69 | -4 | 36 | 46 | -168 |
| | | 15 | 47 | -117 | 88 | 99 | -38 | 10 | 29 | -7 | -63 | 34 | 50 | 58 | 4 | 12 | -12 | -115 | -62 | -56 | -342 | -168 |
| TKL | TKL1 | ... | | | | | | | | | | | | | | | | | | | | |
| TKL | ... | ... | | | | | | | | | | | | | | | | | | | | |
| RGC | AGC0 | ... | | | | | | | | | | | | | | | | | | | | |
| | ... | | | | | | | | | | | | | | | | | | | | | |

*If no sequences are present for a family, no motifs are generated. Here a motif is represented as a position-specific matrix (PSSM) but any other form may be adopted as long as motifs are defined and the score between each motif and a context sequence may be calculated. In this example, MEME (see htto://meme-suite.org) is used. MEME is a tool which inputs a set of sequences of the same length (in this usage according to arrangements; generally the input of MEME may be a set of sequences of various lengths) and outputs a set of motifs. In essence, the algorithm tries to find a pattern in the frequencies of occurrences of specific amino acids in particular locations in the input sequences in order to give a generalisation of the batch of sequences in the form of a convenient motif representation. The EM algorithm is used to find a reasonably optimal pattern of frequency distributions by trying to find a solution as far away from random noise as possible. An authoritative description from the tool authors can be found in the following paper and in related references: https://academic.oup.com/nar/article/37/suppl_2/VV202/1135092/MEME-Suite-tools-formotif-discovery-and-searching.*

*Each motif is a position-specific matrix. A position-specific matrix is a matrix of 20 columns (for 20 letters of the protein alphabet) and rows. The number of rows is equal to the length of the context sequence (again in this usage; generally the rows of the matrix may be of arbitrary various lengths), that is, 15 here (7 to either side of the phosphorylation site). Each cell represents a score. The meaning of the scores and the usage of the matrix will be shown in a later example.*

*In Motif Table 8, for example, for "TKL", several motifs are generated by MEME, or another suitable tool.*

*The motifs are discovered by MEME. However, the number and granularity of the motifs per each family are influenced by two hyperparameters of the motif extracting tool (at least in the current arrangement): the size of the context window and the number of sequences in randomised samples that are processed by MEME at once.*

*The number of motifs discovered per family cannot be directly set by the user - the actual number depends on the data, and also on the values of the context window and the number of sequences in randomised samples, but these are reflected only very indirectly. There is no user defined confidence threshold - the MEME algorithm is based on expectation maximisation and this statistical method finds the optimal motifs without external parameters once a sufficiently high number of good motifs is specified (as in the current arrangement, to make sure all optimal motifs found are included). 10 may be used in the current arrangement, and typically there may be only up to 5 motifs found for any combination of hyperparameters used to run the experiments.*

*Each motif is attached a motif id such as "TKLO" and "TKL1". In the "PSSM" row, a PSSM is filled for a motif id (only the PSSM for "TKLO" is shown).*

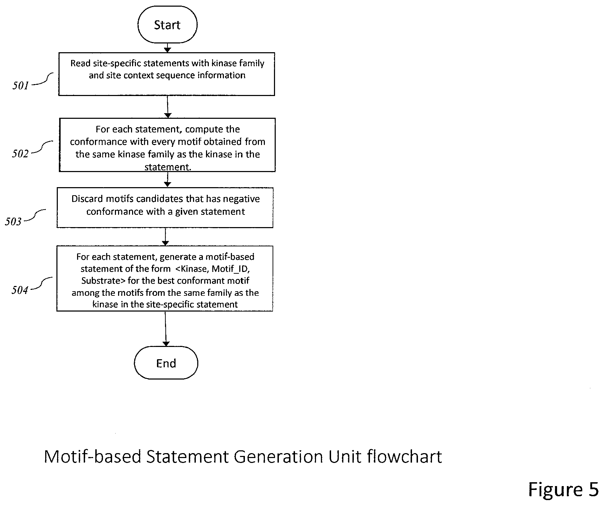

The flowchart in Figure 5 describes how site specific statements are converted in motifbased statements:

- **501.** This process starts by reading all the site-specific statements enriched with the kinase family and phosphorylation site context sequence information. The resulting statements are in the form:
  ⟨*kinase, kinase family, substrate, phosphorylation site context sequence*⟩.
- **502.** Then for each statement's context sequence, the system computes the conformance between the given context sequence and every motif discovered for the family of the kinase in the statement. A conformance score is obtained using the following function:

$$score(S, M) = \sum_{i \in \{1,...,|S|\}} M_{i,id(S_i)},$$

where *S, M* is the sequence (represented as a linear chain, i.e., vector) and the motif scoring matrix, respectively. *id* is a function mapping the letters of the protein alphabet to the corresponding column indices in the motif matrix. For each pair of ⟨*statement, phosphorylation motif of the same kinase family*⟩ a conformance score is given.

- **503.** Only pairs of
  ⟨*statement, phosphorylation motif of the same kinase family*⟩ with a positive conformance score are kept. Negative conformance means there is no conformance and therefore the motif cannot be considered as representing the statement context sequence.
- **504.** For each site-specific statement, a motif-based statement is created by selecting the best performing (in relation to the conformance score) motif. This process therefore generates a list of positive (positive in the sense that they actually occur in real life) motif-based statements of the form:
  ⟨*protein kinase, motif, protein substrate*⟩.

These statements are not context dependent anymore, as the motif-based predicate generalises the context information in a way that may be compared across multiple phosphorylation statements (which is a necessary condition for consequent link prediction).

**Example 5. Motif-Based Statement Generation Unit**

*Step 501 reads Augmented Phosphorylation Table 6 created in the previous example.*

**[Table 6] (reprinted) Augmented Phosphorylation Table**

| Stmt. id | Kinase | Kinase family | Phosphorylation site | Substrate | Context sequence |
|----------|--------|---------------|----------------------|-----------|------------------|
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S619 | Sub02 | SLPKINRSASEPSLH |
| 003 | Kin02 | AGC | T62 | Sub03 | RRPESFTTPEGPKPR |
| ... | ... | ... | ... | ... | ... |

*For each record in Table 6 and each motif of the record's kinase family in Motif Table 8 created in the previous example, Step 502 computes a conformance score and stores a corresponding record in Conformance Score Table 9.*

**[Table 9] Conformance Score Table**

| Stmt. id | Motif id | Conformance score |
|----------|----------|-------------------|
| 000 | TKL0 | 528 |
| 000 | TKL1 | -35 |
| 000 | TKL2 | 187 |
| ... | ... | ... |

*Concretely, suppose the first record with the statement id "000" in Table 6 is processed. Because the kinase family is "TKL", the motifs whose kinase family is "TKL" in Table 8 are processed. Here the calculation details are explained for "TKLO".*

*The meaning of a PSSM corresponding to a kinase family is as follows. First, the meaning of a score in a cell:*

- *If a score in a column i and a row X is greater than 0, then the likelihood of an amino acid X occurring on the position i in a sequence corresponding to the kinase family in question is greater than it would be in a purely random case (i.e., the kinase family tends to prefer the amino acid at that position).*
- *If a score in a column i and a row X is equal to 0, then the occurrence of an amino acid X on the position i cannot be distinguished from a random case (i.e., the kinase family does not have any preference for the amino acid at that position).*
- *If a score in a column i and a row X is less than 0, then the likelihood of an amino acid X occurring on the position i in a sequence corresponding to the kinase family in question is lower than it would be in a purely random case (i.e., the kinase family tends to stay away from the amino acid at that position).*

*For example, some cells of the PSSM for the motif id "TKLO" mean:*

- *The score in the column "3" and the row "R" is 93, which is greater than 0. Therefore, if the third character of a sequence is "R", the sequence is positively associated with the motif in its third letter.*
- *The score in the column "6" and the row "S" is 0. Therefore, if the sixth character of a sequence is "S", the association of the sequence at the sixth letter with the motif cannot be determined as it is about as likely as random.*
- *The score in the column "9" and the row "A" is -16, which is lower than 0. Therefore, if the ninth character of a sequence is "A", the sequence is disassociated with the motif at the ninth letter.*

*A conformance score of a sequence is calculated by summing up the scores of all the positions in the sequence - this corresponds to the likelihood of the sequence's conformance to the motif across all the sequence letters. In the current example, the input sequence is "PFRGRSRSAPPNLWA" (the one for the statement id "000"). The conformance score is calculated by summing up the 15 shaded scores in Table 10.*

## [Table 10] PSSM

|  | A | C | D | E | F | G | H | I | K | L | M | N | P | Q | R | S | T | V | W | Y |
|---|---|---|---|---|---|---|---|---|---|---|---|---|---|---|---|---|---|---|---|---|
| 1 | 1 | 66 | 4 | -22 | 54 | 32 | 28 | -137 | 89 | -47 | -45 | 57 | 37 | 59 | -29 | -72 | -115 | 10 | 45 | 30 |
| 2 | 1 | 66 | -16 | 6 | 29 | -4 | -2 | 22 | 48 | 8 | -134 | 44 | 18 | 10 | 35 | -29 | -64 | -58 | 45 | -39 |
| 3 | -14 | -124 | 3 | 49 | -96 | -20 | 72 | 46 | -68 | -24 | 29 | -25 | 45 | -7 | 93 | -55 | -52 | 22 | -379 | -2 |
| 4 | 12 | 24 | 36 | -61 | 74 | 19 | 51 | -11 | 46 | 5 | 79 | 55 | -8 | 46 | -92 | -65 | -53 | -41 | -390 | -3 |
| 5 | 34 | -476 | -42 | -25 | -4 | 30 | -42 | 46 | 10 | 14 | 47 | -5 | 36 | -45 | 44 | -39 | -53 | 8 | -390 | -3 |
| 6 | 21 | 63 | 34 | 2 | 25 | -40 | 24 | 18 | 18 | -40 | 46 | -52 | -10 | 6 | 69 | 0 | -87 | -90 | -429 | 73 |
| 7 | 10 | 63 | 34 | -12 | -6 | 57 | -98 | -32 | 18 | 12 | 46 | 41 | -38 | 33 | -52 | -20 | -44 | 6 | 42 | -100 |
| 8 | -551 | -637 | -885 | -952 | -686 | -693 | -832 | -903 | -988 | -908 | -896 | -820 | -873 | -902 | -925 | 207 | 238 | -770 | -642 | 76 |
| 9 | -16 | -33 | -19 | 27 | -44 | 18 | 50 | -30 | -12 | -5 | 28 | 28 | 35 | 34 | -121 | -5 | -30 | 20 | -402 | 52 |
| 10 | 23 | -127 | -19 | 27 | -44 | 6 | -5 | 45 | 10 | -26 | -88 | -50 | 53 | -26 | 53 | 7 | -102 | 7 | 43 | -4 |
| 11 | 63 | 65 | -41 | 5 | 109 | -6 | 27 | -10 | -108 | -24 | -17 | -5 | -20 | 23 | -15 | -31 | -66 | 74 | 140 | 75 |
| 12 | 26 | -118 | 52 | 7 | -92 | -51 | -1 | 8 | 40 | 41 | -81 | 15 | -4 | -4 | 1 | -28 | -49 | 11 | 198 | -38 |
| 13 | -26 | -27 | 6 | 8 | 0 | 22 | 53 | -45 | 33 | 26 | -80 | -1 | -4 | -88 | 26 | -51 | 22 | 67 | -342 | -88 |
| 14 | -42 | 98 | -14 | 31 | -38 | -3 | -329 | 70 | 14 | 74 | 32 | -71 | -135 | 60 | 2 | -69 | -4 | 36 | 46 | -168 |
| 15 | 47 | -117 | 88 | 99 | -38 | 10 | 29 | -7 | -63 | 34 | 50 | 58 | -4 | 12 | -12 | -115 | -62 | -56 | -342 | -168 |

For example, since the first character is "P", the cell value "37" in the column "1" and the row "P" is shaded. Similarly, since the second character is "F", the cell value "29" in the column "2" and the row "F" is shaded. By calculation of "37 + 29 + 93 + 19 + 44 + 0 - 52 + 207 - 16 + 53 - 20 + 15 + 26 + 46 + 47", the conformance score is obtained as 528. This score is stored in Table 9 as shown previously.

Similarly, suppose other conformance scores are calculated as shown in Table 9. That is, the conformance score for the statement id "000" and the motif id "TKL1" is -35, and the one for "000" and "TKL2" is 187.

Step 503 discards records with a negative conformance score in Conformance Score Table 9. In the current example, the second record has the negative conformance score "-35". As a result of discard, Conformance Score Table 9 becomes Conformance Score Table 11. Here the threshold for discard was zero but any arbitrary value may be used or with a specified value N the top N may be selected.

### [Table 11]

| Conformance Score Table | | |
|---|---|---|
| Stmt. id | Motif id | Conformance score |
| 000 | TKL0 | 528 |
| 000 | TKL2 | 187 |
| ... | ... | ... |

For each statement id in Augmented Phosphorylation Table 6, Step 504 generates a ⟨Kinase, Motif id, Substrate⟩ statement for the motif with the best conformance score for the statement id, and stores it in Positive Motif-based Statement Table 12.

### [Table 12]

| Positive Motif-based Statement Table | | |
|---|---|---|
| Subject (Kinase) | Predicate (Motif id) | Object (Substrate) |
| Kin00 | TKL0 | Sub00 |
| ... | ... | ... |

If there are no positive conformance scores, no converted statements are generated. In the current example,

*there are two records in Positive Motif-based Statement Table 11 for the statement id "000". Since the conformance score 528 of the first record is greater than the conformance score 187 of the second record, the former is selected, that is, the motif id "TKLO" is selected. As a result, ⟨Kin00, TKL0, Sub00⟩ is generated as shown in Positive Motif-based Statement Table 12.*

**Enriching the Knowledge Base by Protein Contextual Information**

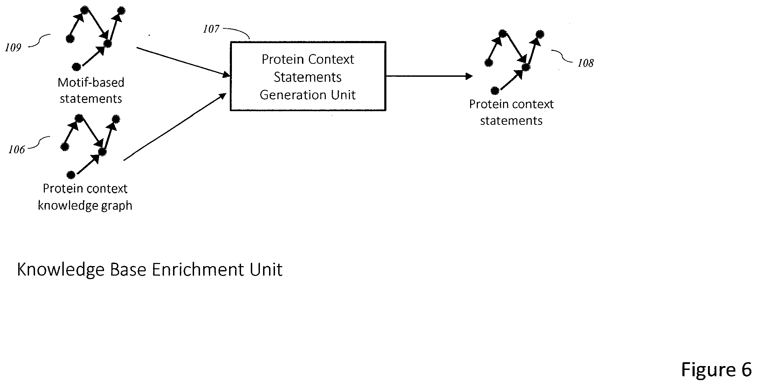

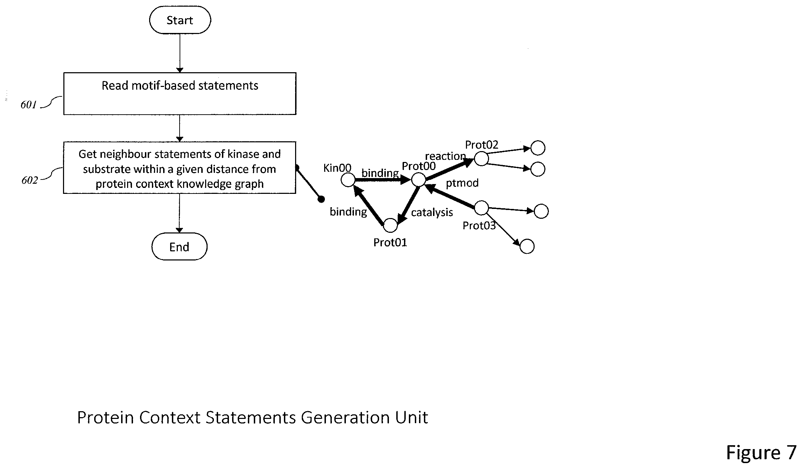

Figures 6 and 7 describe in detail the Protein Context Statement Generation Unit 107. Figure 6 includes the relevant parts already described in Figure 2. The flowchart of Figure 7 composed of the following specific steps:

- **601.** This process starts by reading all positive motif-based statements 109 of the form:
  ⟨*protein kinase, motif, protein substrate*⟩.
- **602.** Then, the system uses the protein context knowledge graph 106 to extract neighbour statements in the knowledge graph within a given distance from the protein kinases and substrates contained in the positive motif-based statements. As a result protein context statements 108 are created. The statements can be optionally used for improving the predictive capability of the model by adding more knowledge (i.e., relationships / statements) about the proteins appearing in phosphorylations. This addition is easy with the method of arrangements, while the prior art either does not allow inclusion of this extra information or allows it in a very superficial way (just for filtering predictions, not for actually computing them).

**Example 6. Protein Context Statements Generation Unit**

*Step 601 reads motif-based statements in Positive Motif-based Statement Table 12.*
*Step 602 first reads Protein Context Knowledge Graph ProCSGU-1 (a simplified example is shown in Figure 7). For visibility, the graph is represented as a graph, not as a table. A node represents a protein, and an arrow represents a relation between the connecting two proteins. Step 602 then, for each protein (kinase, substrate) in Positive Motif-based Statement Table 12, retrieves neighbour statements of the protein within a given distance and stores them in a table. Suppose the distance is set as 2 and the current protein is "Kin00". For retrieving all the concerned statements, arbitrary graph search methods may be used. Since "Prot00" and "Prot01" are one step away from "Kin00" and "Prot02" and "Prot03" are two steps away from "Kin00", the five relations with a bold arrow are retrieved. The resulting table after processing only "Kin00" is Positive Protein Context Statement Table 13. For example, the "ptmod" arrow from "Prot03" to "Prot00" is stored as the record ⟨Prot03, ptmod, Prot00⟩.*

**[Table 13]**

| Positive Protein Context Statement Table | | |
|---|---|---|
| **Subject** | **Predicate** | **Object** |
| Kin00 | binding | Prot00 |
| Prot01 | binding | Kin00 |
| Prot00 | catalysis | Prot01 |
| Prot00 | reaction | Prot02 |
| Prot03 | ptmod | Prot00 |

**Link Prediction Unit**

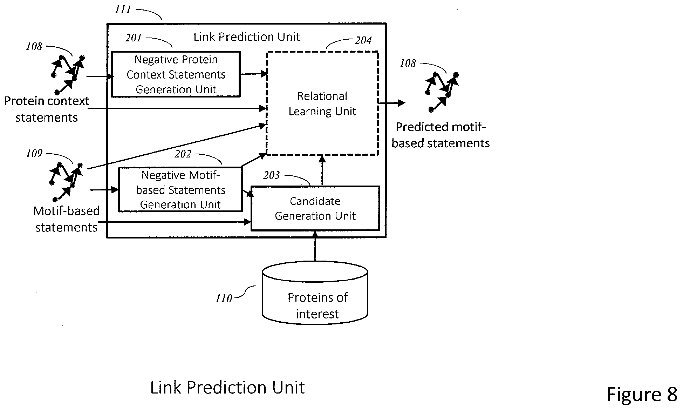

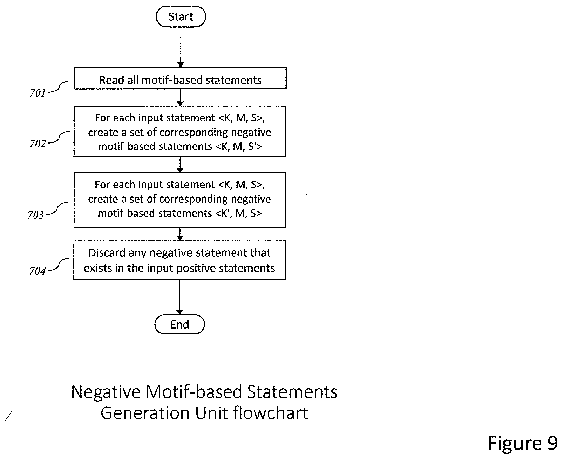

The details of the Link Prediction Unit 111 are depicted in Figure 8, which includes the relevant parts already described for Figure 2, and the output in the form of predicted motifbased statements.
One important part of link predictor 111 in the context of some arrangements is the generation of the motif-based negative statements, detailed in Figure 9.
The flowchart of the negative motif-based statements generation unit 202 is as follows:

- **701.** This process starts by reading all positive motif-based statements 109.
- **702.** For each statement having the form:

⟨*protein kinase K, motif M, protein substrate S*⟩.
the system creates a set of corresponding negative motif-based statements from substrates (substrate corruptions, or substrates which are deemed not to allow phosphorylation at the given motif and using the given kinase) that are merged into the final negative set:

$$- \{(K, M, S') \mid \forall L \in sites(S').\ score(sequence(L, S', \frac{|M|-1}{2}), M) < 0\},$$

where *sites(X)* is a function returning a set of phosphorylation sites of substrate *X* for all sites *S'* at Location *L* with the score as defined. The function score is the motif-based scoring function defined in the description of motif discoverer 302 above. The *sequence (U, V, k)* returns the site *U* context of size *k* within *V*, i.e., a symmetric sequence of *k* amino acids on the left and right side of the site *U* in the sequence of the substrate *V*, including the site itself in the centre. We use |*M*| to indicate the width of the motif |*M*| (i.e., the number of amino acids in a sequence to be meaningfully compared against the motif). Currently arrangements use 15 and 31 which correspond to the sequence context sizes 7 and 15 as hyperparameters. A context size of 7, for example, refers to including 7 amino acids to either side of the phosphorylation site and the phosphorylation site itself. More particularly, 15 corresponds to 1 (for the amino acid which is the actual site) + 7 +7 (7 amino acids on left and right). 31 corresponds to 15 in a similar way.

In essence, for a substrate that is not S, the context sequences are found for the same kinase and checked for conformance with Motif *M*. If this is negative, then a statement <K, M, S'> is stored as a negative statement. In the code the negatives may be generated later on the fly (rather than individually as the context sequences are found) to make the computation more efficient).

- **703**. For each statement having the form:

  ⟨*protein kinase K, motif M, protein substrate S*⟩.
  the system creates a set of corresponding negative motif-based statements from kinases (kinase corruptions or kinase which are deemed not to allow phosphorylation at the given motif and with the given substrate) and merges it to the final set by:

$$- \{(K', M, S) \mid family(K') \neq family(K)\},$$

where *family(X)* is a function returning a family of kinase X.

- **704.** Any negative motif-based statement created that already exists in the positive motif-based statements is removed. The output of this process is a list of negative motif-based statements generated from the positive ones.

The rationale behind Step 702 and Step 703 is three-fold:

- First, it is important to make sure that the negatives follow the local Closed World Assumption (see Nickel et al), i.e., that the predicate is the same as in the positive triple and that a corruption occurs either at the subject (kinase) or at the object (substrate) position, but never at both. This is necessary for the relational learning unit (204) to work correctly.
- The corrupted substrates should not be compatible with the predicate motif in order for the resulting triple to be negative with a high confidence even after it is converted back to the site-specific format. A natural way of achieving that is to make sure that the score of all sites in the corruption substrate is lower than zero with respect to the predicate motif of the positive triple. This means that the corruptions will have negative likelihood of being associated with the predicate based on the actual site information.
- Similarly, the corrupted kinases should also be incompatible with the predicate motif. This may be enforced by using kinases that are from a family different than the family of the kinase in the positive statement. This will make the corrupted kinases incompatible with the positive predicate as the kinase families are mutually disjoint and the motifs are always computed from sequences within a single family.

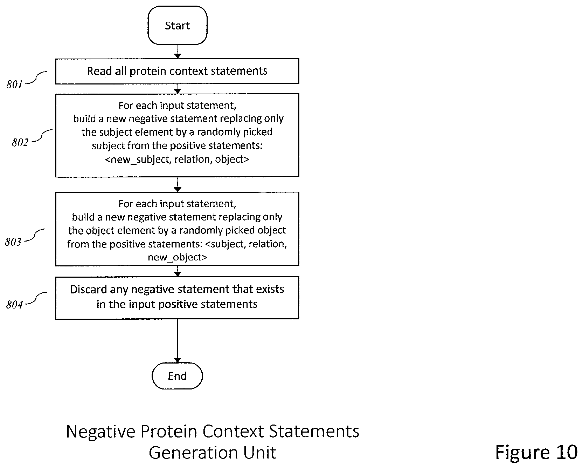

The flowchart in Figure 10 describes the process of creation of negative protein context statements 201 from positive ones.

**Negative Protein Context Statements Generation Unit**

The process is as follows:

- **801.** This process starts by reading all positive protein context statements 108 from Table 13shown previously.
- **802.** For each statement of the form:

  ⟨subject S, relation P, object O⟩
  the system creates new negative statements replacing only the subject element by a randomly picked subject among subjects that never occur with relation P and
  object O in the positives.

- **803.** For each statement of the form:

  (subject S, relation P, object O)
  the system creates new negative statements replacing only the object element by a randomly picked object among objects that never occur with subject S and relation P in the positives.

- **804.** Finally, all generated negative statements present in the positive protein context statements are removed.

The protein context statements (both negative and positive) may enrich the knowledge graph and thus lead to better accuracy of the phosphorylation prediction. If the model has richer information on what may possibly be related to the kinases and substrates (for instance, via other types of protein interaction relations or publication-based cooccurrences), it may very often spot many more implicit regularities in the data and use them for better predictions.

**Example 7. Negative Motif-based Statements Generation Unit**

*Step 701 reads all the motif-based statements in Positive Motif-based Statement Table 12. Step 702 creates negative statement records for each positive statement record in Table 12 and stores them in Negative Motif-based Statement Table 15. Here the first record ⟨Kin00, TKL0, Sub00⟩ is used for explanation.*

**[Table 14] (enriched version of Table 6)**

| Augmented Phosphorylation Table | | | | | |
|---|---|---|---|---|---|
| **Stmt. id** | **Kinase** | **Kinase family** | **Phosphorylation site** | **Substrate** | **Context sequence** |
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S619 | Sub02 | SLPKINRSASEPSLH |
| 003 | Kin02 | AGC | T62 | Sub03 | RRPESFTT PEGPKPR |
| ... | ... | | ... | ... | ... |
| 100 | Kin100 | AGC | S489 | Sub10 | SDSEDDSSEFDEDDW |
| 101 | Kin11 | CAMK | S484 | Sub10 | IAVEYSDSEDDSSEF |
| 102 | Kin12 | AGC | T276 | Sub11 | LGGGRVKTWKRRWFI |
| 103 | Kin13 | CAMK | S392 | Sub11 | AARKKRISVKKKQEQ |
| ... | ... | ... | ... | ... | ... |

*Step 702 processes the following sub-steps for all the possible substrates in Augmented Phosphorylation Table 14 (or Table 12; either may be used). Table 14is an enriched version of Table 6, containing records (with the statement ids "100", "101", "102", and "103") required for explanation here.*

*In the explicitly described records in Table 14, the substrates are "Sub00", "Sub01", "Sub02", "Sub03", "Sub10", and "Sub11". In the following explanation, "Sub 10" and "Sub11" are used.*
*• "Sub10"*

◦ In Sub-step 702-1, all the possible context sequences (e.g., sequence around a site where phosphorylation has actually occurred for that substrate) for "Sub10" are spotted. They are "SDSEDDSSEFDEDDW" in the record of "100" and "IAVEYSDSEDDSSEF" in the record of "101".

◦ Recall that the first record <Kin00, TKLO, Sub00> is the starting point, and only the substrate is changed. In Sub-step 702-2, for each spotted context sequence, the conformance score for the motif "TKLO" is calculated. In the same way as Step 502 with Motif Table 8, the conformance scores of "SDSEDDSSEFDEDDW" and "IAVEYSDSEDDSSEF" are calculated as -426 and -56 respectively.

◦ In Sub-step 702-3, whether all the calculated conformance scores are negative or not is checked. Since both -426 and -56 are negative, the answer is yes and a negative statement is generated whose kinase and motif id are the same as in the positive statement and whose substrate is the concerned substrate. Concretely expressed here, the kinase and motifs are "Kin00" and "TKLO" in the first record in Table 12, and the substrate is "Sub10". As a result, the first record in Negative Motif-based Statement Table 15 is stored.

**[Table 15]**

| Negative Motif-based Statement Table | | |
|---|---|---|
| **Subject (Kicase)** | **Predicate (Motif id)** | **Object (Substrate)** |
| Kin00 | TKL0 | Sub10 |
| Kin02 | TKL0 | Sub00 |
| ... | ... | ... |

• "Sub11"

◦ In Sub-step 702-1, all the possible context sequences for "Sub11" are spotted. They are "LGGGRVKTWKRRWFI" in the record of "102" and "AARKKRISVKKKQEQ" in the record of "103".

◦ In Sub-step 702-2, for each spotted context sequence, the conformance score for the motif "TKLO" is calculated. In the same way as Step 502 with Motif Table 8, the conformance scores of "LGGGRVKTWKRRINFI" and "AARKKRISVKKKQEQ" are calculated as -635 and 312 respectively.

◦ In Sub-step 702-3, whether all the calculated conformance scores are negative or not is checked. Since 312 is positive, the answer is no and no negative statement is stored.

Step 703 also creates negative statement records for each positive statement record in Table 12 and stores them in Negative Motif-based Statement Table 15. Here the first record ⟨Kin00, TKL0, Sub00⟩ is used for explanation.

Step 703 processes the following sub-steps for all the possible kinases in Kinase Family Table 3 (or Augmented Phosphorylation Table 14; whichever may be used). In the explicitly described records in Table 3, the kinases are "Kin00", "Kin01", and "Kin02". In the following explanation, "Kin01" and "Kin02" are used.

For "Kin01", whether "Kin01" and the positive statement's kinase "Kin00" belong to a same kinase family is checked by referring to Kinase Family Table 3. Since both belong to "TKL", the answer is yes and no negative statement is stored.

For "Kin02", whether "Kin02" and the positive statement's kinase "Kin00" belong to a same kinase family is checked by referring to Kinase Family Table 3. Since the former belongs to "AGC" and the latter belongs to "TKL", the answer is no and a negative statement is stored whose motif id and substrate are the same as in the positive statement and whose kinase is the concerned kinase. Concretely, the motifs and substrate are "TKLO" and "Sub00" in the first record in Table 12, and the kinase is "Kin02". As a result, the second record in Negative Motif-based Statement Table 15 is stored.

Step 704 discards from Negative Motif-based Statement Table 15 all the records appearing in Table 12. In the current example, there is no such record and Table 15 is kept as it is.

**Example 8. Negative Protein Context Statements Generation Unit**

Step 801 reads all the (positive) protein context statements in Positive Protein Context Statement Table 13.

Step 802 creates negative protein context statements for each positive protein context statement and stores them in Negative Protein Context Statement Table 16.

**[Table 16]**

| Negative Protein Context Statement Table | | |
|---|---|---|
| **Subject** | **Predicate** | **Object** |
| Prot00 | binding | Prot00 |
| Prot01 | binding | Prot00 |
| Kin00 | binding | Kin00 |
| Kin00 | binding | Prot01 |
| Kin00 | binding | Prot02 |
| ... | ... | ... |

Here the first record ⟨Kin00, binding, Prot00⟩ is used for explanation. This step first collects all the possible subjects for each (SUBJECT) of which there is no record (SUBJECT, binding, Prot00) (the predicate and the object are the same as in the positive). They are "Prot00" and "Prot01". Note that "Prot03" in the fifth record is not included because the statement violates the condition. In the following, all the subjects may be used, and some of them may be selected randomly or by any other method. Suppose both "Prot00" and "Prot01" are selected. This step then stores a record (SUBJECT, binding, Prot00) for each subject into Negative Protein Context Statement Table 16. In the current example, the first two records in Table 16 are stored. Here the subject and the object in the first record are the same but such a record may be excluded for all the cases or for some specified predicates.

Step 803 also creates negative protein context statements for each positive protein context statement and stores them in Negative Protein Context Statement Table 16. Here the first record ⟨Kin00, binding, Prot00⟩ is used for explanation. This step first collects all the possible objects for each (OBJECT) of which there is no record (Kin00, binding, OBJECT) (the subject predicate and the predicate are the same as in the positive). They are "Kin00", "Prot01", and "Prot02". In the following, all the objects may be used, and some of them may be selected randomly or by any other method. Suppose all of "Kin00", "Prot01" and "Prot02" are selected. This step then stores a record ⟨Kin00, binding, OBJECT⟩ for each object into Table 16. In the current example, the three records from the third to the fifth in Negative Protein Context Statement Table 16 are stored. Here the subject and the object in the third record are the same but such a record may be excluded for all the cases or for some specified predicates.

Step 804 discards from Negative Protein Context Statement Table 16 all the records appearing in Positive Protein Context Statement Table 13. In the current example, there is no such record and Table 16 is kept as it is.

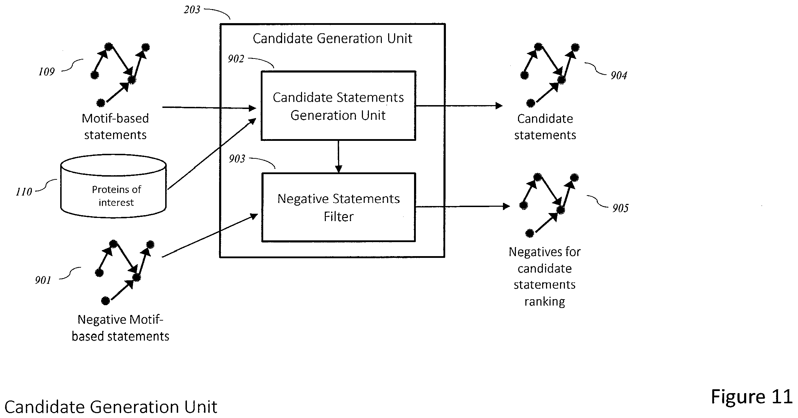

Figure 11 describes in detail the Candidate Generator/Generation Unit 203 according to one arrangement.

This macro-component may be composed of the following specific components:

- **Candidate Statements Generation Unit 902** creates candidate statements to be considered during the prediction by the system. The candidate statements are based on the proteins kinases or substrates of interest 110 given as an input (or on all proteins in the positive statements if no specific list of interest is given). This unit inputs motif-based statements.

- **Negative Statements Filter (or negative candidate statements generation unit) 903** generates the negative candidate motif-based statements required by prediction processes. This is achieved by filtering the set of motif-based negative statements (901) so that for each candidate statement created by 902, only the negative motif-based statements that share the same predicate (motif) and either the subject or the object with it are present.

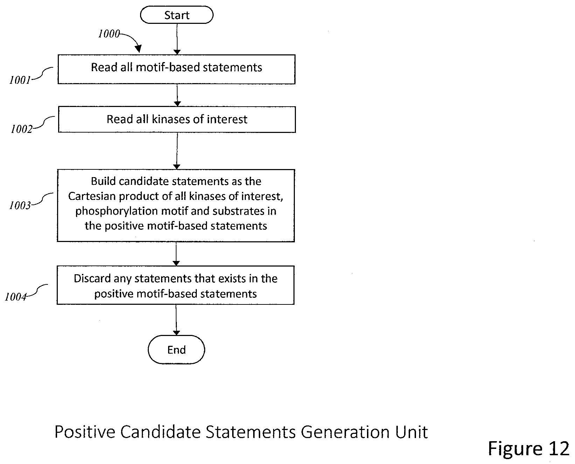

The flowchart in Figure 12 describes the process 902 of creation of candidate statements based on a list of proteins of interest:

- **1001.** This process starts by reading all positive motif-based statements 109.
- **1002.** Then the component reads all kinases of interest 110 (or all kinases).
- **1003.** The system creates candidate statements as the Cartesian product of all kinases of interest, phosphorylation motifs and protein substrates covered by the positive motif-based statements.
- **1004.** Finally, all candidate statements also present in the positive motif-based statements (i.e., including data for all known site-specific phosphorylation statements) are removed ensuring the prediction is not biased by statements used during the training phase.

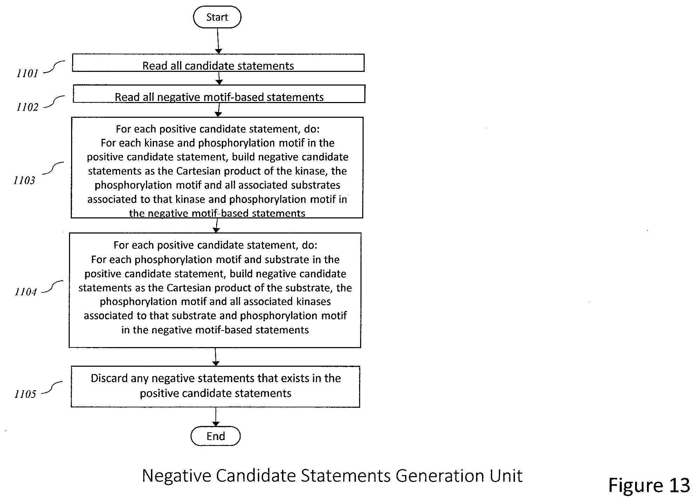

The flowchart in Figure 13 describes the process 903 of creation of negative candidate statements based on the list of positive ones:

- **1101.** This process starts by reading all candidate statements.
- **1102.** Then the component reads all negative motif-based statements 901.
- **1103.** For each protein kinase and phosphorylation motif in the candidate statements, the system creates negative candidate statements as the Cartesian product of the kinase, the phosphorylation motif and all associated substrates associated to that kinase and phosphorylation motif in the negative motif-based statements.
- **1104.** Similarly, for each phosphorylation motif and substrate in the candidate statements, the system creates negative candidate statements as the Cartesian product of the substrate, the phosphorylation motif and all associated kinases associated to that substrate and phosphorylation motif in the negative motif-based statements.
- **1105.** Finally, all negatives for candidate statements ranking also present in the candidate statements are removed.

**Example 9. Positive Candidate Statements Generation Unit**

*Step 1001 reads all the motif-based statements in Positive Motif-based Statement Table 17. Table 17 is a revised version of Table 12 for explaining this specific flowchart and has the 5 records as shown.*

**[Table 17] (revised version of Table 12)**

| Positive Motif-based | | |
|---|---|---|
| Statement Table | | |
| **Subject (Kinase)** | **Predicate (Motif id)** | **Object (Substrate)** |
| Kin00 | TKL0 | Sub00 |
| Kin00 | TKL1 | Sub20 |
| Kin20 | TKL0 | Sub00 |
| Kin20 | AGC0 | Sub21 |
| Kin21 | TKL1 | Sub21 |

*Step 1002 reads all the kinases of interest in Kinase of Interest Table. An end user may specify them. Suppose Kinase of Interest Table 18, containing one kinase "Kin00", is given.*

**[Table 18]**

| Kinase of Interest Table |
|---|
| **Kinase** |
| Kin00 |

*For each kinase of interest, Step 1003 builds all possible combinations of a statement whose subject is the kinase, whose property is a motif id appearing in Positive Motif-based Statement Table 17, and whose object is a substrate appearing in Positive Motif-based Statement Table. Since there are three unique motif ids ("AGC0", "TKLO", and "TKL1") and three unique substrates ("Sub00", "Sub20", and "Sub21"), nine (=3 × 3) records are stored in Positive Candidate Table 19 as shown.*

**[Table 19]**

| Positive Candidate Table | | | |
|---|---|---|---|
| **Cand. id** | **Kinase** | **Motif id** | **Substrate** |
| | Kin00 | TKL0 | Sub00 |
| | Kin00 | TKL0 | Sub20 |
| | Kin00 | TKL0 | Sub21 |
| | Kin00 | TKL1 | Sub00 |

(continued)

| Positive Candidate Table | | | |
|---|---|---|---|
| Cand. id | Kinase | Motif id | Substrate |
| | Kin00 | TKL1 | Sub20 |
| | Kin00 | TKL1 | Sub21 |
| | Kin00 | AGC0 | Sub00 |
| | Kin00 | AGC0 | Sub20 |
| | Kin00 | AGC0 | Sub21 |

Step 1004 discards from Positive Candidate Table 19 all the records appearing in Positive Motif-based Statement Table 17. In the current example, ⟨Kin00, TKL0, Sub00⟩ and (Kin00, TKL1, Sub20) appear in Table 17 and are discarded. As a result, Positive Candidate Table 19 becomes Positive Candidate Table 20 (each candidate id is also filled for later processing).

**[Table 20] (reprinted)**

| Positive Candidate Table | | | |
|---|---|---|---|
| Cand. Id | Subject (Kinase) | Predicate (Motif id) | Object (Substrate) |
| 100 | Kin00 | TKL0 | Sub20 |
| 101 | Kin00 | TKL0 | Sub21 |
| 102 | Kin00 | TKL1 | Sub00 |
| 103 | Kin00 | TKL1 | Sub21 |
| 104 | Kin00 | AGC0 | Sub00 |
| 105 | Kin00 | AGC0 | Sub20 |
| 106 | Kin00 | AGC0 | Sub21 |

**Example 10. Negative Candidate Statements Generation Unit**

Step 1101 reads all the positive candidate statements in Positive Candidate Table 20.
Step 1102 reads all the negative motif-based statements in Negative Motif-based Statement Table 21.

**[Table 21] (revised version of Table 15) Negative Motif-based Statement Table**

| Subject (Kinase) | Predicate (Motif id) | Object (Substrate) |
|---|---|---|
| Kin00 | TKL0 | Sub30 |
| Kin00 | TKL0 | Sub31 |
| Kin00 | TKL1 | Sub32 |
| Kin30 | TKL0 | Sub20 |
| Kin31 | TKL0 | Sub20 |
| Kin32 | AGC0 | Sub20 |

Table 21 is a revised version of Table 15 for explaining this part of the method and has the 6 records as shown.
For each positive candidate, Step 1103 generates negative candidate statements and stores them in Negative Candidate Table 22.

**[Table 22]**

| Negative Candidate Table | | | |
| --- | --- | --- | --- |
| Cand. Id | Subject (Kinase) | Predicate (Motif id) | Object (Substrate) |
| 100 | Kin00 | TKL0 | Sub30 |
| 100 | Kin00 | TKL0 | Sub31 |

*Suppose the statement with the candidate id "100" is to be processed. This step picks up, from Negative Motif-based Statement Table 21, statements whose subject and predicate are the same as the ones of the positive candidate, and stores them in Negative Candidate Table. In the current example, since the subject and the predicate of the "100" statement are "Kin00" and "TKLO", the first two records with "Kin00" and "TKLO" in Table 21 are picked up and stored in Table 22. Note that the third record is not picked up because the subjects are the same ("Kin00" but the predicates are different ("TKL0" and "TKL1").*

*For each positive candidate, Step 1104 generates negative candidate statements and stores them in Negative Candidate Table. Suppose the statement with the candidate id "100" is to be processed. This step picks up, from Negative Motif-based Statement Table 21, statements whose predicate and object are the same as the ones of the positive candidate, and stores them in Negative Candidate Table 22. In the current example, since the predicate and the object of the "100" statement are "TKLO" and "Sub20", the fourth and the fifth records with "TKLO" and "Subj20" in Table 21 are picked up and stored in Table 22. As a result, Table 22 becomes Table 23. Note that the sixth record is not picked up because the objects are the same ("Sub20") but the predicates are different ("TKL0" and "AGC0").*

**[Table 23]**

| Negative Candidate Table | | | |
| --- | --- | --- | --- |
| Cand. Id | Subject (Kinase) | Predicate (Motif id) | Object (Substrate) |
| 100 | Kin00 | TKL0 | Sub30 |
| 100 | Kin00 | TKL0 | Sub31 |
| 100 | Kin30 | TKL0 | Sub20 |
| 100 | Kin31 | TKL0 | Sub20 |

*Step 1105 discards from Negative Candidate Table 23 all the records appearing in Positive Candidate Table 20. In the current example, there is no such record and Table 23 is kept as it is.*

**Relational Learning Unit**

The Relational Learning Unit 204 consists of an off-the-shelf statistical relational learning model (see Nickel et al) that learns from the positive and negative motif-based statements (outputs of the motif converter 105 and of the negative motif-based statements generator 202), and potentially from the positive and negative protein context statements (output of the protein context statements generator 107 and the negative protein context statements generator 201). The learned model is used for *link prediction,* i.e., to predict the existence of the candidate statements generated by candidate generator 203.

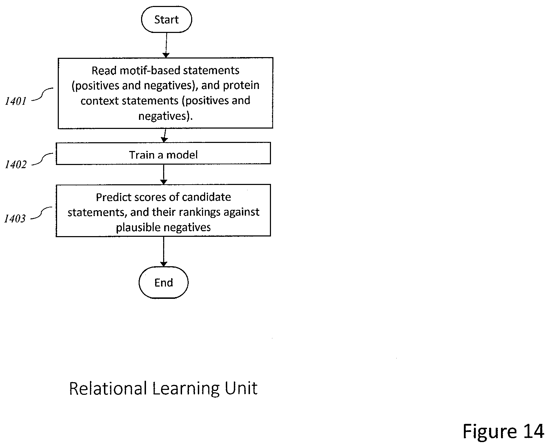

The flowchart in Figure 14 describes the process of the relational learning unit 204:

- **1401.** Read motif-based statements (positives 109 and negatives - output of negative statement generator 202), and protein context statements (positives 108 and negatives - output of negative statement generator 201).
- **1402.** Split motif-based statements in train and test sets. Add protein context statements to train set. Train the model. Training may include model selection over combinations of hyperparameters (and may adopt cross-validation), and may adopt a state-of-the-art, learning-to-rank evaluation protocol.
  In more detail, the selection is carried out by cross-validation over multiple embedding models and an exhaustive grid search over multiple combinations of hyperparameters. In each iteration of the cross-validation, the model is trained on a fraction of the phosphorylation network and tested on a disjoint validation set.
  Model selection chooses as best the model with the highest selected performance metric, which is typically a learning-to-rank metric such as the mean reciprocal rank (MRR). The adopted protocol is also based on learning-to-rank: each positive in the validation set is scored by the system, with a number of negatives that are either generated on the fly with an appropriate heuristic (e.g. by corrupting subjects or objects of positive relations), or retrieved from a gold standard. Positives and negatives scores are ranked, and ranks are used to compute the learning-to-rank performance metric (e.g. MRR).

- **1403.** The best performing model obtained at 1402 is used to predict the existence of positive candidate statements 904. Such existence is determined in a learning-to-rank fashion by the ranking of each of these statements against all plausible negatives 905 (such negatives share the same predicate and either the subject or the object). In addition to the rankings, the Relational Learning Unit may also output scores (such as normalised confidence weights), which may be used to implement custom ranking and filtering later on.

## Example 11. Relational Learning Unit

*Step 1401 reads Positive Motif-based Statement Table, Negative Motif-based Statement Table, Positive Protein Context Statement Table, and Negative Protein Context Statement Table. Examples of these tables are Table 12, Table 15, Table 13, Table 16 (all shown previously). These are just examples and the sizes of the tables are usually much bigger.*

*Step 1402 trains a model with a Relational Learning method. Any method satisfying the following conditions may be used.*

- *Includes a training phase based on known phosphorylation as converted to motifbased statements and the negatives of these statements and a prediction phase using candidate statements*
- *In the training phase:*

  o *Inputs positive 3-ary (ternary) statements and negative 3-ary statements.*
  o *Trains a model, which put a score to a statement after training, by trying to put a higher score to each input positive statement and a lower score to each input negative statement as much as possible.*

- *In the prediction phase:*

  o *Inputs positive 3-ary candidate statements and negative candidate 3-ary statements that model facts known to be false in the real world. The negative candidate 3-ary statements may have been generated as corrupted versions of a positive statement (i.e. by replacing the subject or the object of the statement with something else that does not make sense biologically speaking),*
  o *Outputs, for each positive statement, a score and a score rank in the positive statement and the corresponding negative statements.*

*Examples of such methods are CP (*Frank L Hitchcock. "The expression of a tensor or a polyadic as a sum of products". In: Studies in Applied Mathematics 6.1-4 (1927), pp. 164-189*), DistMult (*Bishan Yang et al. "Embedding entities and relations for learning and inference in knowledge bases". In: arXiv preprint arXiv:1412.6575 (2014)*)), RESCAL (*Maximilian Nickel, Volker Tresp, and Hans-Peter Kriegel. "A three-way model for collective learning on multi-relational data". In: Proceedings of the 28th international conference on machine learning (ICML-11). 2011, pp. 809-816*), TransE (*Antoine Bordes et al. "Translating embeddings for modeling multi-relational data". In: Advances in neural information processing systems. 2013, pp. 2787-2795)., ComplEx (*Theo Trouillon et al. "Complex embeddings for simple link prediction". In: arXiv preprint arXiv:1606.06357 (2016)*)), NTN (*Richard Socher et al. "Reasoning with neural tensor networks for knowledge base completion". In: Advances in neural information processing systems. 2013, pp. 926-934), HolE (*Maximilian Nickel, Lorenzo Rosasco, and Tomaso Poggio. "Holographic embeddings of knowledge graphs". In: arXiv preprint arXiv:1510.04935 (2015)) etc.*

*In the current example, suppose ComplEx is used and a model is learned with the method and the input data above (the 4 tables). The details of training are omitted.*

*Step 1403 predicts the rank and the score and stores them in Output Table for each positive candidate in Positive Candidate Table 20, shown previously.*

*The details of prediction are shown here. Suppose the "100" positive candidate in Table 20 is processed. Negative candidates corresponding to the positive candidate are the 4 records in Negative Candidate Table 23. Step 1403 first calculates a score for each of the 5 candidates. The score is computed by the inference (i.e. prediction) step of the model. During learning, the relational learning model learns the parameters of a scoring function that maps a 3-ary input to a real number (the score). The actual function depends on the adopted relational model (DistMult, and ComplEX use dot products, TransE uses vector norm, RESCAL uses matrix products, etc). Note that the score may or may not be a probability estimate. If it is not a probability estimate, it can be converted using a sigmoid function. Suppose the scores are 0.78 for the positive and 0.23, 0.85, 0.45, 0.87 for the 4 negatives. Then, the rank of the positive is 3 because 0.78 is the third largest score among the five scores. Step 1403 then stores 3 and 0.78 with the statement*

*information in Output Table. The first record of Output Table 24 is the result of this process for "100". Similarly other candidates are processed and Output Table 24 is obtained.*

**[Table 24]**

| Output Table | | | | | |
|---|---|---|---|---|---|
| **Cand. Id** | **Kinase** | **Motif id** | **Substrate** | **Rank** | **Score** |
| 100 | Kin00 | TKL0 | Sub20 | 3 | 0.78 |
| 101 | Kin00 | TKL0 | Sub21 | 10 | 0.55 |
| 102 | Kin00 | TKL1 | Sub00 | 2 | 0.05 |
| 103 | Kin00 | TKL1 | Sub21 | 8 | 0.17 |
| 104 | Kin00 | AGC0 | Sub00 | 1 | 0.95 |
| 105 | Kin00 | AGC0 | Sub20 | 4 | 0.66 |
| 106 | Kin00 | AGC0 | Sub21 | 9 | 0.05 |

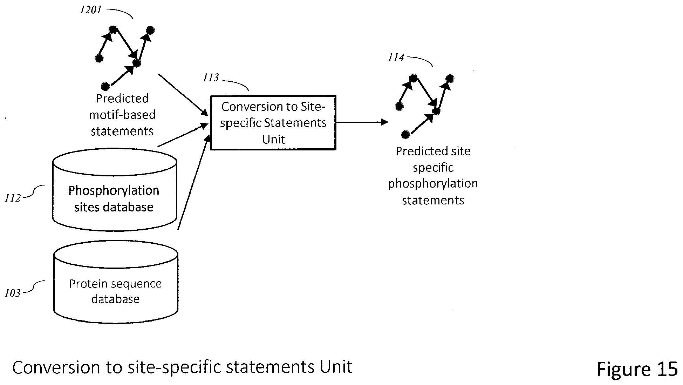

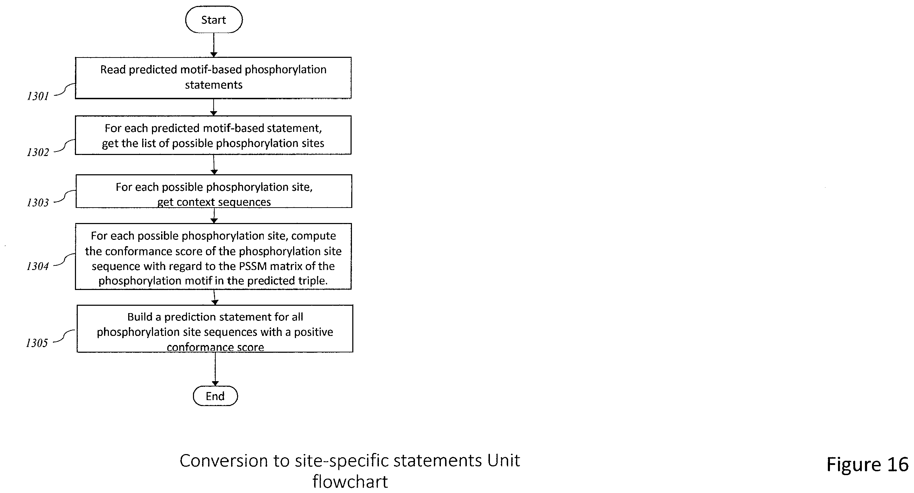

The flowchart in Figure 16 details the workings of the converter to site-specific statements 113, illustrated in Figure 15 which shows the relevant parts of Figure 1 and the input of the predicted motif-based statements, and describes the process of conversion of the predicted motif-based statements into predicted site-specific statements:

- **1301.** This process starts by reading all predicted motif-based phosphorylation statements 1201.
- **1302.** For each predicted motif-based statement, the system extracts a list of all possible phosphorylation sites for the substrate in the input statement from the phosphorylation sites database 112.
- **1303.** For each phosphorylation site, the system extracts the site context sequence from the protein sequence database 103.
- **1304.** For each possible phosphorylation site, the system computes the conformance score of the phosphorylation site sequence with regard to the phosphorylation motif in the predicted triple.
- **1305.** The system produces a set of transformed site-specific statements 114 where the kinase and substrate are as present in the predicted motif-based statement, and the sites are those that have a positive predicate motif conformance score. The score is attached to the statement scores produced by the Relational Learner/Learning Unit 204. These scores may be used for arbitrary ranking, filtering, etc., of the system results.

**Example 12.**

*Step 1301 reads predicted motif-based phosphorylation statements in Output Table 24.*

*Step 1302 gets the list of possible phosphorylation sites from Phosphorylation Sites Table 25 for each predicted motif-based phosphorylation statement and creates Motif Match Score Table 28. In the explanation below, only the first record (predicted motif-based phosphorylation statement) in Table 24 is used. Since the substrate is "Sub20" in the "100" record, the phosphorylation sites S269, S27 and T271 in the corresponding record in Phosphorylation Sites Table 25 are obtained.*

**[Table 25]**

| Phosphorylation Sites Table | |
|---|---|
| **Substrate** | **Phosphorylation sites** |
| Sub20 | {S85, S120, T97} |
| ... | ... |

*Then Step 1302 creates Motif Match Score Table 26.*

**[Table 26]**

| Motif Match Score Table | | | |
|---|---|---|---|
| Cand. Id | Phosphorylation site | Context sequence | motif match score |
| 100 | S85 | | |
| 100 | S120 | | |
| 100 | T97 | | |
| ... | ... | | ... |

Each record in Table 26 corresponds to Table 24 a phosphorylation site ("Cand. id" is also inserted for linking data between the Output Table and Motif Match Score Table). The empty fields "context sequence" and "motif match score" will be filled in later steps.

For each possible phosphorylation site, Step 1303 refers to Protein Sequence Table 27 and updates Motif Match Score Table 28.

**[Table 27] Protein Sequence Table (enriched version of Table 5)**

| Protein | Sequence |
|---|---|
| Sub00 | MFQIPEFEPS EQEDSSSAER GLGPSPAGDG PSGSGKHHRQ APGLLWDASH QQEQPTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWWDRNLG RGSSAPSQ |
| ... | ... |
| Sub20 | MYQAINPCPQ SWYGSPQLER EIVCKMSGAP HYPNYYPVHP NALGGAWFDT SLNARSLTTT PSLTTCTPPS LAACTPPTSL GMVDSPPHIN PPRRIGTLCF DFGSAKSPQR CECVASDRPS TTSNTAPDTY RLLITNSKTR KNNYGTCRLE PLTYGI |
| ... | ... |

Table 27 is an enriched version of Table 5, with the "Sub20" record. Processing of the first record in Motif Match Score Table 26 is as follows:

- Since the substrate is "Sub20" (obtained from Output Table 24 by the candidate id "100"), the record for "Sub20" in Protein Sequence Table 27 is looked up. Similarly to Step 403, the corresponding sequence to the phosphorylation site "S85" is extracted. Concretely, the sequence from the 78th (=85 - 7) character to the 92nd (=85+7) character in the sequence "MYQAINPCPQ ...", that is "TSLGMVDSPPHINPP", is extracted and filled in Motif Match Score Table.

After processing all, Motif Match Score Table 26 becomes Motif Match Score Table 28.

**[Table 28]**

| Motif Match Score Table | | | |
|---|---|---|---|
| Cand. id | Phosphorylation site | Context sequence | Motif match score |
| 100 | SBS | TSLGMVDSPPHIKPP | |
| 100 | 5120 | CVASDRPSTTSNTAP | |
| 1000 | T97 | HPPRRIGTLCFDFGS | |
| ... | ... | | ... |

For each possible phosphorylation site, Step 1304 calculates the conformance score and updates Motif Match

*Score Table. Processing of the first record in Motif Match Score Table 28 is as follows:*

- *Since the motif id is "TKL0" (obtained from Output Table 24 by the candidate id "100") and the sequence is "TSLGMVDSPPHINPP", the conformance score of "TSLGMVDSPPHINPP" for the PSSM of the "TKLO" in Motif Table 8 is calculated similarly to Step 502. Suppose "32" is obtained. Then, Motif Match Score Table is updated as shown in the first record of Table 29.*

*After processing all, Motif Match Score Table 28 becomes Motif Match Score Table 29.*

**[Table 29]**
**Motif Match Score Table**

| Cand. id | Phosphorylation site | Context sequence | Motif match score |
|---|---|---|---|
| 100 | S85 | TSLGMVDSPPHINPP | 32 |
| 100 | S120 | CVASDRPSTTSNTAP | -47 |
| 100 | T97 | NPPRRIGTLCFDFGS | 296 |
| ... | ... | | ... |

*Step 1305 creates Prediction Table 30 based on Output Table 24 and Motif Match Score Table 29. For each record in Motif Match Score Table 29, a record is created in Table 30 as long as the motif match score is positive. This is the most relaxed strategy, generating statements with sites that have a higher chance than random for being associated with the predicate motif (sites with negative scores should never be included as they indicate disassociation). Another option (not shown here but perfectly possible with the arrangements) is to use just the site with the best positive score. This will put emphasis on high accuracy of the conversion, but may discard many potential valid predictions. An approach between these extremes is also feasible.*
*Suppose the first record in Table 29 is processed. Since the score is 32 and positive, the record is used. The kinase "Kin00", the substrate "Sub20", the rank "3", and the score "0.78" for "100" are taken from Table 24, and the phosphorylation site "S85" and the motif match score "32" are taken from Table 29, and the first record in Table 30 is created.*
*Next, suppose the second record in Table 29 is processed. Since the score is -47 and negative, the record is discarded.*
*Finally, Prediction Table 30 is used.*

**[Table 30]**
**Prediction Table**

| Kinase | Phosphorylation site | Substrate | Rank | Score | Motif match score |
|---|---|---|---|---|---|
| Kin00 | S85 | Sub20 | 3 | 0.78 | 32 |
| Kin00 | T97 | Sub20 | 3 | 0.78 | 296 |
| ... | ... | ... | ... | ... | ... |

*Outputted Prediction Table 30 is supposed to be used by those who perform biological experiments. For example, with the first record in Table 30, a biologist is supposed to perform an experiment investigating whether "Kin00" really phosphorylates "S85" on "Sub20".*
*For such biologists, the rank, the score, and the motif match score may be used for deciding the priorities of experiments. For helping them, the system may sort and/or filter records in Prediction Table 30. For example, the threshold value "50" for the motif match score may be set for filtering out records with a motif match score lower than the value. Then, the first record is excluded because 32 < 50 and the second record is kept 296 > 50.*

**Scope**

By providing quantitative and ranked predictions of site-specific phosphorylation, the arrangements support the design of biomedical experiments aimed at discovery of functional impacts of phosphorylation pathways. The quality of the system's predictions is directly related to the quality of the input data. The system performs best with manually curated input, even though it may also cope with some level of noise in the data.

The system expects that all protein-related input databases (namely the Phosphorylation Network 102, the Protein database 103, the Kinase family database 104, the Kinases of interest 108 and the Protein context knowledge graph 106) use the same identification scheme to refer to the same entities (e.g. Protein kinase "Q02156" has the same identifier in all databases). This guarantees that a single entity, such as a protein, is represented as a single node in the knowledge graph used by the system. This is an important condition for the relational learning model to learn the features correctly from the knowledge graph, without extra conversion.

**Functionality, Effectiveness**

The arrangements provide an apparatus and a method to predict new phosphorylations from phosphorylation knowledge bases and potentially additional context information such as protein-protein interaction networks. The arrangements may yield reliable phosphorylation predictions in a more comprehensive manner than the prior art (i.e., for arbitrary combinations of known kinases, sites and substrates). Also, the approach is fully automated (even the optimal features are extracted autonomously) and thus may produce the results from publicly available data sets without any human intervention. This is another advantage when compared to prior art, which often requires extensive manual efforts in data preparation and feature engineering in order to work properly.

The arrangements may solve the three technical problems A), B) and C) as follows:

A. Arrangements may solve Technical Problem A by yielding phosphorylation predictions that leverage latent features extracted by a relational learning model that learns from the whole phosphorylation network (and, when available, additional context information).

B. Arrangements may solve Technical Problem B by overcoming the lack of models applicable to context-dependent data by converting phosphorylation knowledge bases into typed binary relations based on discovery of motifs specific to particular kinase families.

C. Arrangements may solve Technical Problem C by generating negatives based on the actual phosphorylation biology, which are much more reliable than negatives produced with the standard techniques.

Some arrangements have one or more of the following practical effects:

- **More efficient design of computational experiments.** Since all steps in the presented method are fully automated (including the feature engineering) and may be easily adjusted by tuning the module parameters, the technology may be used for many different set ups of phosphorylation prediction experiments. This may facilitate efficient identification of the models best complying with the requirements of the downstream laboratory experiments (e.g., maximising certain performance metrics or focusing on a limited sub-set of kinases).
- **Less time consuming and cheaper design of biomedical experiments aimed at discovery of functional impacts of phosphorylation pathways.** This is due to automated discovery of small numbers of (ranked) phosphorylation candidates, which may be used in much faster design of specific hypothetical phosphorylation networks to be verified by wet lab (physical laboratory) experiments.
- **More flexibility in designing the wet lab experiments.** As the arrangements may easily generate prediction candidates for any type of phosphorylation, they may be used for a broad range of experimental designs covering all known kinases and/or substrates off-the-shelf, which is not so straightforward with the prior art.
- **More comprehensive support of contextual information.** The arrangements may easily incorporate additional knowledge graphs with relevant information on the kinases or substrates (e.g., protein interaction networks or protein databases). This allows biomedical researchers to utilise additional sources of facts relevant to phosphorylation with an unprecedented ease, leading to more focused and reliable prediction results.

Arrangements may provide a system that may automatically:

- combine phosphorylation data with related protein contextual data, in the form of knowledge graphs (see discovery system 101), such as the protein context knowledge graph;

- predict previously unknown site-specific phosphorylation statements from the combined data using a link prediction technique (see link prediction unit 111).

These features address Technical Problem A.

Arrangements may provide a system that may realise two-way conversion between site-specific phosphorylation statements and actionable triples with generalised predicates based on motifs (converters 105, 113). These features address Technical Problem B.

Existing link prediction techniques cannot produce meaningful results from the phosphorylation networks. The inventors have come to the realisation that the data may be converted to an internal format in order to make predictions and then converted back to the standard site-specific phosphorylation statements so that users may benefit from the predictions.

Finally, arrangements may provide a system that may realise generation of negative motif-based phosphorylation statements that may be safely used for link prediction (using Negative Motif-based Statements Generation Unit 202). These features address Technical Problem C. Step 702 and Step 703 in Figure 9 are inventive both taken separately or together in the way they create negative motif-based statements by substrate and/or by kinase.

Applicable link prediction techniques require reliable negative examples that cannot be safely generated with the techniques typically used in prior art.

**Worked Example** - **LinkPhinder**

**1 Introduction**

Nearly all aspects of cell behaviour are controlled by phosphorylation events and intricate networks of kinases-substrate relationships mediating these phosphorylations. Phosphorylation is the attachment of a phosphoryl group by a kinase to its substrate, and usually alters the activity of the substrate, thereby mediating important cellular functions such as signal-transduction, differentiation, migration, cell division and apoptosis. Dysregulation of these kinase-substrate relationship can have devastating consequences often leading to diseases, such as cancers or immune diseases. Perhaps unsurprisingly, kinases present one of the largest protein family within the human genome. It was estimated that 500-800 kinases phosphorylate about 200,000 different phosphorylation sites on their protein substrates. Kinases also make excellent drug targets, because they often present tangible pockets within their 3-D structure where drugs can bind.

Only a small fraction of 200,000 kinase-substrate interactions has been mapped. With the advent of modern high-throughput mass-spectrometry based phosphoproteomics, the identification of phosphorylation sites in substrate proteins has become relatively easy Predicting which kinase can phosphorylate which substrates at which sites is one of the biggest unresolved challenges in biology today. High-throughput methods cannot be used in this case and one-off experimentation is prohibitively expensive and time-consuming due to the large number of candidate interactions to be tested. As a consequence, the rate of discoveries of new phosphorylation reactions and their function is still rather slow.

Reliable automated prediction of phosphorylation candidates is therefore much desired, because it can substantially reduce the number possibilities that have to be tested experimentally. During the last decade, several tools for predicting phosphorylations have become available. The most popular, recent and/or actively maintained are the following: Scansite [J. C. Obenauer, L. C. Cantley, and M. B. Yaffe. Scansite 2.0: Proteome-wide prediction of cell signaling interactions using short sequence motifs. Nucleic acids research, 31(13):3635-3641, 2003], GPS [Y. Xue, J. Ren, X. Gao, C. Jin, L. Wen, and X. Yao. GPS 2.0, a tool to predict kinase-specific phosphorylation sites in hierarchy. Molecular & cellular proteomics, 7(9): 1598-1608, 2008], NetPhos [N. Blom, T. Sicheritz-Pont'en, R. Gupta, S. Gammeltoft, and S. Brunak. Prediction of post-translational glycosylation and phosphorylation of proteins from the amino acid sequence. Pro-mics, 4(6):1633-1649, 2004], NetPhorest [H. Horn, E. M. Schoof, J. Kim, X. Robin, M. L. Miller, F. Diella, A. Palma, G. Cesareni, L. J. Jensen, and R. binding. KinomeXplorer: an integrated platform for kinome biology studies. Nature methods, 11(6):603-604, 2014], NetworKin [H. Horn, E. M. Schoof, J. Kim, X. Robin, M. L. Miller, F. Diella, A. Palma, G. Cesareni, L. J. Jensen, and R. binding. KinomeX-plorer: an integrated platform for kinome biology studies. Nature methods, 11(6): 603-604, 2014], [R. binding, L. J. Jensen, G. J. Ostheimer, M. A. van Vugt, C. Jorgensen, I. M. Miron, F. Diella, K. Colwill, L. Taylor, K. Elder, et al. Systematic discovery of in vivo phosphorylation networks. Cell, 129(7):1415-1426, 2007], PhosphoPredict [J. Song, H. Wang, J. Wang, A. Leier, T. Marquez-Lago, B. Yang, Z. Zhang, T. Akutsu, G. I. Webb, and R. J. Daly. Phosphopredict: A bioinformatics tool for prediction of human kinase-specific phosphorylation substrates and sites by integrating heterogeneous feature selection. Scientific Reports, 7(1):6862, 2017].

Some of the existing tools can predict putative kinases for given phosphorylations sites or motifs, but they all have an inherent limitation - they predominantly use local protein features (such as consensus sequence motifs or protein structure) for making the predictions. As pointed out in R. binding, L. J. Jensen, G. J. Ostheimer, M. A. van Vugt, C. Jorgensen, I. M. Miron, F. Diella, K. Colwill, L. Taylor, K. Elder, et al. Systematic discovery of in vivo phosphorylation networks. Cell, 129(7):1415-1426, 2007, interaction context is an important predictor of possible phosphorylations. Yet even in the system of the same authors, NetworKin, the interaction context is used for filtering predictions, not for actually computing them. As a result, current tools are limited in a number of ways: 1. Their predictions largely disregard the wealth of knowledge on existing phosphorylation and protein-interaction networks. 2. Due to reliance on local features, the existing tools tend to work rather poorly for large number of proteins that are not well researched and/or atypical. 3. The existing tools cannot easily incorporate relevant contextual data such as protein interaction and pathway networks or publication data. This worked example shows that all these limitations can be overcome by representing the known phosphorylation network as a knowledge graph. The knowledge graph representation allows incorporation of network knowledge into the prediction process itself, rather than using it as a filtering mechanism, computation of highly flexible, meaningful predictions for any kinase and substrate in the network, including many that cannot be computed with current tools, and incorporation of context in principle as input to the prediction process.

## 2. Results

### 2.1 Overview of LinkPhinder

This approach is based on a method for converting site-specific statements
(kinase, site, substrate) available in existing phosphorylation networks into motif-based statements
(kinase, predicate, substrate) ,
where predicates are relation labels that preserve the semantics of the original statement. Specifically, the predicates generalise the site information via kinase family consensus motifs and thus they can be compared across multiple substrates and interpreted as kinase-substrate links (in a knowledge graph). This is not possible with the original statements since the meaning of a site is dependent on the substrate and thus it cannot be used as a general relation type. Disregarding the site information allows for treating the data as a graph, but only with non-specific kinase-substrate links, losing the site context. Addressing these problems, the proposed conversion allows for:

- computing phosphorylation predictions with robust discovery techniques recently proposed for knowledge graphs, namely statistical relational learning;
- generation of biologically meaningful negative examples required for training the predictive model;
- easy integration of the phosphorylation information with other relevant graph-based data (such as protein interactions, co-occurrences extracted from publications or pathways) in future research.

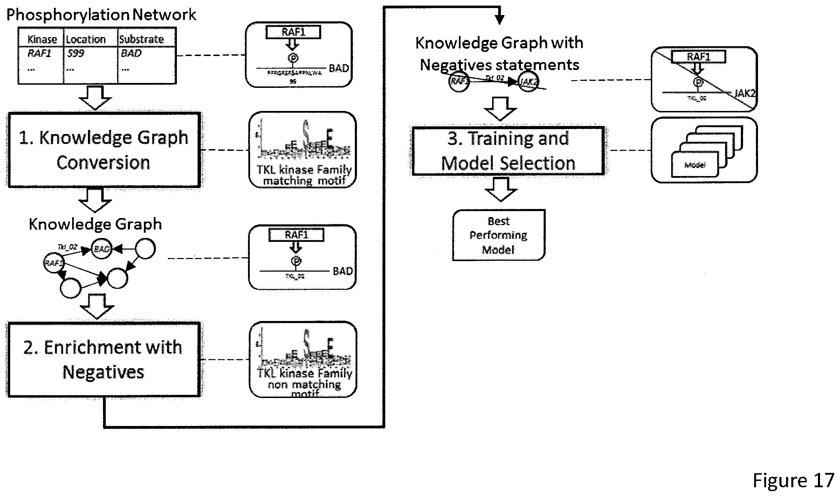

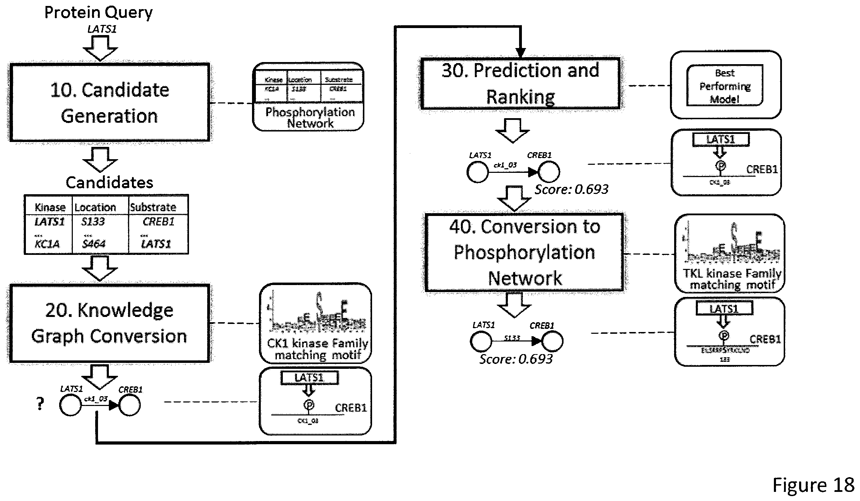

The workings of LinkPhinder are illustrated in Figures 17 and 18 that give an overview of training the model and using it for predictions, respectively. Further details on the particular steps are provided in dedicated sections below.
Figure 17 shows training of the LinkPhinder system. An existing phosphorylation network is taken as an input (top left) and for each substrate/phosphorylation site represented in it, the sub-sequence of the substrate surrounding the phosphorylation site is identified (as shown to the right of the network representation in the Figure). In knowledge graph conversion 1, consensus motifs of these sequences grouped based on the kinase families that bind to them are computed. The phosphorylation statements from the input network are transformed to knowledge graph relations using compatible consensus motifs by allocating a compatible consensus motif (as shown in the Figure with larger letters representing higher probability amino acids) and storing all the thus produced statements in a knowledge graph format (a graph view rather than a table view of the data, as shown in the Figure). Here, RAF1 is shown with predicate TKL_02. A dual process as previously described is used for generating 2 negative examples from the positive statements in the knowledge graph, such as the one shown in the top right of the Figure. The positives and negatives are required for training link prediction models by means of supervised statistical relational learning. A wide range of models is trained 3 based on a comprehensive set of hyper-parameters in order to find the best model based on their performance on train/test/validation splits of the input data.
Figure 18 is a similar figure using the same components that gives an overview of making predictions with the LinkPhinder system based on an input query protein and the phosphorylation network used for training the model. Firstly, a set of candidate phosphorylation statements is generated 10. The candidates span all possible interactions of the query protein with kinases and substrates present in the training data, across all possible phosphorylation sites. Similarly to the training stage, the candidate statements are converted 20 into knowledge graph relations. The best-performing model is then used for computing 30 probabilistic scores of all candidate statements. The scored statements are ranked and converted 40 back to phosphorylation statements using the previously computed consensus motifs. The candidate phosphorylations involving the query protein either as a kinase or as a substrate can then conveniently be browsed in or filtered out of the output list, sorted in descending order by the prediction scores. Optionally, high-stringency cut-off can be applied on the result list.

### 2.1.1 Knowledge Graph Conversion

The primary input is a phosphorylation network compiled from the **PhosphoSitePlus** [P. V. Hornbeck, J. M. Kornhauser, S, Tkachev, B. Zhang, E. Skrzypek, B. Murray, V. Latham, and M. Sullivan. PhosphoSitePlus: a comprehensive resource for investigating the structure and function of experimentally determined post-translational modifications in man and mouse. Nucleic acids research, 40(D1):11-22, 2011] data set. The network consists of statements **<K, L,S >** where *K, L, S* are kinase, phosphorylation site and substrate, respectively. The semantics of such statements is that the kinase **K** phosphorylates the substrate **S** by binding to it at site **L.**

Conversion of the phosphorylation network to knowledge graph utilises motifs of phosphorylation sites preferred by specific kinase families. For each kinase family [G. Manning, D. B. Whyte, R. Martinez, T. Hunter, and S. Sudarsanam. The protein kinase complement of the human genome. Science, 298(5600):1912-1934, 2002], the method computes a set of motifs characteristic to the local context of the sites in substrates that the kinases in that family bind to. The local context is a window of **2*k* + 1** amino acids surrounding the phosphorylation site in the substrate sequence, where k is a configurable parameter of the conversion algorithm. It gives the number of amino acids on the left and right side of the phosphorylation site that should be taken into account as a context.

The motifs are used for converting the **(K, L,S)** phosphorylation network statements to labeled knowledge graph edges **(K,M,S)**, where **M** is a predicate label that corresponds to a motif compatible with the family of **K** and the site **L** in the substrate **S.** In other words, the scoring matrix of the motif **M** computes a positive score for the local context of **L** in **S**).

### 2.1.2 Enrichment with Negatives

The knowledge graph generated from the phosphorylation network can be used for discovering new phosphorylations by means of link prediction. However, training a link prediction model is a supervised machine learning process, and therefore requires negative examples in addition to the positive statements in the phosphorylation knowledge graph. Such negative examples are typically created by corruption of the positive statements. In this case, such a technique could lead to actual phosphorylations being treated as negatives due to the indirect nature of the knowledge graph. Therefore specific restrictions are imposed on the negative statements, based on the biology of phosphorylation.

The corruptions uses are based on two facts. First, kinases in different families tend to phosphorylate different substrates. Second, substrates are unlikely to be phosphorylated by certain kinases if they have highly incompatible phosphorylation sites with respect to the kinase consensus motif. This directly corresponds to two types of corruption. For a statement **(K,M,S),** valid corruptions are statements **(K',M,S)** and **(K, M,S')** such that $K^l$ is from different family than **K** and all phosphorylation sites in $S^l$ score negatively with respect to the scoring matrix of the motif **M** .

### 2.1.3 Training and Model Selection

The phosphorylation knowledge graph enriched by negative statements can be used for training statistical relational learning models in order to perform link prediction (and thus allow for potential discovery of previously unknown phosphorylations). Training a link prediction model is essentially a learning-to-rank problem in which parameters of a scoring function are optimised based on the positive and negative statements in the knowledge graph.

The range of possible models that can be trained on the knowledge graph corresponds to the space of all combinations of various hyper-parameters of the training process. The most important hyperparameters pertain to the motif discovery (e.g. size of the sequences surrounding the phosphorylation sites or number of sequences processed at once for the motif discovery) and to the type and parameters of the model used for learning knowledge graph embeddings. Details on the range of possible options are provided in Annex A.

The best-performing model can be selected by exhaustive search in the space of all hyper-parameter combinations, where the performance of each combination is tested by training the corresponding model on one part of the knowledge graph and testing it on another. The average precision metric (equivalent to area under the precision-recall curve) is used to select the final model as it is considered the most appropriate metric for assessing overall performance of machine learning, and especially ranking models.

This approach based on knowledge graph embeddings allows for finding a model that reflects the long-range properties, global features and multi-variate nature of known phosphorylation networks in a comprehensive manner. This is substantially different to the existing approaches that only use local features of substrate proteins (sequence, structure, kinase affinities, etc.) and do not take the whole phosphorylation network context in computing their scoring.

### 2.1.4 Prediction and Ranking

Once the best performing link prediction model has been identified, it can be trained on the whole phosphorylation knowledge graph to maximise its generalisation power. This model can then be used for computing ranking scores (values between 0 and 1) of statements ranging across all possible combinations of kinases, sites and substrates provided in the training data. Given a single protein as a query, the model can produce a ranked set of candidate phosphorylations that involve the protein either as a substrate or as a kinase. The ranked list can optionally be filtered based using high- or medium-stringency threshold. The threshold used is derived from the input phosphorylation network - the high-stringency threshold is a value such that 99.5% of the known phosphorylations score above it (the value is 0.672 in the reported model). The medium-stringency threshold is 0.5 (i.e. a score that indicates higher-than-random plausibility of the given statement based on its probabilistic interpretation).

The ranking of the results reflects the global network context of all known phosphorylations represented in the input knowledge graph, which is not done by any other existing tool. Moreover, the predictions can be generated on any protein, be it a kinase or substrate. This makes the model more powerful than most existing phosphorylation prediction tools that can only be queried for substrate proteins (in the GPS tool, one can start generating predictions from a kinase, however, they only cover about 80 kinases when compared to over 300 in case of this system).

### 2.1.5 Conversion to Phosphorylation Network

The link prediction model works on the converted knowledge graph (i.e. it produces motif-based statements in the form (*K,M,S*)). Candidate phosphorylations for which the model is supposed to compute scores are thus required to be converted into this form. After the motif-based statements are scored, they have to be converted back to actual phosphorylation statements before presenting them to users. This conversion is dual to the knowledge graph conversion - each statement (*K,M,S*) corresponds to statements (*K,L,S*) such that *L* is a valid phosphorylation site in S (as per the PhosphoSitePlus data set) that scores positively with respect to the scoring matrix of the motif **M** .

### 2.2 Datasets and Tools Used

To compile the phosphorylation network that is the primary input for building the LinkPhinder model, the method used the PhosphoSitePlus dataset in a version available on 26th of June 2017 (c.f. https:// www.phosphosite.org/staticDownloads.action). There were 10,173 phosphorylation statements on 362, 7,302 and 2,377 distinct kinases, substrate-site combinations and substrates in the compiled phosphorylation network, respectively. Note that the construction of all datasets focuses only on the Homo Sapiens species, unless specified otherwise.

In order to convert the phosphorylation statements extracted from PhosphoSitePlus into a knowledge graph, the method computed motifs characteristic to the context sequences of phosphorylation sites. For that task, the MEME tool is used, version 4.11.2 (c.f. http://meme-suite.org/doc/download.html?man_type=web).

Three state of the art knowledge graph embedding and link prediction methods were employed to train a model that can discover new links in the phosphorylation knowledge graph. The methods are **TransE** [A. Bordes, N. Usunier, A. Garcia-Duran, J. Weston, and O. Yakhnenko. Translating embeddings for modeling multi-relational data. In Advances in neural information processing systems, pages 2787-2795, 2013], **DistMult** [B. Yang, W.-t. Yih, X. He, J. Gao, and L. Deng. Embedding entities and relations for learning and inference in knowledge bases. arXiv preprint arXiv:1412.6575, 2014] and **ComplEx** [T. Trouillon, J. Welbl, S. Riedel, E' . Gaussier, and G. Bouchard. Complex embeddings for simple link prediction. arXiv preprint arXiv:1606.06357, 2016].

The PhosphoSitePlus dataset, together with UniProt (c.f. http://www.uniprot.org/) was also used for generating a mapping between substrates and their possible phosphorylation sites. This mapping was used in the conversion of the internal, motif-based knowledge graph statements to phosphorylation statements when computing scores of possible phosphorylations that have not been known before. The focus was only on substrates present in the knowledge graph, which resulted in 74,142 distinct substrate-site pairs that can be used for generating candidate phosphorylations (i.e. potential discoveries).

To assess LinkPhinder in comparison with related state of the art systems, the inventors downloaded and/or generated full sets of phosphorylation predictions that can be made with the following tools: Scansite 3 (c.f. http://scansite3.mit.edu), KinomeExplorer (predictions produced by two tools, NetworKIN and NetPhosK, c.f. http://kinomexplorer. info/), Netphos (c.f. http://www.cbs.dtu.dk/services/NetPhos/) and GPS (c.f. http://gps.biocuckoo.org/index.php). The numbers of predictions that can be made with the corresponding tools are as follows: 6,908,157 (GPS), 5,192,236 (KinomeExplorer), 3,614,298 (Netphos), 401,260 (Scansite 3), 2,006,185 (PhosphoPredict).

Full description on data sources, tool settings and the processing steps employed in order to arrive to the data used in the computational experiments is provided in Annex A.

### 2.3 Predictive Power of the LinkPhinder Model

The predictive power of the model was validated using the metrics of area under the ROC curve (AU-ROC) commonly used to illustrate the diagnostic ability of a binary classifier system and average precision (AP), which is equivalent to the area under the precision-recall curve (AU-PR). These metrics are arguably most appropriate for validating a predictive model based on raking [T.-Y. Liu et al. Learning to rank for information retrieval. Foundations and Trends in Information Retrieval, 3(3):225-331, 2009]. They validate the models across their whole operating range and thus are useful for determining their general predictive power [J. Davis and M. Goadrich. The relationship between precision-recall and roc curves. In Proceedings of the 23rd international conference on Machine learning, pages 233-240. ACM, 2006].

First the best-performing configuration of hyper-parameters of the model constructed were found, as described in the previous sections (full details on the hyper-parameters, etc., are given in Annex A). A random 80-20% split of the phosphorylation network into training and testing datasets was used. The best-performing model was trained using 10-fold cross-validation on the training split and validated using the unseen statements from the testing split. This experiment was performed 100-times with different random splits each time to verify the stability of the results. The number of repetitions was set so as to make the confidence intervals of the values of the measured metrics stable.

The model achieved stable scores of 0.906 ($\pm$ 0.02) and 0.958 ($\pm$ 0.006) in the metrics of AU-PR and AU-ROC, respectively, over corresponding base-lines of 0.51 and 0.506 (balanced positive/negative classes). This is typically considered as a sign of very high predictive power. A direct comparison with related existing tools in this particular experiment is unfortunately not possible. The reason is that the publicly available interfaces and data dumps of these tools cannot be used for generating scores of any negative statements, and of some positive statements present in the inventors' data set. Computing the values of selected metrics and comparing them would thus be meaningless. This limitation of the existing tools is inherent to the different prediction methodology focused on single proteins instead of the global network context and all possible phosphorylation combinations.

## 2.4 Targeted Experiments Confirm Two Biologically Meaningful Predictions

The rich dataset of nearly 27 million candidate predictions was assessed regarding its potential for discovering clinically relevant phosphorylation reactions. To this end, the inventors extracted high-stringency predictions for the LATS1 and AKT1 kinases. By visual inspection of this list, they were able immediately pinpoint several known and promising substrates of these kinases. YAP1 for example is the best characterised substrate of LATS1, and the tool predicted that LATS1 would phosphorylate YAP at serine 127. Additionally the list of the top 200 predictions for AKT contained eight bona-fide AKT substrates (https://www.cellsignal.com/contents/resources-reference-tables/pi3k-akt-substrates-table/science-tables-akt-substrate), for which several site specific phosphorylation reactions were predicted. This means that the tool generated interesting, biologically relevant predictions.

Next some of the new substrates were validated experimentally. In order to select the most promising candidates, three proteins were selected that are part of the wider LATS1 signalling network and for which antibodies were commercially available. The following strategy was used to validate LATS1 substrates we used. HEK293 cells were transfected with two specific siRNA against LAST1 in order to downregulate LATS1 levels (knockdown). Following this LATS1 knockdown, expected decrease in the phosphorylation of LATS1 substrates. For confirmation, a positive control was used by measuring the phosphorylation of the known LATS1 substrate YAP1-S127, and indeed a decrease in YAP1-S127 phosphorylation in total lysates was observed. This control experiment demonstrated that the LATS1 knockdown works as expected and can be used to confirm potential LATS1 substrates.

One of the proteins selected for validation was CREB which is transcription factor that is regulated by phosphorylation. This transcription factor is one of the best characterised effectors of the MAPK and PKA pathways. Evidence in the literature indicates that CREB modulates important hippo pathway functions by direct interaction with YAP1 and regulation of transcription. The tool predicted serine 133 of CREB (CREB-S133) as a putative substrate of LAST1. To confirm this, a specific antibody against CREB-S133 was used, and downregulation of LATS1 resulted in about 50% decrease CREB-S133 phosphorylation. This result clearly indicated that CREB is a LATS1 substrate and highlights the potential of the tool to identify previously unknown kinase substrates. Another prediction tested was the phosphorylation of p53 at serine 46. p53 is an important transcription factor that regulates cell fates such as cell cycle arrest and apoptosis in response to DNA damage. Although p53-S46 was predicted as potential LATS substrate, a decreased p53-S46 phosphorylation in the LATS1 knockdown experiments was not observed, indicating that this prediction is a false positive for the experimental conditions used.

In the case of AKT, putative substrates were monitored by manipulating the level of AKT activation using two strategies. Firstly, AKT activity was inhibited by using the specific chemical inhibitor AKT IV. Secondly, AKT activity was increased by transfecting the kinase hyperactive form of AKT gag-AKT. One of the predicted AKT substrates is STK3 (MST2), which is an important protein in the hippo pathway, and which according to the prediction should be phosphorylated at serine 18.

Unfortunately, no commercially available antibody existed that could measure this phosphorylation site. Therefore an indirect approach was employed to validate this prediction. This used an antibody that specifically binds to phosphorylated AKT substrates, which will immunoprecipitate (IP) all the proteins that are phosphorylated By AKT. Next, this IP was blotted using a specific antibody against STK3. These experiments show that inhibition of AKT resulted in a slight decrease of STK3 phosphorylation, and that expression of active AKT resulted in a 10 fold increase. The results validate the prediction that AKT1 phosphorylates STK3.

None of these discoveries could have been predicted with four out of six of the related existing tools starting with the LATS1 or AKT1 kinases as queries. GPS and PhosphoPredict support such queries, but only for less than 25% and 3.5% of the kinases covered by the LinkPhinder system, respectively. Further, querying for the substrates directly did not reproduce LinkPhinder predictions with any existing system on the high stringency settings (if applicable, if controlling the stringency was not offered by a particular tool, the experiments used all predictions made by the given tool). On medium stringency, the GPS tool could reproduce one prediction; the CREB1 phosphorylation by LATS1. On low stringency, the NetPhosK tool could also reproduce one prediction; STK3 phosphorylation by ATK1. No tool could reproduce both prediction in any setting.

### 2.5 Mass Spectrometry Experiments Confirmed an Additional 7 Phosphorylation Predictions

In a complementary laboratory validation, the high-and medium-stringency predictions were cross-referenced with high-throughput phosphorproteomic data on the LATS1 interactome. The basic idea is the following. To phosphorylate a target, LATS1 needs to bind to it and phosphorylate it. Thus, by isolating all proteins that are bound to LATS1 by immunoprecipitation (IP) and analysing this interactome using mass-spectrometry based proteomics, it should be possible to identify a large number of LATS1 phosphorylation targets. Thus, phosphoproteomic data on LATS1 interactome was obtained from cells treated with two proapoptotic signals: FAS and etoposide. To identify the interactome transiently expressed GFP-LAST1 was immunoprecipitated, the associated protein complexes digested using trypsins, the resulting peptides analysed using mass-spectrometry, and phosphorylated residues identified using MaxQuant. This identified six proteins that were bound to LATS1 and phosphorylated at at least one residue. (Unspecific phosphoproteins that were also identified in the non-LATS1 transfected control were discarded.)

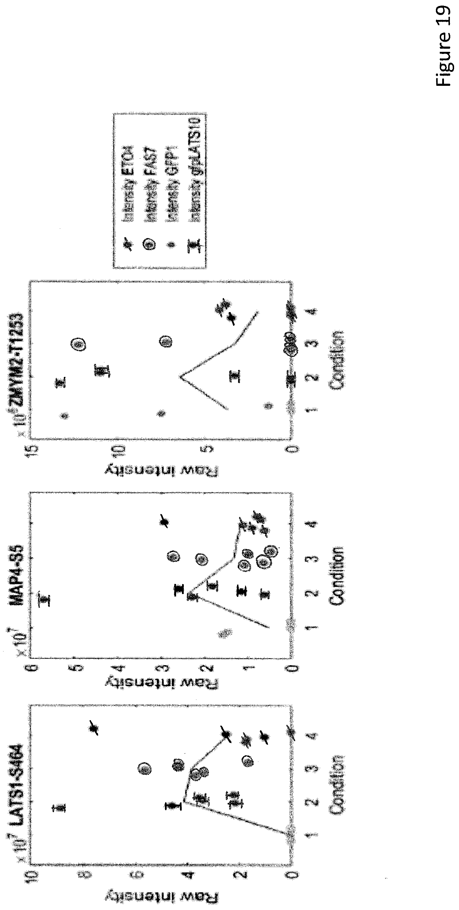

Although this list would contain LATS1 substrates, it is important to note that not all these phosphoproteins are LATS1 targets, because LATS1 also binds to proteins that are phosphorylated by other kinases. Next this list of phosphorylated LATS1 interactors was with the list of predicted LATS1 phosphorylation targets from LinkPhinder. This confirmed 7 previously unknown phosphorylations on three substrates; five residues were phosphorylated on LATS1, one on MAP4, and one on ZMYM2. Figure 19 shows mass-spectrometer validation of a subset of predicted phosphorylations. Shown are the raw intensity values (dots) of the detected phospho-proteins of the LATS1-IP in the indicated conditions (n=6 replicates), and corresponding mean value (lines). Because these phosphorylations were obtained under different treatment conditions (as shown in Fig.19), it makes them prime candidates for mediating biological effects of FAS and etoposide treatments, and demonstrates that LinkPhinder is useful for making interesting biologically relevant predictions.

Importantly, most of these experimentally validated predictions could not be predicted by any of the existing prediction tools. Specifically, one tool (GPS) could predict one of the predictions (LATS1 - Ser464) on high stringency (and no more on lower stringencies). The other five tools could not reproduce any of our predictions independently of the settings these tools provide. When cross-referencing the list of LATS1 predictions made by other tools, no addition predictions which could not be made by LinkPhinder were found.

Summarising, these results demonstrate that LinkPhinder can make many useful, biologically meaningful and experimentally verifiable predictions that other tools cannot.

### 2.6 LinkPhinder Results Overview

In a computational validation, the system achieved 0.906 ($\pm$ 0.02) and 0.958 ($\pm$ 0.006) scores in the metrics of area under the precision-recall and ROC curve, respectively. This shows high generalisation power. To evaluate the capability of the tool in terms of predicting previously unknown phosphorylations, two types of laboratory validations were performed - experiments targeted at selected high-stringency phosphorylation predictions on two clinically significant kinases (LATS1, AKT1), and mass-spectrometry experiments focused on substrates phosphorylated by LATS1. The targeted experiments confirmed two actual discoveries: LATS1 phosphorylates CREB1 at Ser133 and AKT1 phosphorylates STK3 at Ser18. None of these discoveries could be predicted with four out of five state of the art tools starting with the LATS1 or AKT1 kinases as queries (only GPS and PhosphoPredict support such queries, but only for less than 25% and 3.5% of the kinases covered by the LinkPhinder, respectively). Querying for the substrates did not reproduce the LinkPhinder high-stringency predictions with any tool on high stringency settings. On medium stringency, the GPS tool could reproduce one prediction (the CREB1 phosphorylation by LATS1). On low stringency, the NetPhosK tool could reproduce the other prediction (the STK3 phosphorylation by AKT1).

A complementary laboratory validation cross-referenced the high- and medium-stringency predictions with mass spectrometry data on LATS1. This confirmed five more previously unknown auto-phosphorylations of LATS1 (high stringency), and two phosphorylations of MAP4 and ZMYM2 by LATS1 (medium stringency). Five tools out of six could not reproduce any of these predictions at all. The GPS tool came up with one of the predictions (14%). When cross-referencing the list of all predictions GPS can make with the inventors' mass spectrometry data on LATS1, no additional predictions were found which the LinkPhinder tool could not make. These results clearly demonstrate superiority of the presented approach when compared to related state of the art solutions, and show high potential for increasing the pace of discovery in phosphoproteomics.

**Discussion**

LinkPhinder overcomes several limitations of the current phosphorylation prediction tools by representing phosphorylation networks as knowledge graphs, which is a novel approach that has never been tried before in this domain. Knowledge graphs are a relatively new approach to representing relational knowledge in the Machine Learning, Artificial Intelligence and Semantic Web communities. They have quickly gained popularity for two main reasons. First of all, they can represent diverse types of knowledge using a simple format. Secondly, they are amenable to robust techniques of statistical relational learning that can be used for tasks like discovering new facts. The discovery naturally makes use of the entire structure of the knowledge graph (i.e. latent features and long range, implicit relationships instead of just local, explicit features). This makes the representation very useful in domains where complex network dependencies are assumed. Since phosphoproteomics is an example of such domain, the inventors' initial assumption was that representing phosphorylation networks as knowledge graphs can bring radically new possibilities in terms of making accurate predictions on a broad range of proteins. The presented results demonstrate that this thought has been correct.

In particular, it has been shown that knowledge graphs can "naturally" capture the essence of kinase networks for which kinases, phosphorylation motifs and substrates take the roles of subject, predicate and object. Therewith, modern link prediction methods can be used to predict novel phosphorylation reactions and estimate their likelihood based on the entire network context. The model built on top of knowledge graphs and link prediction techniques allows for making predictions about any protein represented in the input data. This is a substantial usability advantage when compared with the existing tools as they often only allow for substrates as initial queries, and include only a limited number of kinases. It has been demonstrated that arrangements not only cover a much broader range of possible phosphorylations than existing tools, but also exhibit very high generalisation power. This aspect has been validated in experiments showing that arrangements can generate many biologically valid predictions that are not possible with other tools.

**Annex A**

**Detailed Description of the Computational Experiments**

**A.1 Construction of the Phosphorylation Network and Knowledge Graph for Training the Model**

The construction of the phosphorylation network requires data sources containing relation information of kinase, substrate and substrate's amino acid phosphorylation site. The experiments used PhosphoSitePlus® kinase-substrate dataset, an experimentally determined substrates, sequences, cognate kinases, and metadata curated from the literature. Only relations involving a kinase and substrate protein for the Human species were considered (KIN ORGANISM == SUB ORGANISM == 'human'). Although the dataset includes phosphorylation site's amino acids context sequence of size 7, that information was not used in order to experiment with different and potentially larger context sequence sizes. Instead the context sequence from UniProt (Universal Protein Resource) was extracted and more specifically from the reviewed (Swiss-Prot) main protein sequence (uniprot_sprot.fasta) and from isoform sequences (uniprot sprot varsplic.fasta). Any relation in the kinase-substrate dataset for which the phosphorylation site does not match the UniProt sequence. Table 31 presents some statistics about the phosphorylation network.

**[Table 311**

| Phosphorylation network components statistics | No. of elements in the phosphorylation network |
|---|---|
| Phosphorylation relations | 9,802 |
| Kinases | 327 |
| Substrates | 2,350 |
| Phosphorylation sites | 7,083 |
| Avg. No. of substrate/kinase | 7.19 |
| Avg. No. of substrate's site/kinase | 21.66 |

The knowledge graph conversion makes use of kinase family consensus motifs to transform phosphorylation network statements to knowledge graph relations. Kinase families dictionary is extracted from UniProt's Human and mouse protein kinases: classification and index. Only information about human kinases part of the phosphorylation network is kept.

The conversion of phosphorylation network data into knowledge made use of the MEME tool. MEME was applied in parallel on batches of site context sequences drawn from substrates targeted by kinases of the same family. The size of the batches was a configurable hyper-parameter of the conversion and model training process. Values were used ranging over {50, 100}. The static parameters used for every invocation of the MEME tool were: -text, -protein, -mod zoops, -x branch, -minw 2.

The MEME parameters that were dependent on the specific properties of the sequence batch and/or hyper-parameters of the whole model were: '-maxw MW, -maxsize MS, - nmotifs NM, -bfile BF where MW was the maximum width of a sequence in the batch, MS was the maximum width multiplied by the number of sequences in the batch, NM was the maximum number of motifs to be generated (set conservatively to 10 in the reported experiments as no batch generated more motifs than that number under any tested settings) and BF was a background Markov model of order 5 generated from the sequence batch.

Table 32 presents some statistics about the generated knowledge graph.

**[Table 32]**

| Knowledge graph components statistics | No. of elements in the knowledge graph |
| --- | --- |
| Motif-based relations | 9,956 |
| Kinase families | 12 |
| Kinase family motifs (predicate | 24 |
| Avg. No. of motif/kinase family | 2.00 |

## A.2 Construction of the State of the Art Prediction Datasets

**Scansite 3** Scansite searches for motifs within protein substrates that are likely to be phosphorylated by a specific protein kinase. Scansite takes as input a protein substrate ID and sequence and gives as output a confidence score for given substrate amino acid sites to be phosphorylated by one of 70 kinases handled by the system. The system was queried with all 93 substrates contained in the validation dataset. Scansite can only provide prediction for AKT1 and MAPK1 kinases. Only the system statement responses that intersect with the validation set were kept (same kinase, phosphorylation site, substrate) regardless of the produced score.

**NetworKIN and NetPhorest** KinomeXplorer framework contains results of both NetworKIN and NetPhorest systems with only the score changing and over a dump for download. KinomeXplorer dataset uses gene identifiers to refer to protein phosphorylation. In order to compare the results with the validation set first UniProt gene query was used to recover the protein identifier. After downloading the dataset, UniProt was queried using both EmbID and gene name to resolve a protein ID. In case a query did not yield any result or multiple proteins are returned, the original statement was dropped. Finally only the system protein identifier-based statement responses were kept that intersected with the validation set (same kinase, phosphorylation site, substrate).

**NetPhos 3.1** The NetPhos 3.1 system predicts serine, threonine or tyrosine phosphorylation sites in eukaryotic proteins using ensembles of neural networks. The system can provide predictions for 17 kinases only including AKT1 from the validation dataset. Using the stand-alone software package, the system was queried with all 93 substrates and associated sequences contained in the validation dataset. Finally only NetPhos 3.1 statement responses that intersected with the validation set were kept (same kinase, phosphorylation site, substrate).

**GPS 3.0** Group-based Prediction System (GPS) predicts phosphorylation sites with their cognate protein kinases using a four level kinase hierarchical structure in multiple species. Thanks to the desktop application, the batch predictor was used to pull out results for the 93 substrates and associated sequences contained in the validation dataset. The system handles protein kinases MAPK1, AKT1 and AKT2. Only statement responses that intersected with the validation set were kept (same kinase, phosphorylation site, substrate).

**PhosphoPredict** The PhosphoPredict system predict kinase-specific substrates and the corresponding phosphorylation sites for 12 human kinases, including ATM, CaM, CDKs, CK1, CK2, GRK, GSK-3, MAPKs, PKA, PKB (aka. AKT1), PKC and SRC. PhosphoPredict employs a feature selection method based on the minimum Redundancy and Maximum Relevance (mRMR) to select the most informative feature subsets that contribute to the prediction success of each of the kinase families. Only statement responses corresponding to AKT1 were kept (the system cannot handle LATS1 kinase).

**B Detailed description of the Biological Experiments**

**Cell culture experiments for targeted validation**

Hek-293 cells were regularly grown in Dulbeccos modified medium supplemented with 10% foetal serum. Subconfluent cell were transfected with Lipofectamine (Invitrogene) following manufacturers instructions. pSG5-gag-AKT was described in the art. LATS1 siRNA and AKT siRNA were from Dharmacorn and sequences have been described in the art. 24 hours after tranfection HEK293 cells were serum deprived for 16 hours. Subsequently, cell were lysed in 20mM HEPES (pH 7.5), 150 mM NaCl, 1% NP-40, 2mM NaF, 10mMb-Gycerolphosphate, 2 MM Na4P2O4 and protease and phosphatase inhibitors. Cell lysates were separated by SDS-PAGE analysed by western blotting. Phosphorylated proteins were immunoprecipitated with pAKT-Substrate specific antibody. Briefly, the lysates were incubated with 1 ul of antibody and 5ul of protein-G sepharose beads for 1 hour at 4C in an orbital wheel. The immunoprecipitates were washed 3 times with lysis buffer. 2 bed volumes of denaturing laemli buffer were added to the dry pelleted beads and immunocomplex were eluted by boiling the samples at 100C for 5 minutes. Anti-creb, antiLATS1 anti-P53 anti-tub, mTOR, p-YAP-S127.

**Mass-spectrometry experiments for extended validation**

HeLa cells were transiently transfected with GFP-tagged LATS1 construct. After 2 days they were either starved overnight or treated with FasL, Etoposide 3. Cells were lysed with Lysis buffer (20mM 4-(2 hydroxyethyl)-1piperazineethanesulfonic acid (HEPES) pH7.5, 150mM NaCl, 1% NP-40, Phosphatase Inhibitors, 10 mM -Gly, 1 Mm Na3VO4, 2mM Na4P2O7, 2 mM Na), Protease Inhibitor 5g/ml Leu) and proteins were immunoprecipitated using GFP-trap A Chromotek. The beads were washed 3 times using a washing buffer. The proteins immunoprecipitated onto GFP-beads were digested as described in the art. Briefly, the immunoprecipitates were digested in two steps. Firstly, by adding 60l of elution buffer-1 (2M urea, 50mM Tris-HCL pH7.5, 5g/ml Tryspin), to each sample and incubated at 27C on shaker. After 30 minutes incubation the samples were centrifuged at 13000 rpm for 30 seconds and the supernatant was collected into a new autoclaved Eppendorf. Secondly by adding 2 times 25l of elution buffer-2 (2M urea, 50mM Tris-HCL pH7.5, 1mM Dithiothreitol), per sample followed by centrifugation as above, the supernatant was collected into an autoclaved Eppendorf. Both supernatants were combined and incubated overnight at room temperature. Samples were alkylated (20 l iodoacetamide, 5mg/ml, 30 min in dark at room temperature). Then, the reaction was stopped by adding 1 l 100% Trifluoracetic acid (TFA) to each sample and 100 l of the sample was immediately loaded into equilibrated handmade C18 StageTips containing Octadecyl C18 disks (Supelco). Tips were previously activated by loading 50l of 50% AcN+ 0.1%TFA, after a quick centrifugation another 50l of 0.1%TFA was loaded to the tip and discarded. Samples were desalted by using two times 50 l of 0.1% TFA and eluted with two times 25 l of 50% AcN and 0.1% TFA solution. Final eluates were combined and concentrated until volume was reduced to 5l using a CentriVap Concentrator (Labconco). Samples were diluted to obtain a final volume of 15 l by adding 0.1% TFA and centrifuged for 10 minutes at 13000rpm. 12l of the samples was transferred into a micro-vial PP 0.3ml with Snap Ring (VWR) locked with a snap ring cap 11mm, blue (VWR) and analysed by MS. Data were acquired with the mass spectrometer operating in automatic data dependent switching mode selecting the 12 most in- tense ions prior to MS/MS analysis. Mass spectra were analysed by MaxQuant. Label-free quantitation was performed using MaxQuant.

**Peptide identification**

MaxQuant (version 1.3.0.5.) was used to analyse raw mass spectrometric data files from LC-MS/MS for protein quantification. Default settings were used unless stated otherwise, including the following parameters: Trypsin/P digest: variable modifications included Oxidation and Acetyl: fixed modification included Carbamidomethyl (Cys) only: multiplicity = 1: first search at 20 ppm : main search at 6 ppm mass accuracy (MS) and 20 mass deviation for the fragment ions: data searched against a human database (Uniprot HUMAN): minimum peptide length of 6: unfiltered for labelled amino acids: false discovery rate (FDR) of 0.01 selected for peptides and proteins: results refined through re-quantify option: match between runs selected with 1 min time window: label free quantification with minimum ratio count set at 1.

**Computer Implementation**

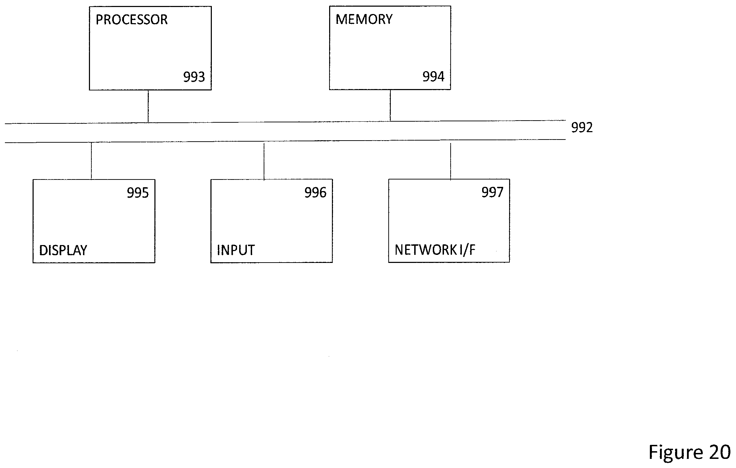

Figure 20 is a block diagram of a computing device, such as a data storage server which may be used to implement a method of finding specific phosphorylation sites. The computing device comprises a processor 993, and memory, 994. Optionally, the computing device also includes a network interface 997 for communication with other computing devices, for example with other computing devices of arrangements, or for connection to the phosphorylation data set/network 102, protein sequence database 103 and kinase family database 104 used in arrangements. Alternatively, this information may be stored in local data storage.

For example, an arrangement may be composed of a network of such computing devices. Optionally, the computing device also includes one or more input mechanisms such as keyboard and mouse 996, for example to input proteins of interest 110, and a display unit such as one or more monitors 995, which may display results such as Table 30 to the user. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of the modules (the converters 105, 103, the link predictor 111 their subcomponents) described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more arrangements, a processor is configured to execute instructions for performing the operations and steps discussed herein.

The display unit 997 may display a representation of data stored by the computing device (for example the various statements input and created) and may also display a cursor and dialog boxes and screens enabling interaction between a user and the programs and data stored on the computing device. The input mechanisms 996 may enable a user to input data and instructions to the computing device, such as any parameters for the relational learner and any proteins of interest.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

The converters 105, 113 may comprise processing instructions stored on a portion of the memory 994, the processor 993 to execute the processing instructions, and a portion of the memory 994 to store the statements during the execution of the processing instructions for conversion between motif-based and site-specific statements. The converted statements may be stored on the memory 994 and/or on a connected storage unit.

The link predictor 111 may comprise processing instructions stored on a portion of the memory 994, the processor 993 to execute the processing instructions, and a portion of the memory 994 to store the statements during the execution of the processing instructions for producing candidate statements, negative statements, negative candidate statements and predicted statements. The converted statements may be stored on the memory 994 and/or on a connected storage unit.

Methods may be carried out on a computing device such as that illustrated in Figure 20. Such a computing device need not have every component illustrated in Figure 20, and may be composed of a subset of those components. A method may be carried out by a single computing device in communication with one or more data storage servers via a network. The computing device may be a data storage itself storing the various statements including the final predicted statements and their scores.

A method may be carried out by a plurality of computing devices operating in cooperation with one another. One or more of the plurality of computing devices may be a data storage server storing at least a portion of various statements.

**Glossary**

Kinase

"A type of enzyme (a protein that speeds up chemical reactions in the body) that adds chemicals called phosphates to other molecules, such as sugars or proteins. This may cause other molecules in the cell to become either active or inactive. Kinases are a part of many cell processes. Some cancer treatments target certain kinases that are linked to cancer." https://www.cancer.gov/publications/dictionaries/cancer-terms?CdrID=641114

Phosphorylation

Phosphorylation (the addition of a phosphoryl group $PO_3$) is one of the most important protein interactions essential to many major signalling pathways in all live organisms. It is a directed and context-dependent relation - an agent (*kinase*) protein $K$ phosphorylates a patient (*substrate*) protein $S$ at a site $L$ (a *location* within the amino acid chain of the substrate protein). Phosphorylation regulates the behaviour of the substrate protein - it may be activated or deactivated, or its function may be modified in a more complex way. The downstream impact of phosphorylation pathways often results in major changes in the behaviour of the corresponding cells - the cells may start growing, proliferating or enter the process of programmed cell death (apoptosis).

Phosphorylation Site

*A* location (at an amino acid) within the amino acid chain of the substrate protein.

Phosphorylation Site Context (of size 2k+1)

A subsequence of the substrate amino acid sequence that covers the phosphorylation site at its middle and k amino acids on both left and right side.

Phosphorylation Network

A knowledge base of (human or other animal) phosphorylation reactions, made of relations between kinases and specific phosphorylation sites in substrates.

Substrate

"In chemistry, a substrate is typically the chemical species being observed in a chemical reaction, which reacts with reagent to generate a product". https://en.wikipedia.org/wiki/Substrate (chemistry)

Signalling pathway

"Describes a group of molecules in a cell that work together to control one or more cell functions, such as cell division or cell death. After the first molecule in a pathway receives a signal, it activates another molecule. This process is repeated until the last molecule is activated and the cell function is carried out. Abnormal activation of signaling pathways may lead to cancer, and drugs are being developed to block these pathways. These drugs may help block cancer cell growth and kill cancer cells." https://www.cancer.gov/publications/dictionaries/cancer-terms?cdrid=561720

Motif

A common (or consensus) sequence pattern of a protein, with a likely biological function. It may be a part or all of a protein sequence and in this context is derived as a consensus from a group of sequences. It may be represented as a "position-specific scoring matrix" or PSSM. A motif may provide the log likelihood score for each amino acid at a given position in a sequence. In arrangements, motifs directly correspond to predicates in the phosphorylation knowledge graphs.

Site-specific statement

A site-specific statement represents a phosphorylation interaction between a protein kinase and a substrate kinase along with the specific amino acid residue (site location) where the phosphorylation happen on the protein residue.

It is assumed that the statement is of the triple form set out below, but it may form a longer tuple.
⟨*protein kinase, phosphorylation site location, protein substrate*⟩.

Motif-based statement

A motif-based statement represents a phosphorylation interaction between a protein kinase and a protein substrate with an abstract (non-example specific) notion of phosphorylation site: motif. This motif is learned from regularities in phosphorylation site sequence among a kinase family. A motif provides the log likelihood score for each amino acid at a given position in a sequence. Motif-based statements are of the tuple form as above: ⟨*protein kinase, motif, protein substrate*⟩.

A set of such statements naturally forms a phosphorylation knowledge graph where kinases and substrates correspond to subjects and objects, respectively, and the motifs play the role of predicates (as they can be meaningfully compared across different statements, which would not be possible with the mere substrate-dependent site location in the site-specific statements).

Protein context statement

A protein context statement is a relational statement related to protein kinases and protein substrates such as additional protein-protein interactions. It comes in the form of a tuple, as for the other statements:
⟨*subject, relation, object*⟩,
indicating that *subject* and *object* entities hold a relationship *relation*.

Candidate statement

Candidate statements are the statements to be considered during the prediction step by the system. Thus they are statements that are not contained in the initial phosphorylation network but are predicted as possible phosphorylations by the model. Candidate statements may be based on the kinases of interest given as an input by the user. The prediction step consists of associating a ranking score to selected candidate statements by a trained model. The ranking can then be straightforwardly used for determining the most likely unknown phosphorylations (according to the model and a selected decision threshold). The candidate statements may be typically site-specific, converted to the motif-based and back during the prediction process. They may include information as to a probability of the candidate statement.

Knowledge Graph

A collection of items (represented by *nodes or entities*) any of which may be connected by links (i.e. binary relations) between them. They may be understood as a graph-structured knowledge base/database.

If the links are directed, knowledge graphs are typically represented as a set of relations in the form:
<subjects, predicate, objects>,
where predicate represents type of the relationship, and subject and object are typically interpreted as the active and passive entities, respectively. In the phosphoproteomics context the predicate would correspond to phosphorylation, while the subject and object would be a kinase and substrate, respectively.

Knowledge Graph Embedding

Representation of the knowledge graph entities and relations as vectors in a low-dimensional continuous space, computed from the relational information contained in the knowledge graph while preserving its inherent structure. Can be used for efficient solutions of inherently multi-variate, large scale problems (e.g. discovery in knowledge graphs).

Statistical relational learning

A machine learning branch in which a feature vector representing an input item may contain its relationships to other input items.

Link Prediction

A technique that attempts to estimate the likelihood of the existence of a link between two nodes, based on observed links and attributes of nodes. The typical use case is discovery of new links not explicitly present in a knowledge graph.

**Claims**

1. A system for discovery of predicted site-specific protein phosphorylation statements, the statements linking a kinase, a predicted phosphorylation site, and a protein substrate, the system comprising:

   a motif converter which is to receive inputs from:

      a phosphorylation data set storing known site-specific statements each in a site-specific format linking a kinase, a phosphorylation site and a protein substrate;
      a kinase family database; and
      a protein sequence database,

   the motif converter being arranged to use the site-specific statements and additional information from the kinase family database and protein sequence database to produce motif-based phosphorylation statements, <K, M, S>, each in a generalised format linking a kinase, K, a motif, M, in the form of a generalised scoring matrix for amino acids at a phosphorylation site and to either side of the phosphorylation site; and a protein substrate, S; a link predictor to predict new motif-based statements, the link predictor including:

      a negative motif-based statements generator to generate negative statements from the motif-based statements;
      a candidate generator to generate candidate statements in the generalised format by combination of the motif-based statements and to generate negative candidate statements by combination of the candidate statements with the negative statements; and
      a relational learner to use the candidate statements and the negative candidate statements in a statistical relational learning model to score the candidate statements and retain predicted motif-based phosphorylation statements in the generalised format; and

   a site-specific converter to receive inputs from the protein sequence database and to receive the predicted motif-based phosphorylation statements in the generalised format and which is arranged to produce predicted site-specific phosphorylation statements in the site-specific format by using a motif conformance score for possible phosphorylation sites; wherein
   the negative motif-based statements generator is to create negative statements by one or more of:

      replacing the substrate in a motif-based statement <K, M, S> with a different substrate in that for each phosphorylation site on the different substrate, the context sequence is compared with the motif of the motif-based statement, and if the conformance is negative, the substrate S in a motif-based statement <K, M, S> is replaced by a different substrate S' and a negative statement <K, M, S'> is provided; and
      replacing the kinase in a motif-based statement with a kinase from a different kinase family in that for each phosphorylation site on the different substrate, the context sequence is compared with the motif of the motif-based statement, and if the conformance is negative, the kinase K in a motif-based statement <K, M, S> is replaced by a different kinase K' and a negative statement <K', M, S> is provided.

2. A system according to claim 1, wherein
   the motif converter is to:

      associate each site-specific statement with a kinase family in the kinase family database;
      use the protein sequence database to form a context sequence of an amino acid at the phosphorylation site for each site-specific statement and a plurality of amino acids to either side of the phosphorylation site;
      group the context sequences by kinase family;
      for the context sequences in each kinase family, compute one or more position-specific scoring matrices each of which provides a score for each amino acid at each position of the context sequence length, each position-specific scoring matrix defining a motif;
      for each site-specific statement, compute the conformance between the context sequence for that site-specific statement and each motif in the kinase family in which the context sequence is grouped; and to

   for each site-specific statement, create a motif-based statement linking the kinase, the best performing motif and the protein substrate.

3. A system according to any of the preceding claims, wherein the negative motif-based statements generator is configured to compare the negative statements and the motif-based statements and to delete from the negative statements any statement that appears both as one of the negative statements and as one of the motif-based statements.

4. A system according to any of the preceding claims, wherein a negative statements filter in the candidate generator is also to, for each kinase and motif in a candidate statement, create negative candidate statements as the combination of the kinase, the motif and all substrates associated with the kinase and the motif in the negative statements.

5. A system according to any of the preceding claims, wherein a negative statements filter in the candidate generator is also to, for each motif and substrate in a candidate statement, create negative candidate statements as the combination of the motif, the substrate and all kinases associated with the substrate and the motif in the negative statements.

6. A system according to claim 3, 4 or 5, wherein the negative statements filter is to discard from the negative candidate statements any statements that appears both as one of the negative candidate statements and as one of the candidate statements.

7. A system according to any of the preceding claims, wherein the relational learner is to input motif-based statements and negative statements and to learn a model from these inputs which is then used to score the candidate statements and the negative candidate statements.

8. A system according to any of the preceding claims, wherein the candidate generator is to create candidate statements as the combination of every kinase included in the motif-based statements with every motif included in the motif-based statements and every substrate included in the motif-based statements minus the motif-based statements themselves.

9. A system according to any of the preceding claims, wherein the relational learner is to score and rank a candidate statement and all the negative candidate statements with the same kinase and motif or with the same motif and substrate and to output the rank and score of the candidate statement.

10. A system according to any of the preceding claims, further comprising a protein statements generator to receive input from a protein context database linking proteins by their interactions and to produce protein context statements, which are for use in the link predictor.

11. A system according to any of the preceding claims, wherein the site-specific converter is to:

   input the predicted motif-based phosphorylation statements;
   for each predicted motif-based phosphorylation statements extract a list of possible phosphorylation sites from a phosphorylation sites database;
   for each phosphorylation site, extract the site context sequence from the protein sequence database and compute the conformance of the site context sequence with the motif in the predicted motif-based phosphorylation statement;
   where the conformance for the phosphorylation site is above a predefined score, produce a predicted site-specific phosphorylation statement in the site-specific format linking the kinase and the substrate in the predicted motif-based statement with the phosphorylation site.

12. A computer-implemented method for discovery of predicted site-specific protein phosphorylation statements, the statements linking a kinase, a predicted phosphorylation site, and a protein substrate, the method comprising:
   using data from:

   a phosphorylation data set storing known site-specific statements each in a site-specific format linking a kinase, a phosphorylation site and a protein substrate;
   a kinase family database; and
   a protein sequence database,
   using the site-specific statements and additional information from the kinase family database and protein sequence database to produce motif-based phosphorylation statements each in a generalised format linking a kinase, a motif in the form of a generalised scoring matrix for amino acids at a phosphorylation site and to either side of the phosphorylation site; and a protein substrate;

generating negative statements from the motif-based statements;

generating candidate statements in the generalised format by combining the motif-based statements;

generating negative candidate statements by combination of the candidate statements with the negative statements;

using the candidate statements and negative candidate statements in a statistical relational learning model to score the candidate statements and retain predicted motif-based phosphorylation statements in the generalised format;

receiving data inputs from the protein sequence database and the predicted motif-based phosphorylation statements in the generalised format and producing predicted site-specific phosphorylation statements in the site-specific format by using a motif conformance score for possible phosphorylation sites; wherein the generation of negative motif-based statements generator creates negative statements by one or more of:

replacing the substrate in a motif-based statement <K, M, S> with a different substrate in that for each phosphorylation site on the different substrate, the context sequence is compared with the motif of the motif-based statement, and if the conformance is negative, the substrate S in a motif-based statement <K, M, S> is replaced by a different substrate S' and a negative statement <K, M, S'> is provided; and

replacing the kinase in a motif-based statement with a kinase from a different kinase family in that for each phosphorylation site on the different substrate, the context sequence is compared with the motif of the motif-based statement, and if the conformance is negative, the kinase K in a motif-based statement <K, M, S> is replaced by a different kinase K' and a negative statement <K', M, S> is provided.

13. A computer-implemented method according to claim 12, further comprising testing whether there is phosphorylation as predicted by the predicted site-specific phosphorylation statements in a physical laboratory to verify whether the prediction is correct.

14. A computer program comprising instructions, which when executed by a computer, cause the computer to carry out the method of claim 12.

**Patentansprüche**

1. System zur Entdeckung von vorhergesagten ortsspezifischen Proteinphosphorylierungsaussagen, wobei die Aussagen eine Kinase, einen vorhergesagten Phosphorylierungsort und ein Proteinsubstrat verknüpfen, wobei das System umfasst:
einen Motivkonverter, der Eingaben empfangen soll von:

einem Phosphorylierungsdatensatz, in dem bekannte ortsspezifische Aussagen gespeichert sind, die jeweils in einem ortsspezifischen Format eine Kinase, einen Phosphorylierungsort und ein Proteinsubstrat verknüpfen;
einer Kinase-Familiendatenbank; und
einer Proteinsequenzdatenbank,
wobei der Motivkonverter dazu ausgelegt ist, die ortsspezifischen Aussagen und zusätzliche Informationen aus der Kinasefamiliendatenbank und der Proteinsequenzdatenbank zu verwenden, um motivbasierte Phosphorylierungsaussagen, <K, M, S>, zu erzeugen, die jeweils in einem verallgemeinerten Format eine Kinase, K, ein Motiv, M, in Form einer verallgemeinerten Bewertungsmatrix für Aminosäuren an einem Phosphorylierungsort und zu beiden Seiten des Phosphorylierungsorts und ein Proteinsubstrat, S, verknüpfen;
einen Verknüpfungsprädiktor zum Vorhersagen neuer motivbasierter Aussagen, wobei der Verknüpfungsprädiktor Folgendes beinhaltet:

einen Generator für negative motivbasierte Aussagen, um negative Aussagen aus den motivbasierten Aussagen zu erzeugen;
einen Kandidatengenerator zum Erzeugen von Kandidatenaussagen in dem verallgemeinerten Format durch Kombination der motivbasierten Aussagen und zum Erzeugen von negativen Kandidatenaussagen durch Kombination der Kandidatenaussagen mit den negativen Aussagen; und
einen relationalen Lerner, um die Kandidatenaussagen und die negativen Kandidatenaussagen in einem statistischen relationalen Lernmodell zu verwenden, um die Kandidatenaussagen zu bewerten und die vorhergesagten motivbasierten Phosphorylierungsaussagen in dem verallgemeinerten Format zu behalten; und

einen ortsspezifischen Konverter, um Eingaben von der Proteinsequenzdatenbank zu empfangen und die vorhergesagten motivbasierten Phosphorylierungsaussagen in dem verallgemeinerten Format zu empfangen, und der dazu ausgelegt ist, vorhergesagte ortsspezifische Phosphorylierungsaussagen in dem ortsspezifischen Format zu erzeugen, indem ein Motivkonformitätswert für mögliche Phosphorylierungsorte verwendet wird; wobei

der Generator für negative motivbasierte Aussagen dazu dient, negative Aussagen durch eine oder mehrere der Folgenden zu erzeugen:

Ersetzen des Substrats in einer motivbasierten Aussage <K, M, S> durch ein anderes Substrat, indem für jeden Phosphorylierungsort auf dem anderen Substrat die Kontextsequenz mit dem Motiv der motivbasierten Aussage verglichen wird, und wenn die Übereinstimmung negativ ist, das Substrat S in einer motivbasierten Aussage <K, M, S> durch ein anderes Substrat S' ersetzt wird und eine negative Aussage <K, M, S'> bereitgestellt wird; und

Ersetzen der Kinase in einer motivbasierten Aussage durch eine Kinase aus einer anderen Kinasefamilie, indem für jeden Phosphorylierungsort auf dem anderen Substrat die Kontextsequenz mit dem Motiv der motivbasierten Aussage verglichen wird, und

wenn die Übereinstimmung negativ ist, die Kinase K in einer motivbasierten Aussage <K, M, S> durch eine andere Kinase K' ersetzt wird und eine negative Aussage <K', M, S> bereitgestellt wird.

2. System nach Anspruch 1, wobei
der Motivkonverter zu Folgendem dient:

Zuordnen jeder ortsspezifischen Aussage zu einer Kinasefamilie in der Kinasefamiliendatenbank;
Verwenden der Proteinsequenzdatenbank, um eine Kontextsequenz einer Aminosäure an dem Phosphorylierungsort für jede ortsspezifische Aussage und eine Vielzahl von Aminosäuren zu beiden Seiten des Phosphorylierungsorts zu bilden;
Gruppieren der Kontextsequenzen nach Kinasefamilie;
für die Kontextsequenzen in jeder Kinasefamilie, Berechnen einer oder mehrerer positionsspezifischer Bewertungsmatrizen, von denen jede eine Bewertung für jede Aminosäure an jeder Position der Länge der Kontextsequenz bereitstellt, wobei jede positionsspezifische Bewertungsmatrix ein Motiv definiert;
für jede ortsspezifische Anweisung, Berechnen der Konformität zwischen der Kontextsequenz für diese ortsspezifische Anweisung und jedem Motiv in der Kinasefamilie, in der die Kontextsequenz gruppiert ist; und zum
für jede ortsspezifische Anweisung, Erzeugen einer motivbasierten Anweisung, die die Kinase, das am besten funktionierende Motiv und das Proteinsubstrat verknüpft.

3. System nach einem der vorhergehenden Ansprüche, wobei der Generator für negative motivbasierte Aussagen dazu konfiguriert ist, die negativen Aussagen und die motivbasierten Aussagen zu vergleichen und aus den negativen Aussagen jede Aussage zu löschen, die sowohl als eine der negativen Aussagen als auch als eine der motivbasierten Aussagen erscheint.

4. System nach einem der vorhergehenden Ansprüche, wobei ein Filter für negative Aussagen im Kandidatengenerator auch dazu dient, für jede Kinase und jedes Motiv in einer Kandidatenaussage negative Kandidatenaussagen als Kombination der Kinase, des Motivs und aller der Kinase und dem Motiv zugeordneten Substrate in den negativen Aussagen zu erzeugen.

5. System nach einem der vorhergehenden Ansprüche, wobei ein Filter für negative Aussagen in dem Kandidatengenerator auch dazu dient, für jedes Motiv und Substrat in einer Kandidatenaussage negative Kandidatenaussagen als Kombination des Motivs, des Substrats und aller dem Substrat und dem Motiv zugeordneten Kinasen in den negativen Aussagen zu erzeugen.

6. System nach Anspruch 3, 4 oder 5, wobei der Filter für negative Aussagen dazu dient, aus den negativen Kandidatenaussagen alle Aussagen zu verwerfen, die sowohl als eine der negativen Kandidatenaussagen als auch als eine der Kandidatenaussagen erscheinen.

7. System nach einem der vorhergehenden Ansprüche, wobei der relationale Lerner motivbasierte Aussagen und negative Aussagen eingibt und aus diesen Eingaben ein Modell lernt, das dann zur Bewertung der Kandidatenaussagen und der negativen Kandidatenaussagen verwendet wird.

8. System nach einem der vorhergehenden Ansprüche, wobei der Kandidatengenerator Kandidatenaussagen als Kombination jeder in den motivbasierten Aussagen enthaltenen Kinase mit jedem in den motivbasierten Aussagen enthaltenen Motiv und jedem in den motivbasierten Aussagen enthaltenen Substrat abzüglich der motivbasierten Aussagen selbst erzeugt.

9. System nach einem der vorhergehenden Ansprüche, wobei der relationale Lerner dazu dient, eine Kandidatenaussage und alle negativen Kandidatenaussagen mit derselben Kinase und demselben Motiv oder mit demselben Motiv und Substrat zu bewerten und einzustufen und den Rang und die Bewertung der Kandidatenaussage auszugeben.

10. System nach einem der vorhergehenden Ansprüche, ferner umfassend einen Proteinaussagengenerator, um Eingaben von einer Proteinkontextdatenbank zu empfangen, die Proteine durch ihre Interaktionen verknüpft, und um Proteinkontextaussagen zu erzeugen, die zur Verwendung im Verknüpfungsprädiktor bestimmt sind.

11. System nach einem der vorhergehenden Ansprüche, wobei der ortsspezifische Konverter zu Folgendem dient:

Eingeben der vorhergesagten motivbasierten Phosphorylierungsaussagen;
für jede vorhergesagte motivbasierte Phosphorylierungsaussage, Extrahieren einer Liste von möglichen Phosphorylierungsorten aus einer Phosphorylierungsortdatenbank;
für jeden Phosphorylierungsort, Extrahieren der Kontextsequenz des Ortes aus der Proteinsequenzdatenbank und Berechnen der Konformität der Kontextsequenz des Ortes mit dem Motiv in der vorhergesagten motivbasierten Phosphorylierungsaussage;
wenn die Konformität für den Phosphorylierungsort über einer vordefinierten Punktzahl liegt, Erzeugen einer vorhergesagten ortsspezifischen Phosphorylierungsaussage im ortsspezifischen Format, die die Kinase und das Substrat in der vorhergesagten motivbasierten Aussage mit dem Phosphorylierungsort verknüpft.

12. Computerimplementiertes Verfahren zur Entdeckung von vorhergesagten ortsspezifischen Proteinphosphorylierungsaussagen, wobei die Aussagen eine Kinase, einen vorhergesagten Phosphorylierungsort und ein Proteinsubstrat verknüpfen, wobei das Verfahren umfasst:
Verwenden von Daten von:

einem Phosphorylierungsdatensatz, in dem bekannte ortsspezifische Aussagen gespeichert sind, die jeweils in einem ortsspezifischen Format eine Kinase, einen Phosphorylierungsort und ein Proteinsubstrat verknüpfen;
einer Kinase-Familiendatenbank; und
einer Proteinsequenzdatenbank,
Verwenden von ortsspezifischen Aussagen und zusätzlichen Informationen aus der Kinasefamiliendatenbank und der Proteinsequenzdatenbank, um motivbasierte Phosphorylierungsaussagen zu erzeugen, die jeweils in einem verallgemeinerten Format eine Kinase, ein Motiv in Form einer verallgemeinerten Bewertungsmatrix für Aminosäuren an einem Phosphorylierungsort und zu beiden Seiten des Phosphorylierungsorts und ein Proteinsubstrat verknüpfen;
Erzeugen negativer Aussagen aus den motivbasierten Aussagen;
Erzeugen von Kandidatenaussagen im verallgemeinerten Format durch Kombinieren der motivbasierten Aussagen;
Erzeugen negativer Kandidatenaussagen durch Kombination der Kandidatenaussagen mit den negativen Aussagen;
Verwenden der Kandidatenaussagen und der negativen Kandidatenaussagen in einem statistischen relationalen Lernmodell, um die Kandidatenaussagen zu bewerten und die vorhergesagten motivbasierten Phosphorylierungsaussagen in dem verallgemeinerten Format zu behalten;
Empfangen von Dateneingaben von der Proteinsequenzdatenbank und vorhergesagten motivbasierten Phosphorylierungsaussagen in dem verallgemeinerten Format, und Erzeugen von vorhergesagten ortsspezifischen Phosphorylierungsaussagen in dem ortsspezifischen Format, indem ein Motivkonformitätswert für mögliche Phosphorylierungsstellen verwendet wird; wobei die Erzeugung des Generators für negative motivbasierte Aussagen negative Aussagen durch eines oder mehrere von Folgendem erzeugt:

Ersetzen des Substrats in einer motivbasierten Aussage <K, M, S> durch ein anderes Substrat, indem für jeden Phosphorylierungsort auf dem anderen Substrat die Kontextsequenz mit dem Motiv der motivbasierten Aussage verglichen wird, und wenn die Übereinstimmung negativ ist, das Substrat S in einer motivbasierten Aussage <K, M, S> durch ein anderes Substrat S' ersetzt wird und eine negative Aussage <K, M, S'> bereitgestellt wird; und

Ersetzen der Kinase in einer motivbasierten Aussage durch eine Kinase aus einer anderen Kinasefamilie, indem für jeden Phosphorylierungsort auf dem anderen Substrat die Kontextsequenz mit dem Motiv der motivbasierten Aussage verglichen wird, und

wenn die Übereinstimmung negativ ist, die Kinase K in einer motivbasierten Aussage <K, M, S> durch eine andere Kinase K' ersetzt wird und eine negative Aussage <K', M, S> bereitgestellt wird.

**13.** Computerimplementiertes Verfahren nach Anspruch 12, ferner umfassend Testen, ob eine Phosphorylierung wie durch die vorhergesagten ortsspezifischen Phosphorylierungsaussagen vorhergesagt vorliegt, in einem physikalischen Labor, um zu überprüfen, ob die Vorhersage korrekt ist.

**14.** Computerprogramm, das Anweisungen umfasst, die, wenn sie von einem Computer ausgeführt werden, den Computer veranlassen, das Verfahren nach Anspruch 12 auszuführen.

**Revendications**

**1.** Système de découverte d'états de phosphorylation de protéines spécifiques à un site prédits, les états reliant une kinase, un site de phosphorylation prédit et un substrat protéique, le système comprenant :

un convertisseur de motifs qui est destiné à recevoir des entrées depuis :

un ensemble de données de phosphorylation stockant des états spécifiques à un site connus chacun dans un format spécifique à un site reliant une kinase, un site de phosphorylation et un substrat protéique ;
une base de données de familles de kinases ; et
une base de données de séquences protéiques,
le convertisseur de motifs étant agencé pour utiliser les états spécifiques à un site et des informations supplémentaires provenant de la base de données de familles des kinases et de la base de données de séquences protéiques pour produire des états de phosphorylation basés sur un motif, <K, M, S>, chacun dans un format généralisé reliant une kinase, K, un motif, M,
sous la forme d'une matrice de notation généralisée pour les acides aminés au niveau d'un site de phosphorylation et de part et d'autre du site de phosphorylation ; et un substrat protéique, S ;
un prédicteur de lien pour prédire de nouveaux états basés sur un motif, le prédicteur de lien comprenant :

un générateur d'états négatifs basés sur un motif pour générer des états négatifs à partir des états basés sur un motif ;
un générateur de candidats pour générer des états candidats dans le format généralisé par combinaison des états basés sur un motif et pour générer des états candidats négatifs par combinaison des états candidats avec les états négatifs ; et
un dispositif d'apprentissage relationnel pour utiliser les états candidats et les états candidats négatifs dans un modèle d'apprentissage relationnel statistique pour noter les états candidats et conserver les états de phosphorylation basés sur un motif prédits dans le format généralisé ; et
un convertisseur spécifique à un site pour recevoir des entrées de la base de données de séquences protéiques et pour recevoir les états de phosphorylation basés sur un motif prédits dans le format généralisé et qui est agencé pour produire des états de phosphorylation spécifiques à un site prédits dans le format spécifique à un site en utilisant une note de conformité de motif pour les sites de phosphorylation possibles ;
le générateur d'états négatifs basés sur un motif est destiné à créer des états négatifs par un ou plusieurs parmi :

le remplacement du substrat dans un état basé sur un motif <K, M, S> par un substrat différent en ce que pour chaque site de phosphorylation sur le substrat différent, la séquence de contexte est comparée au motif de l'état basé sur un motif, et
si la conformité est négative, le substrat S dans un état basé sur un motif <K, M, S> est remplacé par un substrat différent S' et un état négatif <K, M, S'> est fourni ; et
le remplacement de la kinase dans un état basé sur un motif par une kinase d'une famille de kinases différente en ce que pour chaque site de phosphorylation sur le substrat différent, la séquence de contexte est comparée au motif de l'état basé sur un motif, et si la conformité est négative, la kinase K dans un état basé sur un motif <K, M, S> est remplacée par une kinase différente K' et un état négatif <K', M, S> est fourni.

2. Système selon la revendication 1, dans lequel le convertisseur de motifs est destiné à :

associer chaque état spécifique à un site à une famille de kinases dans la base de données de familles de kinases ;

utiliser la base de données de séquences protéiques pour former une séquence de contexte d'un acide aminé au niveau du site de phosphorylation pour chaque état spécifique à un site et une pluralité d'acides aminés de chaque côté du site de phosphorylation ;

regrouper les séquences de contexte par famille de kinases ;

pour les séquences de contexte dans chaque famille de kinases, calculer une ou plusieurs matrices de notation spécifiques à une position dont chacune fournit un score pour chaque acide aminé au niveau de chaque position de la longueur de la séquence de contexte, chaque matrice de notation spécifique à une position définissant un motif ;

pour chaque état spécifique à un site, calculer la conformité entre la séquence de contexte pour cet état spécifique à un site et chaque motif de la famille de kinases dans laquelle la séquence de contexte est regroupée ; et à

pour chaque état spécifique à un site, créer un état basé sur un motif reliant la kinase, le motif le plus performant et le substrat protéique.

3. Système selon l'une quelconque des revendications précédentes, dans lequel le générateur d'états négatifs basés sur un motif est configuré pour comparer les états négatifs et les états basés sur un motif et pour supprimer des états négatifs tout état qui apparaît à la fois comme l'un des états négatifs et comme l'un des états basés sur un motif.

4. Système selon l'une quelconque des revendications précédentes, dans lequel un filtre d'états négatifs dans le générateur de candidats est également destiné, pour chaque kinase et chaque motif dans un état candidat, à créer des états candidats négatifs en tant que combinaison de la kinase, du motif et de tous substrats associés à la kinase et au motif dans les états négatifs.

5. Système selon l'une quelconque des revendications précédentes, dans lequel un filtre d'états négatifs dans le générateur de candidats est également destiné, pour chaque motif et chaque substrat dans un état candidat, à créer des états candidats négatifs en tant que combinaison du motif et du substrat et de toutes les kinases associées au substrat et au motif dans les états négatifs.

6. Système selon la revendication 3, 4 ou 5, dans lequel le filtre d'états négatifs est destiné à écarter des états candidats négatifs tout état qui apparaît à la fois comme l'un des états candidats négatifs et comme l'un des états candidats.

7. Système selon l'une quelconque des revendications précédentes, dans lequel le dispositif d'apprentissage relationnel est destiné à entrer des états basés sur un motif et des états négatifs et apprendre un modèle à partir de ces entrées qui est ensuite utilisé pour noter les états candidats et les états candidats négatifs.

8. Système selon l'une quelconque des revendications précédentes, dans lequel le générateur de candidats est destiné à créer des états candidats comme la combinaison de chaque kinase incluse dans les états basés sur un motif avec chaque motif inclus dans les états basés sur un motif et chaque substrat inclus dans les états basés sur un motif moins les états basés sur un motif eux-mêmes.

9. Système selon l'une quelconque des revendications précédentes, dans lequel le dispositif d'apprentissage relationnel est destiné à noter et classer un état candidat et tous les états candidats négatifs avec la même kinase et le même motif ou avec le même motif et le même substrat et produire le rang et la note de l'état candidat.

10. Système selon l'une quelconque des revendications précédentes, comprenant en outre un générateur d'états protéiques pour recevoir une entrée d'une base de données de contextes protéiques reliant des protéines par leurs interactions et pour produire des états de contexte protéique, qui sont destinés à être utilisés dans le prédicateur de lien.

11. Système selon l'une quelconque des revendications précédentes, dans lequel le convertisseur spécifique à un site est destiné à :

entrer les états de phosphorylation basés sur un motif prédits ;

pour chaque état de phosphorylation basé sur un motif prédit, extraire une liste de sites de phosphorylation possibles à partir d'une base de données de sites de phosphorylation ;

pour chaque site de phosphorylation, extraire la séquence de contexte de site de la base de données de séquences protéiques et calculer la conformité de la séquence de contexte de site avec le motif dans l'état de phosphorylation basé sur un motif prédit ;

lorsque la conformité pour le site de phosphorylation est supérieure à un score prédéfini, produire un état de phosphorylation spécifique à un site prédit dans le format spécifique à un site reliant la kinase et le substrat dans l'état basé sur un motif prédit avec le site de phosphorylation.

12. Procédé mis en œuvre par ordinateur de découverte d'états de phosphorylation de protéines spécifiques à un site prédits, les états reliant une kinase, un site de phosphorylation prédit et un substrat protéique, le système comprenant :

l'utilisation de données provenant :

d'un ensemble de données de phosphorylation stockant des états spécifiques à un site connus chacun dans un format spécifique à un site reliant une kinase, un site de phosphorylation et un substrat protéique ;
d'une base de données de familles de kinases ; et
d'une base de données de séquences protéiques,
l'utilisation des états spécifiques à un site et des informations supplémentaires provenant de la base de données de familles des kinases et de la base de données de séquences protéiques pour produire des états de phosphorylation basés sur un motif chacun dans un format généralisé reliant une kinase, un motif, sous la forme d'une matrice de notation généralisée pour les acides aminés au niveau d'un site de phosphorylation et de part et d'autre du site de phosphorylation ; et un substrat protéique ;
la génération d'états négatifs à partir des états basés sur un motif ;
la génération d'états candidats dans le format généralisé en combinant les états basés sur un motif ;
la génération d'états candidats négatifs en combinant les états candidats avec les états négatifs ;
l'utilisation des états candidats et les états candidats négatifs dans un modèle d'apprentissage relationnel statistique pour noter les états candidats et conserver les états de phosphorylation basés sur un motif prédits dans le format généralisé ;
la réception d'entrées de données provenant de la base de données de séquences protéiques et des états de phosphorylation basés sur un motif prédits dans le format généralisé et la production d'états de phosphorylation spécifiques à un site prédits dans le format spécifique à un site en utilisant une note de conformité de motif pour les sites de phosphorylation possibles ; dans lequel
la génération d'un générateur d'états négatifs basés sur un motif crée des états négatifs par un ou plusieurs parmi :

le remplacement du substrat dans un état basé sur un motif <K, M, S> par un substrat différent en ce que pour chaque site de phosphorylation sur le substrat différent, la séquence de contexte est comparée au motif de l'état basé sur un motif, et si la conformité est négative, le substrat S dans un état basé sur un motif <K, M, S> est remplacé par un substrat différent S' et un état négatif <K, M, S'> est fourni ; et
le remplacement de la kinase dans un état basé sur un motif par une kinase d'une famille de kinases différente en ce que pour chaque site de phosphorylation sur le substrat différent, la séquence de contexte est comparée au motif de l'état basé sur un motif, et si la conformité est négative, la kinase K dans un état basé sur un motif <K, M, S> est remplacée par une kinase différente K' et un état négatif <K', M, S> est fourni.

13. Procédé mis en œuvre par ordinateur selon la revendication 12, comprenant en outre le fait de tester s'il existe une phosphorylation telle que prédite par les états de phosphorylation spécifiques à un site prédits dans un laboratoire physique pour vérifier si la prédiction est correcte.

14. Programme informatique comprenant des instructions qui, lorsqu'elles sont exécutées par un ordinateur, amènent l'ordinateur à réaliser le procédé selon la revendication 12.

System Overview Block Diagram –Tech. Prob. A & B

Figure 1A

Figure 1B

System Overview Block Diagram —Tech. Prob. A & B

Figure 1C

EP 3 404 568 B1

Figure 2

System Overview Block Diagram –Tech. Prob. C

Figure 3

Conversion to motif-based statements Unit

```
                          ┌─────────────┐
                          │    Start    │
                          └──────┬──────┘
                                 │
                                 ▼
          ┌──────────────────────────────────────────────┐
     401  │      Read site-specific statements from       │
          │           phosphorylation network             │
          └───────────────────────┬──────────────────────┘
                                   │
                                   ▼
          ┌──────────────────────────────────────────────┐
     402  │  For each site-specific statement, add family │
          │    of the kinase from kinase family database  │
          └───────────────────────┬──────────────────────┘
                                   │
                                   ▼
          ┌──────────────────────────────────────────────┐
          │  For each site-specific statement, add site   │
     403  │  context amino acid sequence information       │
          │       from protein sequence database          │
          └───────────────────────┬──────────────────────┘
                                   │
                                   ▼
          ┌──────────────────────────────────────────────┐
     404  │     Group phosphorylation sequences per        │
          │               kinase family                    │
          └───────────────────────┬──────────────────────┘
                                   │
                                   ▼
          ┌──────────────────────────────────────────────┐
          │    For each kinase family, compute position-   │
     405  │    specific scoring matrices (= motifs).        │
          └───────────────────────┬──────────────────────┘
                                   │
                                   ▼
                          ┌─────────────┐
                          │     End     │
                          └─────────────┘
```

Motif Discovery Unit flowchart

Figure 4

```
            ( Start )
                │
                ▼
┌─────────────────────────────────────┐
│ Read site-specific statements with  │
│ kinase family and site context      │
│ sequence information                │
└─────────────────────────────────────┘
        501
                │
                ▼
┌─────────────────────────────────────┐
│ For each statement, compute the     │
│ conformance with every motif        │
│ obtained from the same kinase       │
│ family as the kinase in the         │
│ statement.                          │
└─────────────────────────────────────┘
        502
                │
                ▼
┌─────────────────────────────────────┐
│ Discard motifs candidates that has  │
│ negative conformance with a given   │
│ statement                           │
└─────────────────────────────────────┘
        503
                │
                ▼
┌─────────────────────────────────────┐
│ For each statement, generate a      │
│ motif-based statement of the form   │
│ <Kinase, Motif_ID, Substrate> for   │
│ the best conformant motif among     │
│ the motifs from the same family as  │
│ the kinase in the site-specific     │
│ statement                           │
└─────────────────────────────────────┘
        504
                │
                ▼
            ( End )
```

Motif-based Statement Generation Unit flowchart

Figure 5

Figure 6

108

Protein context
statements

107

Protein Context
Statements
Generation Unit

109

Motif-based
statements

106

Protein context
knowledge graph

Knowledge Base Enrichment Unit

EP 3 404 568 B1

Protein Context Statements Generation Unit

Figure 7

Link Prediction Unit

111 — Link Prediction Unit

201 — Negative Protein Context Statements Generation Unit

202 — Negative Motif-based Statements Generation Unit

203 — Candidate Generation Unit

204 — Relational Learning Unit

108 — Protein context statements

109 — Motif-based statements

110 — Proteins of interest

108 — Predicted motif-based statements

Figure 8

Start

701 — Read all motif-based statements

702 — For each input statement <K, M, S>, create a set of corresponding negative motif-based statements <K, M, S'>

703 — For each input statement <K, M, S>, create a set of corresponding negative motif-based statements <K', M, S>

704 — Discard any negative statement that exists in the input positive statements

End

Negative Motif-based Statements
Generation Unit flowchart

Figure 9

```
                    ┌──────────┐
                    │  Start   │
                    └──────────┘
                         │
                         ▼
     ┌─────────────────────────────────────────┐
801  │   Read all protein context statements   │
     └─────────────────────────────────────────┘
                         │
                         ▼
     ┌─────────────────────────────────────────┐
     │          For each input statement,      │
     │ build a new negative statement replacing only │
     │   the subject element by a randomly picked │
802  │      subject from the positive statements: │
     │        <new_subject, relation, object>  │
     └─────────────────────────────────────────┘
                         │
                         ▼
     ┌─────────────────────────────────────────┐
     │          For each input statement,      │
     │ build a new negative statement replacing only │
     │   the object element by a randomly picked object │
803  │ from the positive statements: <subject, relation, │
     │              new_object>                │
     └─────────────────────────────────────────┘
                         │
                         ▼
     ┌─────────────────────────────────────────┐
     │ Discard any negative statement that exists │
804  │      in the input positive statements   │
     └─────────────────────────────────────────┘
                         │
                         ▼
                    ┌──────────┐
                    │   End    │
                    └──────────┘
```

Negative Protein Context Statements
Generation Unit

Figure 10

Candidate Generation Unit

Figure 11

```
                          ┌─────────────┐
                          │    Start    │
                          └─────────────┘
              1000                  │
                                    ▼
          ┌──────────────────────────────────────┐
   1001   │      Read all motif-based statements  │
          └──────────────────────────────────────┘
                                    │
                                    ▼
          ┌──────────────────────────────────────┐
   1002   │        Read all kinases of interest   │
          └──────────────────────────────────────┘
                                    │
                                    ▼
          ┌──────────────────────────────────────┐
          │       Build candidate statements as the│
   1003   │  Cartesian product of all kinases of interest,│
          │   phosphorylation motif and substrates in │
          │     the positive motif-based statements   │
          └──────────────────────────────────────┘
                                    │
                                    ▼
          ┌──────────────────────────────────────┐
   1004   │ Discard any statements that exists in the │
          │     positive motif-based statements       │
          └──────────────────────────────────────┘
                                    │
                                    ▼
                          ┌─────────────┐
                          │     End     │
                          └─────────────┘
```

Positive Candidate Statements Generation Unit

Figure 12

66

```
                          ( Start )
                             │
                             ▼
1101 ┌──────────────────────────────────────────┐
     │         Read all candidate statements     │
     └──────────────────────────────────────────┘
                             │
                             ▼
1102 ┌──────────────────────────────────────────┐
     │     Read all negative motif-based statements │
     └──────────────────────────────────────────┘
                             │
                             ▼
1103 ┌──────────────────────────────────────────┐
     │       For each positive candidate statement, do: │
     │       For each kinase and phosphorylation motif in the │
     │  positive candidate statement, build negative candidate │
     │    statements as the Cartesian product of the kinase, the │
     │      phosphorylation motif and all associated substrates │
     │  associated to that kinase and phosphorylation motif in │
     │          the negative motif-based statements │
     └──────────────────────────────────────────┘
                             │
                             ▼
1104 ┌──────────────────────────────────────────┐
     │       For each positive candidate statement, do: │
     │     For each phosphorylation motif and substrate in the │
     │   positive candidate statement, build negative candidate │
     │  statements as the Cartesian product of the substrate, the │
     │       phosphorylation motif and all associated kinases │
     │   associated to that substrate and phosphorylation motif │
     │          in the negative motif-based statements │
     └──────────────────────────────────────────┘
                             │
                             ▼
1105 ┌──────────────────────────────────────────┐
     │   Discard any negative statements that exists in the │
     │            positive candidate statements │
     └──────────────────────────────────────────┘
                             │
                             ▼
                          ( End )
```

Negative Candidate Statements Generation Unit

Figure 13

```
                        ┌──────────┐
                        │  Start   │
                        └──────────┘
                             │
                             ▼
          ┌──────────────────────────────────────┐
          │     Read motif-based statements       │
          │  (positives and negatives), and protein│
  1401 ──│    context statements (positives and   │
          │            negatives).                 │
          └──────────────────────────────────────┘
                             │
                             ▼
          ┌──────────────────────────────────────┐
  1402 ──│           Train a model                │
          └──────────────────────────────────────┘
                             │
                             ▼
          ┌──────────────────────────────────────┐
          │     Predict scores of candidate        │
  1403 ──│  statements, and their rankings against│
          │         plausible negatives            │
          └──────────────────────────────────────┘
                             │
                             ▼
                        ┌──────────┐
                        │   End    │
                        └──────────┘
```

Relational Learning Unit

Figure 14

1201

Predicted
motif-based
statements

113

Conversion to Site-
specific Statements
Unit

114

Predicted site
specific
phosphorylation
statements

112

Phosphorylation
sites database

103

Protein sequence
database

Conversion to site-specific statements Unit

Figure 15

Start

Read predicted motif-based phosphorylation statements

*1301*

For each predicted motif-based statement, get the list of possible phosphorylation sites

*1302*

For each possible phosphorylation site, get context sequences

*1303*

For each possible phosphorylation site, compute the conformance score of the phosphorylation site sequence with regard to the PSSM matrix of the phosphorylation motif in the predicted triple.

*1304*

Build a prediction statement for all phosphorylation site sequences with a positive conformance score

*1305*

End

Conversion to site-specific statements Unit flowchart

Figure 16

Figure 17

Figure 18

Figure 19

EP 3 404 568 B1

Figure 20

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NEUBERGER, G et al.** pkaPS: prediction of protein kinase A phosphorylation sites with the simplified kinase-substrate binding model. *Biology direct,* vol. 2 (1), 1-23
- **MAXIMILIAN NICKEL et al.** A review of relational machine learning for knowledge graphs. *arXiv: 1503.00759,* 2015
- **MATT GARDNER ; TOM M MITCHELL.** Efficient and Expressive Knowledge Base Completion Using Subgraph Feature Extraction. *EMNLP,* 2015, 1488-149
- Knowledge Base Completion via Search-based Question Answering. **ROBERT WEST et al.** Proceedings of the 23rd International Conference on World Wide Web. WWW. ACM, 2014, vol. 14, 515-526
- **FRANK L HITCHCOCK.** The expression of a tensor or a polyadic as a sum of products. *Studies in Applied Mathematics,* 1927, vol. 6 (1-4), 164-189
- **BISHAN YANG et al.** Embedding entities and relations for learning and inference in knowledge bases. *arXiv:1412.6575,* 2014
- **MAXIMILIAN NICKEL ; VOLKER TRESP ; HANS-PETER KRIEGEL.** A three-way model for collective learning on multi-relational data. *Proceedings of the 28th international conference on machine learning (ICML-11),* 2011, 809-816
- **ANTOINE BORDES et al.** Translating embeddings for modeling multi-relational data. *Advances in neural information processing systems,* 2013, 2787-2795
- **THEO TROUILLON et al.** Complex embeddings for simple link prediction. *arXiv:1606.06357,* 2016
- **RICHARD SOCHER et al.** Reasoning with neural tensor networks for knowledge base completion. *Advances in neural information processing systems,* 2013, 926-934
- **MAXIMILIAN NICKEL ; LORENZO ROSASCO ; TOMASO POGGIO.** Holographic embeddings of knowledge graphs. *arXiv:1510.04935,* 2015
- **J. C. OBENAUER ; L. C. CANTLEY ; M. B. YAFFE.** Scansite 2.0: Proteome-wide prediction of cell signaling interactions using short sequence motifs. *Nucleic acids research,* 2003, vol. 31 (13), 3635-3641
- **Y. XUE ; J. REN ; X. GAO ; C. JIN ; L. WEN ; X. YAO.** GPS 2.0, a tool to predict kinase-specific phosphorylation sites in hierarchy. *Molecular & cellular proteomics,* 2008, vol. 7 (9), 1598-1608
- **N. BLOM ; T. SICHERITZ-PONT'EN ; R. GUPTA ; S. GAMMELTOFT ; S. BRUNAK.** Prediction of post-translational glycosylation and phosphorylation of proteins from the amino acid sequence. *Pro-mics,* 2004, vol. 4 (6), 1633-1649
- **H. HORN ; E. M. SCHOOF ; J. KIM ; X. ROBIN ; M. L. MILLER ; F. DIELLA ; A. PALMA ; G. CESARENI ; L. J. JENSEN ; R. BINDING.** KinomeX-plorer: an integrated platform for kinome biology studies. *Nature methods,* 2014, vol. 11 (6), 603-604
- **H. HORN ; E. M. SCHOOF ; J. KIM ; X. ROBIN ; M. L. MILLER ; F. DIELLA ; A. PALMA ; G. CESARENI ; L. J. JENSEN ; R. BINDING.** KinomeX-plorer: an integrated platform for kinome biology studies. *Nature methods,* 2014, vol. 11 (6), 603-604
- **R. BINDING ; L. J. JENSEN ; G. J. OSTHEIMER ; M. A. VAN VUGT ; C. JORGENSEN ; I. M. MIRON ; F. DIELLA ; K. COLWILL ; L. TAYLOR ; K. ELDER et al.** Systematic discovery of in vivo phosphorylation networks. *Cell,* 2007, vol. 129 (7), 1415-1426
- **J. SONG ; H. WANG ; J. WANG ; A. LEIER ; T. MARQUEZ-LAGO ; B. YANG ; Z. ZHANG ; T. AKUTSU ; G. I. WEBB ; R. J. DALY.** Phosphopredict: A bioinformatics tool for prediction of human kinase-specific phosphorylation substrates and sites by integrating heterogeneous feature selection. *Scientific Reports,* 2017, vol. 7 (1), 6862
- **P. V. HORNBECK ; J. M. KORNHAUSER ; S, TKACHEV ; B. ZHANG ; E. SKRZYPEK ; B. MURRAY ; V. LATHAM ; M. SULLIVAN.** PhosphoSitePlus: a comprehensive resource for investigating the structure and function of experimentally determined post-translational modifications in man and mouse. *Nucleic acids research,* 2011, vol. 40 (D1), 11-22
- **G. MANNING ; D. B. WHYTE ; R. MARTINEZ ; T. HUNTER ; S. SUDARSANAM.** The protein kinase complement of the human genome. *Science,* 2002, vol. 298 (5600), 1912-1934

- **A. BORDES ; N. USUNIER ; A. GARCIA-DURAN ; J. WESTON ; O. YAKHNENKO.** Translating embeddings for modeling multi-relational data. *Advances in neural information processing systems,* 2013, 2787-2795
- **B. YANG ; W.-T. YIH ; X. HE ; J. GAO ; L. DENG.** Embedding entities and relations for learning and inference in knowledge bases. *arXiv:1412.6575,* 2014
- **T. TROUILLON ; J. WELBL ; S. RIEDEL ; E'. GAUSSIER ; G. BOUCHARD.** Complex embeddings for simple link prediction. *arXiv:1606.06357,* 2016
- **T.-Y. LIU et al.** Learning to rank for information retrieval. *Foundations and Trends in Information Retrieval,* 2009, vol. 3 (3), 225-331
- The relationship between precision-recall and roc curves. **J. DAVIS ; M. GOADRICH.** Proceedings of the 23rd international conference on Machine learning. ACM, 2006, 233-240